# EUROPEAN PATENT APPLICATION

(11) **EP 3 988 090 A1**
(43) Date of publication of application: **27.04.2022**
(21) Application number: 20826109.9
(22) Date of filing: 18.06.2020
(51) Int. Cl.: A61K 31/137, A61K 31/4412, A61K 31/407, A61K 31/4453, A61K 31/40, A61P 3/10, A61P 25/28, A61P 35/00, G01N 33/68

(54) **P62 LIGAND COMPOUND AND ER-PHAGY-PROMOTING USE THEREOF**

(30) Priority: 18.06.2019 US 201962862788 P
(71) Applicant: Protech Inc., Seoul 03080 (KR)
(72) Inventor: KWON, Yong Tae, Seoul 03080 (KR); JI, Chang Hoon, Seoul 03080 (KR); KIM, Hee Yeon, Seoul 03080 (KR); HEO, Ah Jung, Seoul 03080 (KR); GANIPISETTI, Srinivasrao, Seoul 03080 (KR)
(74) Representative: Maiwald Patent- und Rechtsanwaltsgesellschaft mbH
(86) International application number: PCT/KR2020/007926
(87) International publication number: WO 2020/256444

(57) **Abstract**

Provided are a composition for promoting ER-phagy, a composition for maintaining ER homeostasis or reducing ER stress, and a pharmaceutical composition for preventing and/or treating ER-stress-related diseases, which comprise a p62 ligand compound as an active ingredient.

## Description

### [Technical Field]

Provided are a composition for promoting ER-phagy, a composition for maintaining ER homeostasis or reducing ER stress, and a pharmaceutical composition for preventing and/or treating a disease associated with ER-stress, which comprise a p62 ligand compound as an active ingredient.

### [Background Art]

Macroautophagy is a catabolic process by which cytoplasmic constituents such as misfolded proteins and organelles are sequestered by autophagosomes and digested by lysosomal hydrolases. The targeting of autophagic cargoes involves specific receptors such as p62 and neighbor of BRCA gene 1 (NBR1) that carry the UBA domain to recognize ubiquitin (Ub) chains on protein cargoes and the LIR domain to interact with LC3. Cargo-associated p62 is delivered to autophagic membranes through its interaction with LC3. Selective autophagy by LIR-containing receptors facilitates the removal of various subcellular organelles such as mitochondria, peroxisomes, and the endoplasmic reticulum (ER). Organellophagy initiates when autophagic adaptors bind to both polyubiquitinated transmembrane receptors and LC3 on autophagic membranes. Alternatively, membrane-associated receptors may directly via their LIR domains or indirectly bind to LC3 on autophagic membranes.

Like the mitochondrion, the ER is also known to be subject to turnover constitutively and in a stress-induced manner. Recently, various ER membrane-associated receptors reticulophagy regulator 1 (FAM134B), reticulon 3 (RTN3), and translocation protein SEC62 (Sec62) have been identified, and their LIR domains directly recruit phagophores to facilitate ER-phagy. However, mechanisms for a Ub- and adaptor-dependent ER-phagy, akin to the PINK1-Parkin pathway in mitophagy, have yet to be discovered.

Meanwhile, perturbations in the normal functions and homeostasis of the ER, due to factors such as accumulation of misfolded proteins and their aggregates in the ER lumen, lead to ER stress. To cope with this stress, cells operate adaptive quality control systems, such as the unfolded protein response (UPR). The initial objective of the UPR is to re-establish ER homeostasis via the chaperone-mediated refolding of misfolded proteins during inhibition of general protein translation. Simultaneously, the cell employs protein quality control systems such as ERAD (ER-Associated Degradation) for ubiquitination and proteasomal degradation of soluble misfolded proteins. Should ER stress persist, however, the UPR then induces programmed cell death, which modulates the pathogenesis of neurodegenerative diseases, metabolic disorders and cancer. Thus, targeting ER stress is a highly promising therapeutic avenue of research, with chemical chaperones and small molecule inhibitors of specific UPR pathways showing clinical efficacy in a variety of disease models.

Hence, there is a need to develop a technology for reducing ER stress by removing denatured proteins such as misfolded proteins and aggregates in the endoplasmic reticulum, which is to be applied to the treatment of various diseases.

### [Summary of Invention]

### [Technical Problem]

An embodiment provides the one or more uses of a p62 ligand compound selected from:
(1) modulating (e.g., activating, stimulating, or increasing) p62 to interact with a receptor which is associated with autophagic degradation of an endoplasmic reticulum (ER) component (e.g., ER compartment, etc.) (hereinafter, ER-phagy);
(2) modulating (e.g., activating, stimulating, or increasing) oligomerization and/or aggregation of a receptor associated with ER-phagy;
(3) modulating (e.g., activating, stimulating, or increasing) formation of autophagosome comprising an endoplasmic reticulum component (e.g., ER compartment, etc.);
(4) delivering autophagosome comprising an endoplasmic reticulum component (e.g., ER compartment, etc.) to lysosome;
(5) inducing or modulating (e.g., activating, stimulating, or increasing) ER-phagy and/or ER turnover;
(6) reducing ER-stress (e.g., caused by wear-and-tear, proteotoxic stress, etc.) and/or maintaining or enhancing ER homeostasis;
(7) controlling ER protein quality; and/or
(8) preventing and/or treating a disease associated with ER-stress.

### [Solution to Problem]

An embodiment provides a pharmaceutical composition for modulating (e.g., activating, stimulating, or increasing) p62 to interact with (or recognize or bind to) a receptor associated with ES-phagy, the composition comprising a p62 ligand compound.

Another embodiment provides a method of modulating p62 to interact with a receptor associated with ER-phagy, the method comprising administering a pharmaceutically effective amount of a p62 ligand compound to a subject in need of modulating p62 to interact with a receptor associated with ER-phagy. The method may further comprise identifying the subject in need of modulating p62 to interact with a receptor associated with ER-phagy, prior to the administering step.

Another embodiment provides a pharmaceutical composition for modulating (e.g., activating, stimulating, or increasing) oligomerization and/or aggregation of a receptor associated with ER-phagy, the composition comprising a p62 ligand compound.

Another embodiment provides a method of modulating oligomerization and/or aggregation of a receptor associated with ER-phagy, the method comprising administering a pharmaceutically effective amount of a p62 ligand compound to a subject in need of modulating oligomerization and/or aggregation of a receptor associated with ER-phagy. The method may further comprise identifying the subject in need of modulating oligomerization and/or aggregation of a receptor associated with ER-phagy, prior to the administering step.

Another embodiment provides a pharmaceutical composition for modulating (e.g., activating, stimulating, or increasing) formation of autophagosome comprising an endoplasmic reticulum component, the composition comprising a p62 ligand compound.

Another embodiment provides a method of modulating formation of autophagosome comprising an endoplasmic reticulum component, the method comprising administering a pharmaceutically effective amount of a p62 ligand compound to a subject in need of modulating formation of autophagosome comprising an endoplasmic reticulum component. The method may further comprise identifying the subject in need of modulating formation of autophagosome comprising an endoplasmic reticulum component, prior to the administering step.

Another embodiment provides a pharmaceutical composition for delivering autophagosome comprising an endoplasmic reticulum component to lysosome, the composition comprising a p62 ligand compound.

Another embodiment provides a method of delivering autophagosome comprising an endoplasmic reticulum component to lysosome, the method comprising administering a pharmaceutically effective amount of a p62 ligand compound to a subject in need of delivering autophagosome comprising an endoplasmic reticulum component to lysosome. The method may further comprise identifying the subject in need of delivering autophagosome comprising an endoplasmic reticulum component to lysosome, prior to the administering step.

Another embodiment provides a pharmaceutical composition for inducing or modulating (e.g., activating, stimulating, or increasing) ER-phagy and/or ER turnover, the composition comprising a p62 ligand compound.

Another embodiment provides a method of inducing or modulating ER-phagy and/or ER turnover, the method comprising administering a pharmaceutically effective amount of a p62 ligand compound to a subject in need of inducing or modulating ER-phagy and/or ER turnover. The method may further comprise identifying the subject in need of inducing or modulating ER-phagy and/or ER turnover, prior to the administering step.

Another embodiment provides a composition (reliever) for removing, alleviating, or decreasing ER-stress, the composition comprising a p62 ligand compound.

Another embodiment provides a method of reducing (removing, alleviating, or decreasing) ER-stress, the method comprising administering a pharmaceutically effective amount of a p62 ligand compound to a subject in need of reducing (removing, alleviating, or decreasing) ER-stress. The method may further comprise identifying the subject in need of reducing (removing, alleviating, or decreasing) ER-stress, prior to the administering step.

Another embodiment provides a composition for being added to a cell culture medium, the composition comprising a p62 ligand compound. The composition for being added to a cell culture medium may be for reducing (removing, alleviating, or decreasing) ER-stress of cells during cell culture. Another embodiment provides a cell culture method, comprising adding a p62 ligand compound to a culture medium in order to reduce (remove, alleviate, or decrease) ER-stress of cells during cell culture. The cell may be a cell for producing useful proteins such as antibodies.

Another embodiment provides a pharmaceutical composition for maintaining or enhancing ER homeostasis, the composition comprising a p62 ligand compound.

Another embodiment provides a method of maintaining or enhancing ER homeostasis, the method comprising administering a pharmaceutically effective amount of a p62 ligand compound to a subject in need of maintaining or enhancing ER homeostasis. The method may further comprise identifying the subject in need of maintaining or enhancing ER homeostasis, prior to the administering step.

Another embodiment provides a pharmaceutical composition for reducing ER-stress and/or maintaining or enhancing ER homeostasis, the composition comprising a p62 ligand compound.

Another embodiment provides a method of reducing ER-stress and/or maintaining or enhancing ER homeostasis, the method comprising administering a pharmaceutically effective amount of a p62 ligand compound to a subject in need of reducing ER-stress and/or maintaining or enhancing ER homeostasis. The method may further comprise identifying the subject in need of reducing ER-stress and/or maintaining or enhancing ER homeostasis, prior to the administering step.

Another embodiment provides a pharmaceutical composition for controlling (managing) ER protein quality, the composition comprising a p62 ligand compound.

Another embodiment provides a method of controlling ER protein quality, the method comprising administering a pharmaceutically effective amount of a p62 ligand compound to a subject in need of controlling ER protein quality. The method may further comprise identifying the subject in need of controlling ER protein quality, prior to the administering step.

Another embodiment provides a pharmaceutical composition for preventing and/or treating a disease associated with ER-stress, the composition comprising a p62 ligand compound.

Another embodiment provides a method of preventing and/or treating a disease associated with ER-stress, the method comprising administering a pharmaceutically effective amount of a p62 ligand compound to a subject in need of preventing and/or treating a disease associated with ER-stress. The method may further comprise identifying the subject in need of preventing and/or treating a disease associated with ER-stress, prior to the administering step.

Another embodiment provides a method of screening a candidate drug for preventing and/or treating a disease associated with ER-stress, the method comprising:
treating (contacting) a candidate compound with a biological sample comprising p62 and a receptor associated with ER-phagy;
confirming (or measuring or detecting) formation of a complex of p62 and the receptor associated with ER-phagy, and/or oligomerization and/or aggregation of the receptor associated with ER-phagy; and
determining (or selecting) the candidate compound as a candidate drug for preventing and/or treating a disease associated with ER-stress when the formation of a complex of p62 and the receptor associated with ER-phagy, and/or oligomerization and/or aggregation of the receptor associated with ER-phagy is confirmed (or measured or detected, or increased more than in a biological sample (reference sample) that is not treated (contacted) with the candidate compound.

Hereinafter, the present invention will be described in more detail.

As used herein, the p62 ligand compound may be one or more selected from the compounds listed in Tables 1 and 2 below, or a pharmaceutically acceptable salt, stereoisomer, solvate, hydrate or prodrug thereof:

**[Table 1]**

| No. | Structure | No. | Structure |
|---|---|---|---|
| YOK 110 5 | 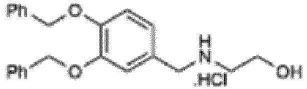 | YOK 220 4 | 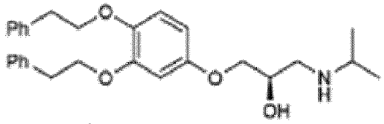 |
| YOK 110 4 | 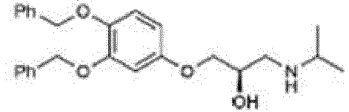 | YOK 110 6 | 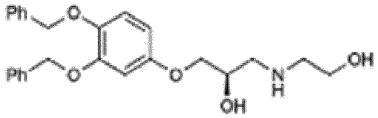 |
| YOK 120 5 | 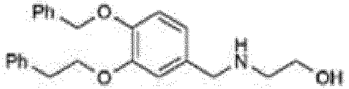 | ATB -15 | 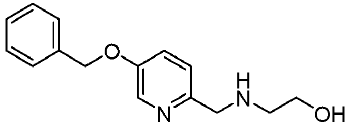 |
| ATB -1 | 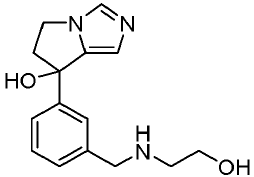 | ATB -16 | 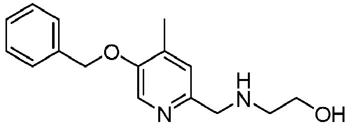 |
| ATB -2 | 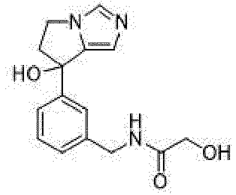 | ATB -17 | 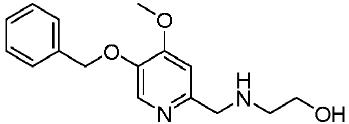 |
| ATB -3 | 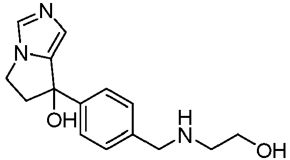 | ATB -18 | 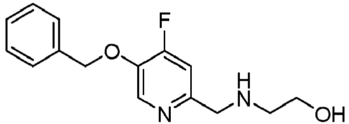 |
| ATB -4 | 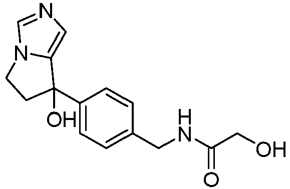 | ATB -19 | 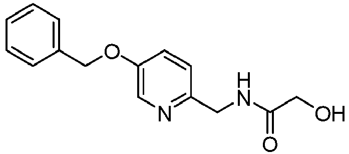 |
| ATB -5 | 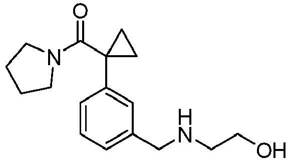 | ATB -20 | 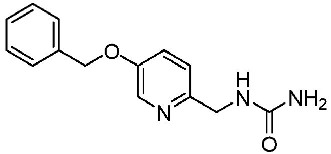 |
| ATB -6 | 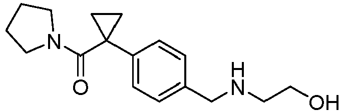 | ATB -21 | 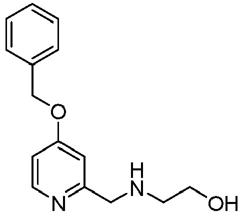 |
| ATB -7 | 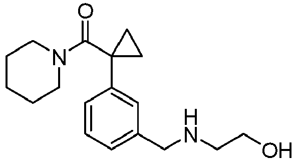 | ATB -22 | 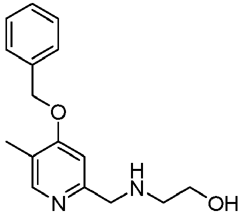 |
| ATB -8 | 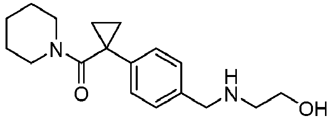 | ATB -23 | 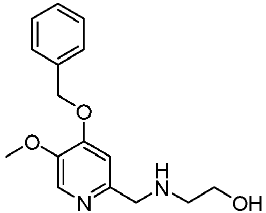 |
| ATB -9 | 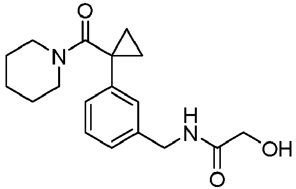 | ATB -24 | 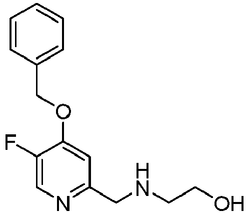 |
| ATB -10 | 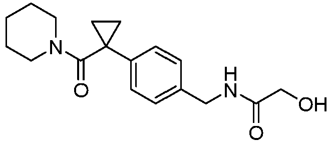 | ATB -25 | 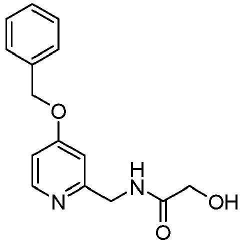 |
| ATB -11 | 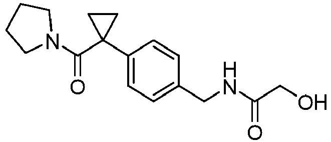 | ATB -26 | 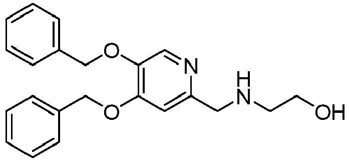 |
| ATB -12 | 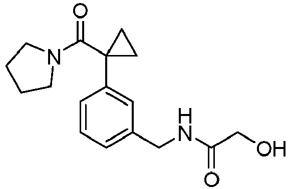 | ATB -27 | 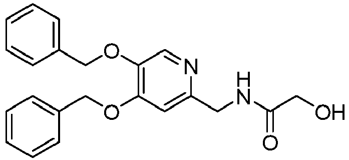 |
| ATB -13 | 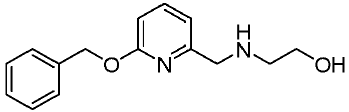 | ATB -28 | 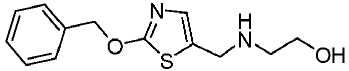 |
| ATB -14 | 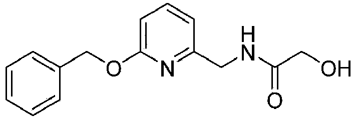 | ATB -29 | 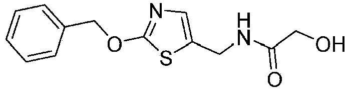 |

**[Table 2]**

| Structural Formula | No. | R1 | R2 |
|---|---|---|---|
| 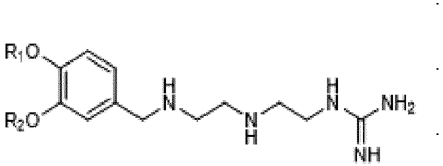 | YtK-1109 | -CH₂Ph | -CH₂Ph |
| | YtK-2209 | -CH₂CH₂Ph | -CH₂CH₂Ph |
| | YtK-3309 | -CH₂CH₂CH₂Ph | -CH₂CH₂CH₂ Ph |
| | YtK-4409 | - CH₂CH₂CH₂CH₂Ph | - CH₂CH₂CH₂CH₂Ph |
| | YtK-1209 | -CH₂Ph | -CH₂CH₂Ph |
| | YtK-1309 | -CH₂Ph | -CH₂CH₂CH₂ Ph |
| | YtK-1409 | -CH₂Ph | CH₂CH₂CH₂CH₂Ph |
| 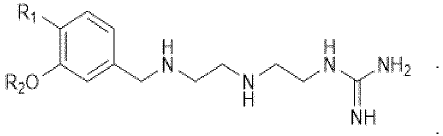 | YTK-109 | -H | -CH₂Ph |
| | YTK-209 | -H | -CH₂CH₂Ph |
| | YTK-309 | -H | -CH₂CH₂CH₂Ph |
| | YTK-409 | -H | CH₂CH₂CH₂CH₂Ph |
| 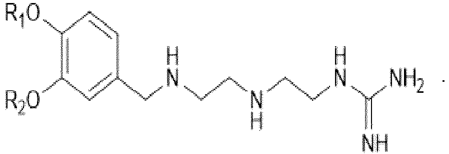 | YT-9-1 | -CH₂PhCl | -CH₂PhCl |
| | YT-9-2 | -CH₂PhF | -CH₂PhF |
| | YT-9-3 | -CH₂PhNMe₂ | -CH₂PhNMe₂ |
| | YT-9-4 | -CH₂PhNO₂ | -CH₂PhNO₂ |
| | YT-9-5 | -CH₂PhOMe | -CH₂PhOMe |
| | YT-9-6 | -CH₂PhOH | -CH₂PhOH |
| 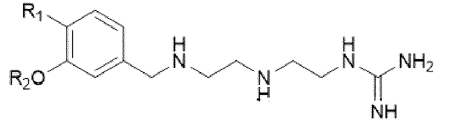 | YT-9-7 | -H | -CH₂PhCl |
| | YT- | -H | -CH₂PhF |
| | 9-8 | | |
| | YT-9-9 | -H | -CH₂PhNMe₂ |
| | YT-9-10 | -H | -CH₂PhNO₂ |
| | YT-9-11 | -H | -CH₂PhOMe |
| | YT-9-12 | -H | -CH₂PhOH |

The term "pharmaceutically acceptable salt" means arbitrary all organic or inorganic addition salts of the compounds, in which the concentrations of the salts have an effective action that is relatively nontoxic and harmless to a subject and side effects attributable to the salts do not impair the beneficial efficacy of the compounds according to the present invention. The salt may be an acid addition salt formed from a pharmaceutically acceptable free acid. An acid addition salt is prepared by a conventional method, for example, by dissolving a compound in an excessive amount of an aqueous acid solution and precipitating the salt using a water-miscible organic solvent such as methanol, ethanol, acetone or acetonitrile. Equimolar amounts of a compound and an acid or alcohol (e.g., glycol monomethyl ether) in water are heated, and then the mixture may be evaporated to dryness, or the precipitated salt may be suction-filtered. At this time, organic acids and inorganic acids may be used as the free acid, and hydrochloric acid, phosphoric acid, sulfuric acid, nitric acid, stannic acid and the like may be used as inorganic acids, and methanesulfonic acid, p-toluenesulfonic acid, acetic acid, trifluoroacetic acid, maleic acid, succinic acid, oxalic acid, benzoic acid, tartaric acid, fumaric acid, mandelic acid, propionic acid, citric acid, lactic acid, glycolic acid, gluconic acid, galacturonic acid, glutamic acid, glutaric acid, glucuronic acid, aspartic acid, ascorbic acid, carbonic acid, vanillic acid, hydroiodic acid and the like may be used as organic acids, but the free acid is not limited thereto.

A pharmaceutically acceptable metal salt may be formed using a base. An alkali metal salt or alkaline earth metal salt is obtained, for example, by dissolving a compound in an excessive amount of an alkali metal hydroxide or alkaline earth metal hydroxide solution, filtering off the undissolved compound salt, and then evaporating and drying the filtrate. At this time, it is pharmaceutically suitable to prepare a sodium, potassium, or calcium salt as the metal salt, but the metal salt is not limited thereto. A silver salt corresponding thereto may be obtained by reacting an alkali metal or alkaline earth metal salt with a suitable silver salt (e.g., silver nitrate).

Pharmaceutically acceptable salts of the compounds of the present invention, unless otherwise indicated, include salts of acidic or basic groups that may be present in the compounds listed in Table 1 or Table 2. For example, the pharmaceutically acceptable salts may include sodium, calcium and potassium salts of hydroxyl groups, and the like, and other pharmaceutically acceptable salts of amino groups include hydrobromide, sulfate, hydrogen sulfate, phosphate, hydrogen phosphate, dihydrogen phosphate, acetate, succinate, citrate, tartrate, lactate, mandelate, methanesulfonate (mesylate) and p-toluenesulfonate (tosylate) salts, and these may be prepared through a method of preparing salts known in the art.

The compounds listed in Table 1 or Table 2 according to the present invention include, without limitation, not only pharmaceutically acceptable salts thereof but also possible solvates such as hydrates and all possible stereoisomers, which may be prepared therefrom. All stereoisomers (e.g., those that may be present because of asymmetric carbons in various substituents) of the present invention, including enantiomeric and diastereomeric forms, are included within the scope of the present invention. Individual stereoisomers of the compounds may be, for example, substantially free of other isomers (e.g., as pure or substantially pure optical isomers having particular activity) or may be admixed, for example, as racemates or with all other or other selected stereoisomers. The chiral center of the compounds of the present invention may have an S or R structure as defined by the IUPAC 1974 recommendations. Racemic forms may be analyzed by physical methods such as separation by chiral column chromatography or separation or crystallization of diastereomeric derivatives, fractional shape crystallization. Individual optical isomers may be obtained from racemates by an arbitrary suitable method including, but not limited to, salt formation with an optically active acid followed by crystallization.

Solvates and stereoisomers of the compounds listed in Table 1 or Table 2 may be prepared from the compounds using methods known in the art.

The compounds listed in Table 1 or Table 2 may be prepared in a crystalline or amorphous form, and may optionally be hydrated or solvated when prepared in a crystalline form. In the present invention, not only stoichiometric hydrates of the compounds listed in Table 1, but also compounds comprising various amounts of water may be included. Solvates of the compounds listed in Table 1 according to the present invention include both stoichiometric and non-stoichiometric solvates.

As used herein, the term "ER-phagy" may refer to the degradation of an endoplasmic reticulum (ER) component (e.g., ER compartment, etc.) via an autophagy pathway.

As used herein, the term "receptor associated with ER-phagy" may refer to a protein that is localized on the target ER membrane by itself or along with p62 (e.g., formation a complex with p62), and/or has autocleavage (e.g., autoubiquitination) activity, and/or comprises a moiety or amino acid residue recognized by autophagy pathway, may be, for example, an ER membrane anchored protein such as E3 ligase (e.g., E3 ubiquitin-protein ligase TRIM13, etc.), but is not limited thereto.

As used herein, the term "disease associated with ER-stress" may refer to any disease caused by ER stress. In an embodiment, the disease associated with ER-stress may be a metabolic proteinopathy, may be selected from, for example, the group consisting of diabetes, neurodegenerative diseases (e.g., Alzheimer's, Parkinson's, prion disease, amyotrophic lateral sclerosis, etc.), cancer, metabolic syndrome (e.g., steatosis, adipocytic inflammation, obesity, chronic obstructive pulmonary disease, alpha1-antitrypsin deficiency, etc.), and the like, but is not limited thereto.

The pharmaceutical composition provided herein may further comprise a pharmaceutically acceptable carrier, diluent or excipient in addition to the active ingredient (p62 ligand compound), may be formulated and used in various forms, such as oral formulations such as powders, granules, tablets, capsules, suspensions, emulsions, syrups, and aerosols, and injections of sterile injection solutions according to conventional methods for each purpose of use, may be administered orally or administered through various routes including intravenous, intraperitoneal, subcutaneous, rectal, and topical administration. Examples of the suitable carrier, excipient or diluent that may be comprised in such a composition include, but are not limited to, lactose, dextrose, sucrose, sorbitol, mannitol, xylitol, erythritol, maltitol, starch, gum acacia, alginate, gelatin, calcium phosphate, calcium silicate, cellulose, methylcellulose, microcrystalline cellulose, polyvinylpyrrolidone, water, methyl hydroxybenzoate, propyl hydroxybenzoate, talc, magnesium stearate, and mineral oil. The pharmaceutical composition may further comprise a filler, an anti-aggregation agent, a lubricant, a wetting agent, a flavoring agent, an emulsifier, a preservative, and the like.

A solid preparation for oral administration includes tablets, pills, powders, granules, capsules and the like, and such a solid preparation is formulated by mixing at least one or more excipients, for example, starch, calcium carbonate, sucrose, lactose, or gelatin in the composition. In addition to simple excipients, lubricants such as magnesium stearate and talc may be used. Examples of a liquid preparation for oral use include suspensions, internal solutions, emulsions, and syrups, and various excipients, for example, a wetting agent, a sweetening agent, a fragrance, and a preservative may be comprised in addition to water and liquid paraffin, which are commonly used simple diluents. A preparation for parenteral administration includes sterile aqueous solutions, non-aqueous solutions, suspensions, emulsions, lyophilized preparations, and suppositories. As non-aqueous solvents and suspending agents, propylene glycol, polyethylene glycol, vegetable oils such as olive oil, injectable esters such as ethyl oleate, and the like may be used. As the base of the suppository, Witepsol, Macrogol, and Tween 61, cacao butter, laurin fat, glycerogelatin and the like may be used. Meanwhile, the injections may comprise conventional additives such as a solubilizer, an isotonic agent, a suspending agent, an emulsifier, a stabilizer, and a preservative.

The formulation may be prepared by conventional mixing, granulation or coating methods, and comprises the active ingredient in an amount effective for medical treatment, specifically for preventing, ameliorating or treating a disease associated with denatured protein aggregation.

As used herein, the "ER stress (endoplasmic reticulum stress)" may refer to a state in which an immature protein exceeding the ability of the ER to process by a physiological or pathological environment is introduced into the ER or calcium in the ER is depleted, resulting in a disorder in ER function, and may be induced by wear-and-tear, proteotoxic stress, and the like.

As used herein, the "pharmaceutically effective amount" means an amount sufficient to treat a disease at a reasonable benefit/risk ratio applicable to medical treatment and not to cause side effects, and the effective dose level may be determined according to factors including the patient's health status, the type of disease, the severity, the activity of the drug, the sensitivity to the drug, the method of administration, the time of administration, the route of administration and the rate of excretion, the duration of treatment, and the drugs used in combination or concurrently and other factors well known in the medical field. The compositions of the present invention may be administered as an individual therapeutic agent or in combination with other therapeutic agents, may be administered sequentially or simultaneously with a conventional therapeutic agent, and may be administered singly or multiple times. In consideration of all of the factors, it is important to administer the composition in an amount in which the maximum effect can be obtained with a minimum amount without side effects, and the amount may be appropriately determined by those skilled in the art. For example, the dosage may be increased or decreased depending on the route of administration, the severity of the disease, sex, weight, age and the like, and thus is not intended to limit the scope of the present invention in any case.

As used herein, the term "subject" may be a eukaryotic organism (animal), for example, a mammal such as human, or a cell or tissue isolated (derived) from a eukaryotic organism (may be a genetically engineered or artificially cultured cell or tissue in some case). The subject of administration of a p62 ligand compound as an active ingredient provided herein or a pharmaceutical composition comprising the same may be selected from mammals or birds, such as monkeys, cows, horses, sheep, pigs, chickens, turkeys, quails, cats, dogs, mice, rats, rabbits, and guinea pigs, including humans, that have the potential to develop ER stress or to develop or have the potential/risk of a disease associated with ER-stress, but is not limited thereto.

As used herein, the term "administration" means providing a predetermined substance to a patient by an arbitrary suitable method, and the compositions of the present invention may be administered through any general route as long as it can reach the target tissue. The administration may be intraperitoneal administration, intravenous administration, intramuscular administration, subcutaneous administration, intradermal administration, oral administration, topical administration, intranasal administration, intrapulmonary administration, or rectal administration, but is not limited thereto. The pharmaceutical composition of the present invention may be administered by an arbitrary device capable of transporting the active substance to the target cell. Preferred administration modes and preparations are intravenous injections, subcutaneous injections, intradermal injections, intramuscular injections, drip injections, and the like. Injections may be prepared using aqueous solvents such as physiological saline solution and Ringer's solution, non-aqueous solvents such as vegetable oils, higher fatty acid esters (e.g., ethyl oleate), and alcohols (e.g., ethanol, benzyl alcohol, propylene glycol, and glycerin), and may comprise pharmaceutical carriers such as a stabilizer to prevent denaturation (e.g., ascorbic acid, sodium hydrogen sulfite, sodium pyrosulfite, BHA, tocopherol, or EDTA), an emulsifier, a buffer for pH adjustment, and a preservative to inhibit the growth of microorganisms (e.g., phenylmercuric nitrate, thimerosal, benzalkonium chloride, phenol, cresol, or benzyl alcohol).

As used herein, the "cell for protein production" may be selected from all natural or recombinant cells capable of producing a foreign protein, and may be one capable of strongly inducing transcriptional initiation in, for example, a viral cell, a bacterial cell, a eukaryotic cell, an insect cell, a plant cell, or an animal cell. The cell may be selected from the group consisting of, for example, *E. coli* JM109, *E. coli* BL21, *E. coli* RR1, *E. coli* LE392, *E. coli* B, *E. coli* X 1776, *E*. *coli* W3110, bacillus genus strains such as Bacillus subtilis and Bacillus thuringiensis, Enterobacteriaceae and strains such as Salmonella typhimurium, Serratia marcescens and various Pseudomonas species as prokaryotic cells; yeast (Saccharomyces cerevisiae), insect cells, plant cells and animal cells, for example, mice (e.g., COP, L, C127, Sp2/0, NS-0, NS-1, At20, and NIH3T3), rats (e.g., PC12, PC12h, GH3, and MtT), hamsters (e.g., BHK, CHO, GS genetically defective CHO, and DHFR genetically defective CHO), monkeys (e.g., COS1, COS3, COS7, CV1, and Vero), and human (e.g., Hela, HEK-293, retina-derived PER-C6, cells derived from diploid fibroblasts, myeloma cells, and HepG2) as eukaryotic cells; hybridomas; and the like, but is not limited thereto.

As used herein, the "biological sample" may be cells, tissues, body fluids and the like that are isolated from the above-mentioned subject (e.g., a lesion site).

As used herein, the "candidate compound" may be one or more selected from the group consisting of small molecular compounds, proteins, peptides, oligopeptides, nucleic acid molecules (polynucleotides, oligonucleotides and the like), extracts of plants or animals, and the like, but is not limited thereto.

### [Advantageous Effects of Invention]

The compound according to the present invention acts as a ligand binding to the ZZ domain of the p62 protein, and p62 mediates ER-phagy to remove the denatured protein, and thus the compound can be usefully applied as a preventive, ameliorating and therapeutic agent for various proteinopathies.

### [Brief Description of Drawings]

FIGS. 1A to 1K illustrate that p62 mediates ER-phagy and is required to maintain ER homeostasis, in which
FIG. 1A is a fluorescence image illustrating the co-localization of p62, calnexin, and LC3-GFP puncta in HeLa cells transfected with LC3-GFP treated with bafilomycin A1 (Baf.A1; 200 nM, 6 h) (scale bar, 10 µm),
FIG. 1B is a fluorescence image illustrating the results acquired by performing the same test as in FIG. 1A except that HeLa cells are treated with *p62* siRNA (48 h) or siControl (scale bar, 10 µm),
FIG. 1C is a graph illustrating the quantified results of FIG. 1B (n = 50 cells),
FIG. 1D illustrates the results of immunoblotting analysis of ER-resident proteins in HeLa cells treated with *p62* siRNA (48 h) and then with HCQ (10 µM, 24 h),
FIG. 1E illustrates the results acquired by performing the same test as in FIG. 1D except that HeLa cells are treated with only HCQ (10 µM, 24 h),
FIG. 1F illustrates the western blotting results in HeLa cells treated with *p62* siRNA and with MG132 (2 µM, 18 h), HCQ (10 µM, 24 h), or both compared to a control,
FIG. 1G is a fluorescence image illustrating the results of immunostaining analysis of p62 and KDEL (Lys-Asp-Glu-Leu) signals in HeLa cells treated with control or *p62* siRNA (48 h) and with MG132 (2 µM, 18 h) (scale bar, 10 µm),
FIG. 1H illustrates the immunoblotting results of HeLa cells treated with MG132 (2 µM, 18 h), HCQ (10 µM, 24 h), or both,
FIG. 1I is a fluorescence image illustrating the results of immunostaining analysis of KDEL and calnexin signals in HeLa cells treated with *p62* siRNA and bafilomycin A1 (200 nM, 24 h) compared to a control (48 h) (scale bar, 10 µm),
FIG. 1J is a graph illustrating the quantified results of FIG. 1I (n = 40 cells), and
FIG. 1K illustrates the immunoblotting results of HeLa cells treated with thapsigargin (200 nM, 18 h), tunicamycin (0.1 µg/mL, 18 h), MG132 (2 µM, 18 h), or Baf. A1 (200 nM, 18 h) and with *p62* siRNA (48 h) or control siRNA (48 h) .
FIGS. 2A to 2N illustrate that N-degron Arg binds to p62 to promote ER-phagy, in which

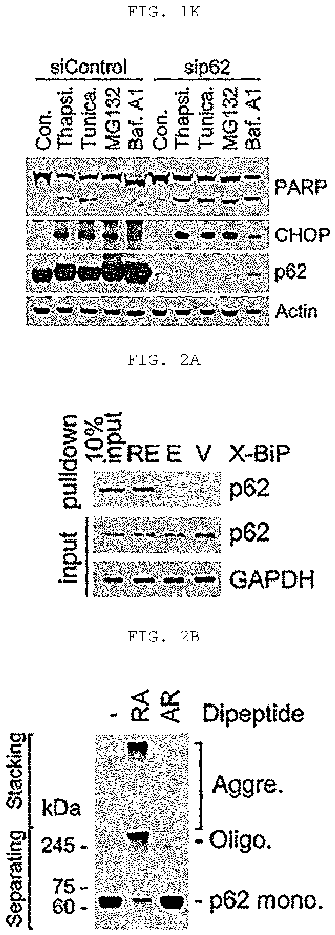

FIG. 2A illustrates the *in vitro* pulldown assay results of the X-BiP (X = E, V or RE) 12-mer peptide corresponding to the N-terminal sequence of BiP after signal peptide cleavage,
FIG. 2B illustrates the *in vitro* p62 oligomerization assay results in HEK293T cells cultured with Arg-Ala or Ala-Arg dipeptide (50 mM, 2 hours),

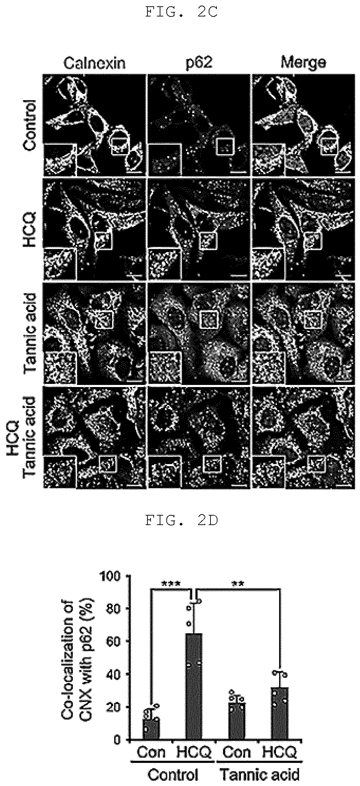

FIG. 2C is a fluorescence image illustrating the co-localization analysis results of p62 and calnexin in HeLa cells treated with HCQ (10 µM, 24 h), tannic acid (30 µM, 24 h) or both (scale bar, 10 µm),
FIG. 2D is a graph illustrating the quantified results of FIG. 2C (n = 50 cells),

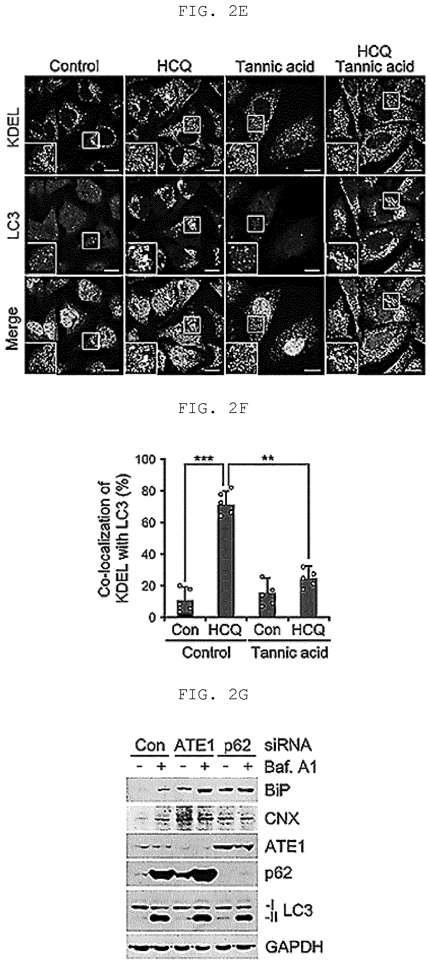

FIG. 2E is a fluorescence image illustrating the co-localization analysis results of KDEL and LC3 in HeLa cells treated with HCQ (10 µM, 24 h), tannic acid (30 µM, 24 h) or both (scale bar, 10 µm),
FIG. 2F is a graph illustrating the quantified results of FIG. 2E (n = 50 cells),
FIG. 2G illustrates the western blotting results of HeLa cells treated with bafilomycin A1 (200 nM, 7 h) under a control, p62 or ATE1 knockdown (siRNA, 48 h) condition,

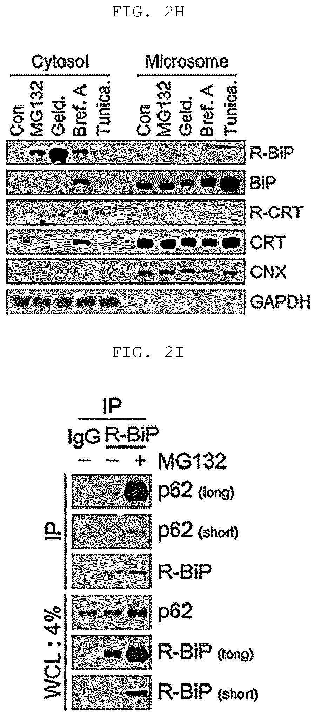

FIG. 2H illustrates the subcellular fractionation results of HeLa cells treated with MG132 (2 µM, 24 h), geldanamycin (1 µM, 24 h), brefeldin A (0.3 µM, 24 h), or tunicamycin (0.25 µg/mL, 24 h),
FIG. 2I illustrates the endogenous co-IP assay results in HEK293T cells treated with MG132 (2 µM, 24 h) or DMSO,

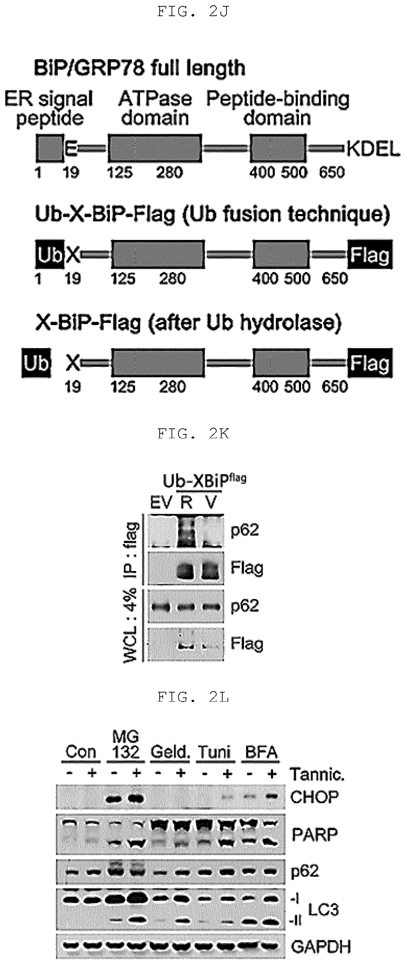

FIG. 2J schematically illustrates a cytosolic Ub-X-BiP-flag (X=R or V) construct constructed utilizing the Ub fusion technology,
FIG. 2K illustrates the Co-IP IP assay results in HEK293T cells expressing Ub-X-BiP-flag (X = R or V),
FIG. 2L illustrates the immunoblotting analysis results of HeLa cells treated with G132 (1 µM, 24 h), geldanamycin (500 nM, 24 h), tunicamycin (0.25 µg/mL, 24 h) or brefeldin A (0.3 µM, 24 h) and with or without tannic acid (25 µM, 24 h),

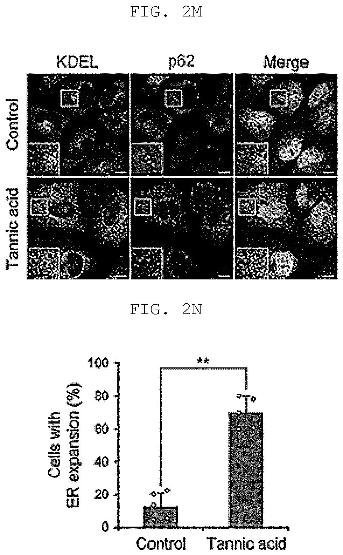

FIG. 2M illustrates the immunocytochemistry results of KDEL signals in HeLa cells treated with tannic acid (30 µM, 24 h) compared to a control (scale bar, 5 µm), and
FIG. 2N is a graph illustrating the quantified results of FIG. 2M (n = 50 cells).

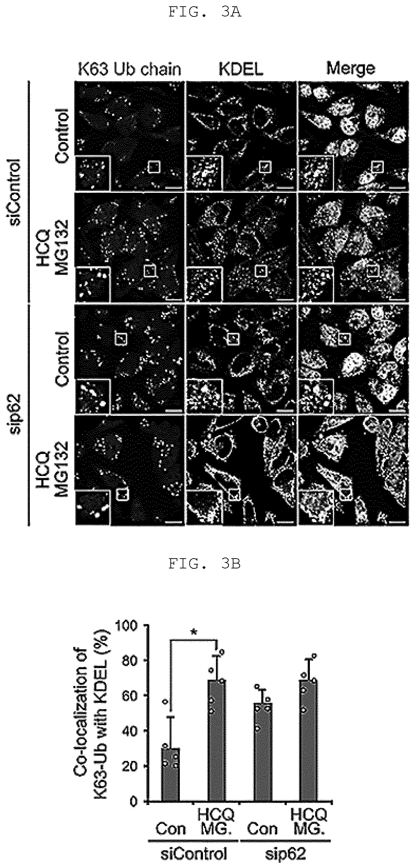

FIGS. 3A to 3K illustrate that the transmembrane E3 ligase TRIM13 acts as a receptor for p62 during ER-phagy, in which
FIG. 3A is a fluorescence image illustrating the co-localization analysis results of K63-linked poly-Ub chains and KDEL in HeLa cells treated with MG132 (2 µM, 18 h) and HCQ (10 µM, 18 h) under control or p62 knockdown (48 h) (scale bar, 10 µm),
FIG. 3B is a graph illustrating the quantified results of FIG. 3A (n = 40 cells),

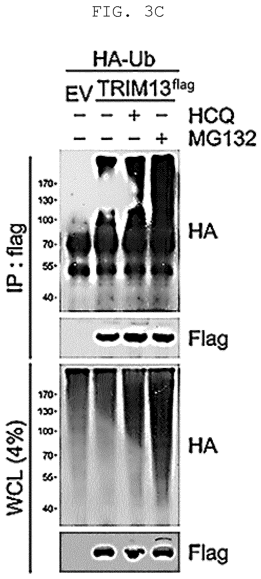

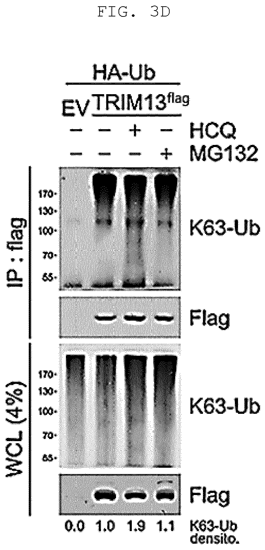

FIGS. 3C and 3D illustrate the denaturation IP assay results in HEK293T cells transfected (24 h) with TRIM13-flag or negative control empty vector (EV) and HA-Ub and then treated with HCQ (10 µM, 24 h) or MG132 (10 µM, 6 h),

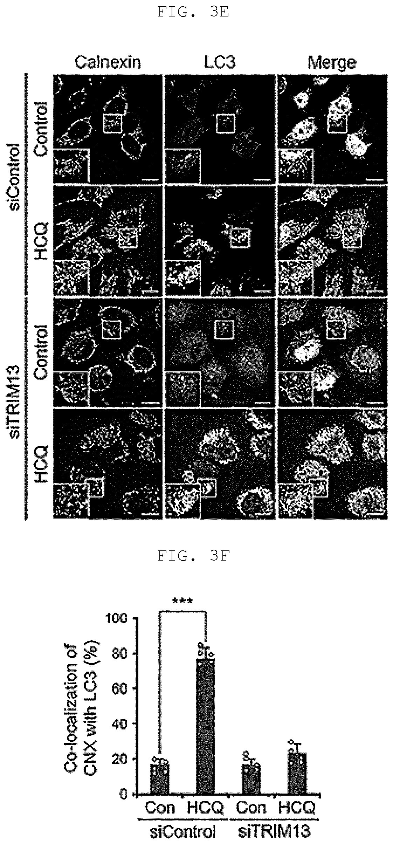

FIG. 3E is a fluorescence image illustrating the co-localization analysis results of LC3 and calnexin in HeLa cells treated with HCQ (10 µM, 24 h) under control or *TRIM13* knockdown (48 h) (scale bar, 10 µm),
FIG. 3F is a graph illustrating the quantified results of FIG. 3E (n = 50 cells),

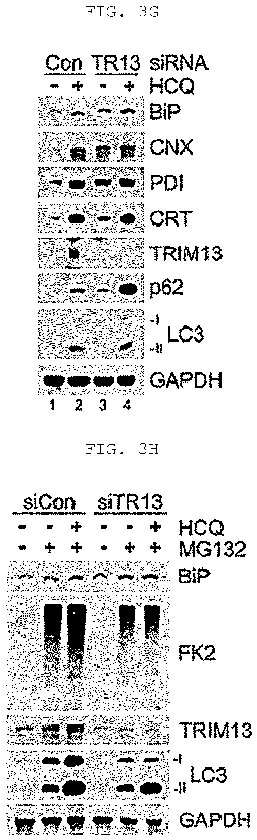

FIG. 3G illustrates the western blotting results of ER protein in HeLa treated with or without HCQ (10 µM, 24 h) under control or *TRIM13* knockdown (48 h),
FIG. 3H illustrates the western blotting results of ER protein in HeLa cells treated with MG132 (2 µM, 18 h) and with or without HCQ (10 µM, 24 h) under control or *TRIM13* knockdown (48 h),

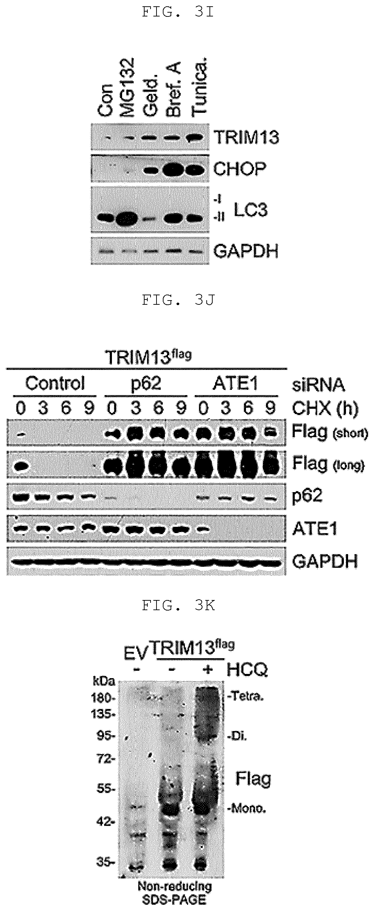

FIG. 3I illustrates the immunoblotting results of HeLa cells treated with MG132 (2 µM, 24 h), geldanamycin (1 µM, 24 h), brefeldin A (0.3 µM, 24 h), or tunicamycin (0.25 µg/mL, 24 h),
FIG. 3J illustrates the cycloheximide chase analysis results in HeLa cells expressing TRIM13-flag under siRNA-mediated knockdown of p62 or ATE1 (48 h), and
FIG. 3K illustrates the *in vivo* oligomerization assay results of TRIM13-flag in HEK293T cells treated with or without HCQ (10 µM, 24 h).

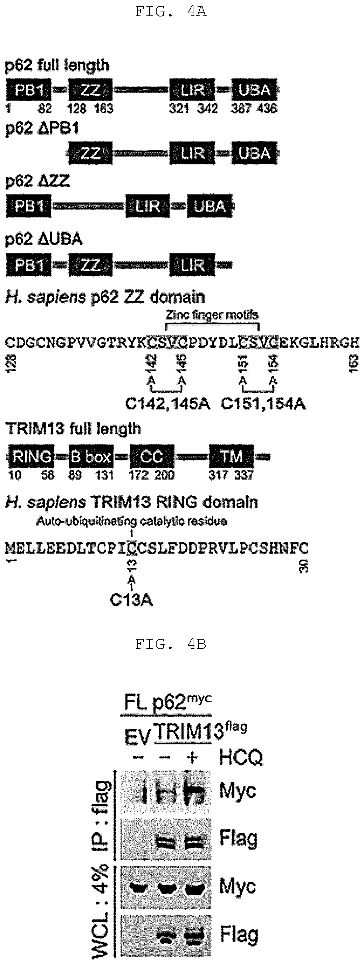

FIGS. 4A to 4O illustrate that TRIM13 mediates autophagic turnover of ER and ER-resident proteins, in which
FIG. 4A is a schematic diagram schematically illustrating the recombinant p62 and TRIM13 constructs used,
FIG. 4B illustrates the Co-IP assay results in HEK293T cells co-transfected with full-length p62-myc and TRIM13-flag or negative control empty vector (EV) (24 h) and treated with HCQ (10 µM, 24 h),

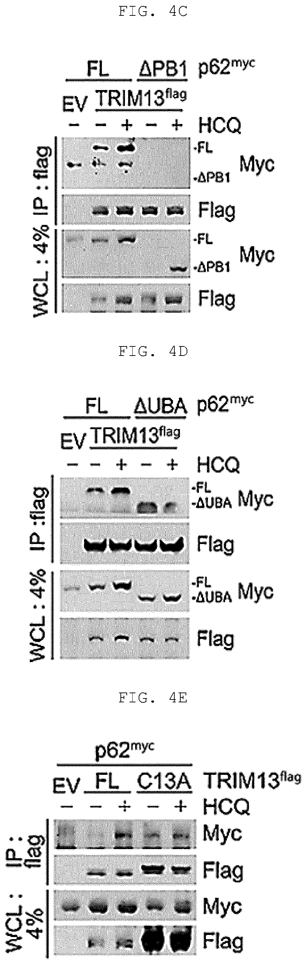

FIGS. 4C and 4D illustrate the results in full-length p62-myc-transfected cells in comparison to those in cells transfected with ΔPB1 or ΔUBA p62-myc, which are acquired by performing the same test as in FIG. 4B,
FIG. 4E illustrates the results in wild-type TRIM13-flag-transfected cells in comparison to those in cells transfected with C13A point mutation-induced TRIM13-flag, which are acquired by performing the same test as in FIG. 4B,

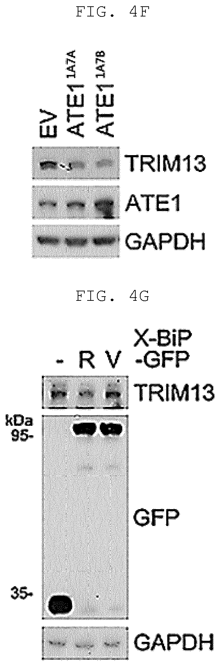

FIG. 4F illustrates the Western blotting results of TRIM13 in HeLa cells transiently expressing murine ATE1 isoforms,
FIG. 4G illustrates the Western blotting results of TRIM13 in HeLa cells expressing Ub-X-BiP-GFP (X = R, V) or negative control GFP,

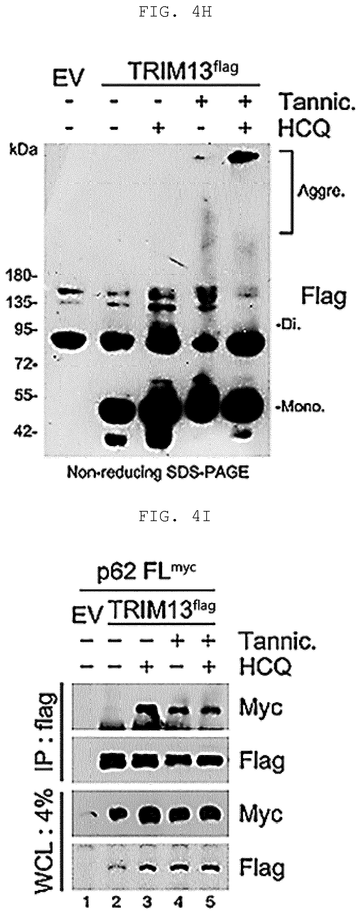

FIG. 4H illustrates the *in vivo* oligomerization assay results of TRIM13-flag in HEK293T cells treated with HCQ (10 µM, 24 h), tannic acid (30 µM, 24 h), or both,
FIG. 4I illustrates the Co-IP assay results in HEK293T cells treated with HCQ (10 µM, 24 h), tannic acid (30 µM, 24 h), or both, and co-transfected (24 h) with p62-myc and TRIM13-flag,

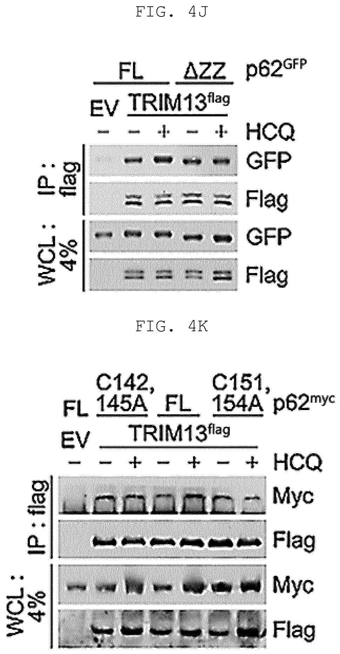

FIG. 4J illustrates the results in cells transfected with full-length p62-myc (FL) in comparison to those in cells transfected with ΔZZ p62-myc, which are acquired by performing the same test as in FIG. 4B,
FIG. 4K illustrates the results in cells transfected with full-length p62-myc (FL) in comparison to those in cells transfected with full-length ZZ domain zinc finger motif mutants C142,145A or C151,154A p62-myc, which are acquired by performing the same test as in FIG. 4B,

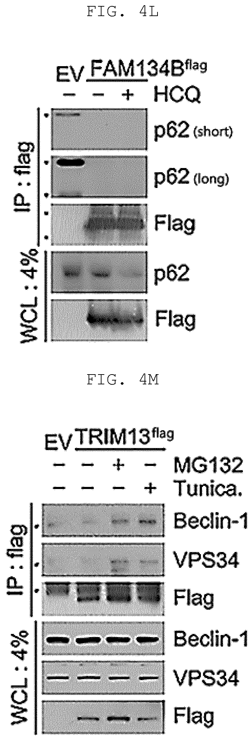

FIG. 4L illustrates the Co-IP assay results in HEK293T cells expressing FAM134B-flag and treated with or without HCQ (10 µM, 24 h),
FIG. 4M illustrates the Co-IP assay results in HEK293T cells expressing TRIM13-flag and treated with MG132 (2 µM, 24 h) or tunicamycin (0.25 µg/mL, 24 h), and

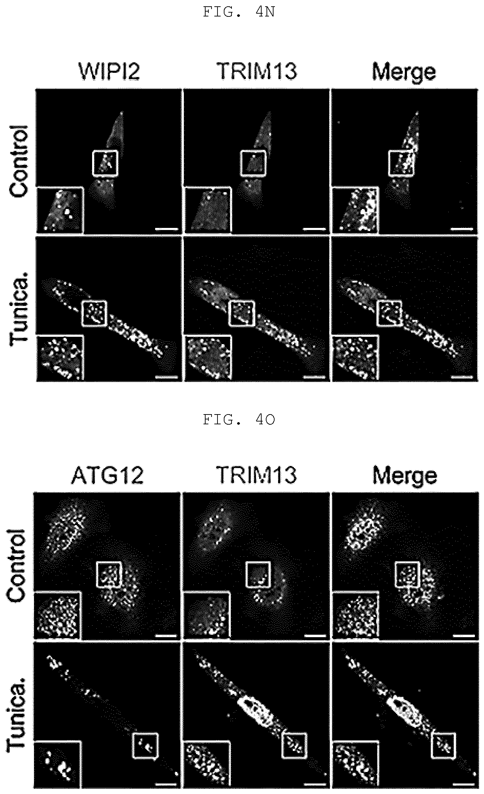

FIGS. 4N and 4O illustrate the co-localization analysis results of endogenous TRIM13 with WIPI2 or ATG16L in HeLa cells treated with tunicamycin (0.1 µg/mL, 18 h) or DMSO (negative control).

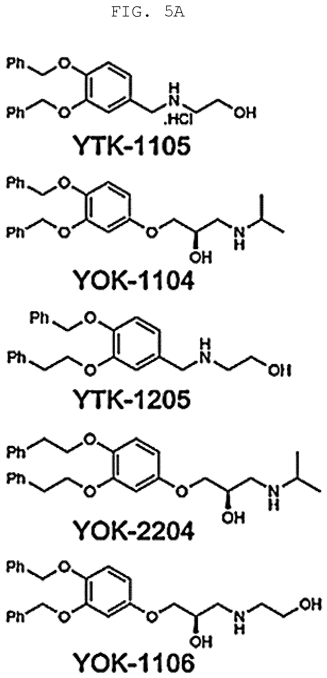

FIGS. 5A to 5L illustrate that a chemical mimic of N-degron Arg mediates ER-phagy, in which
FIG. 5A exemplarily illustrates the chemical structures of p62-ZZ ligands used in an embodiment,

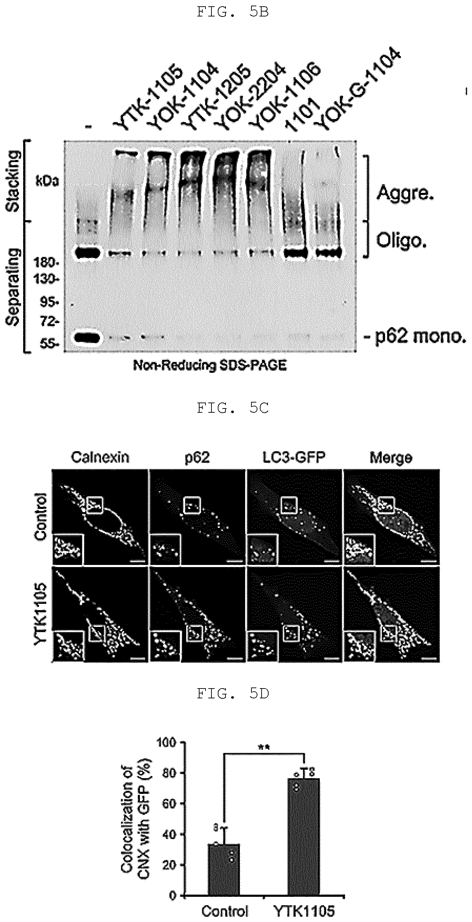

FIG. 5B illustrates the *in vitro* p62 oligomerization assay results in HEK293T cells incubated with the p62-ZZ ligands (1 mM, 2 h) illustrated in FIG. 5A,
FIG. 5C is a fluorescence image illustrating the co-localization analysis results of calnexin, p62 and LC3-GFP puncta structures in HeLa cells transfected with LC3-GFP and treated with YTK1105 (2.5 µM, 9 h) (scale bar, 10 µm),
FIG. 5D is a graph illustrating the quantified results of FIG. 5C (n = 50 cells),

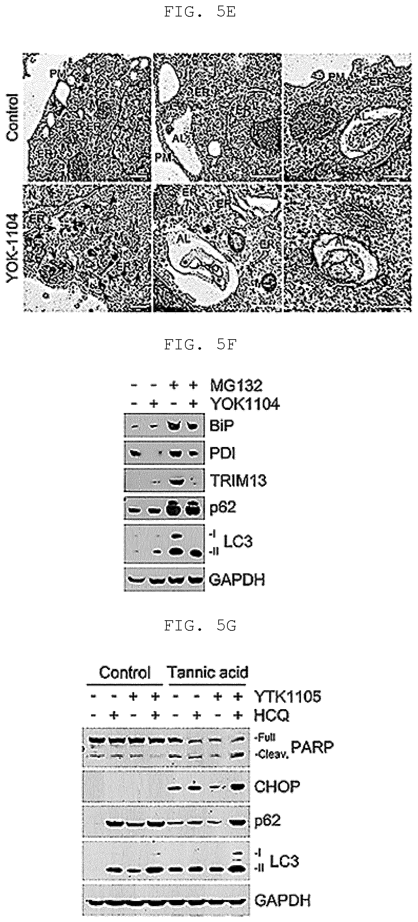

FIG. 5E is a transmission electron microscopy image of HEK293T cells treated with YOK1104 (2.5 µM, 6 h) or DMSO (control), where arrows indicate autolysosomes and asterisks indicate ER fragments inside autolysosomes (scale bars, 500 nm (1st & 2nd column), 200 nm (3rd column)) ,
FIG. 5F illustrates the immunoblotting analysis results of HeLa cells treated with MG132 (2 µM, 24 h), YOK1104 (2.5 µM, 4 h), or both,
FIG. 5G illustrates the Western blotting analysis results of HeLa cells treated with HCQ (10 µM, 24 h), YTK1105 (5 µM, 9 h), or both in the presence or absence of tannic acid (30 µM, 24 h),

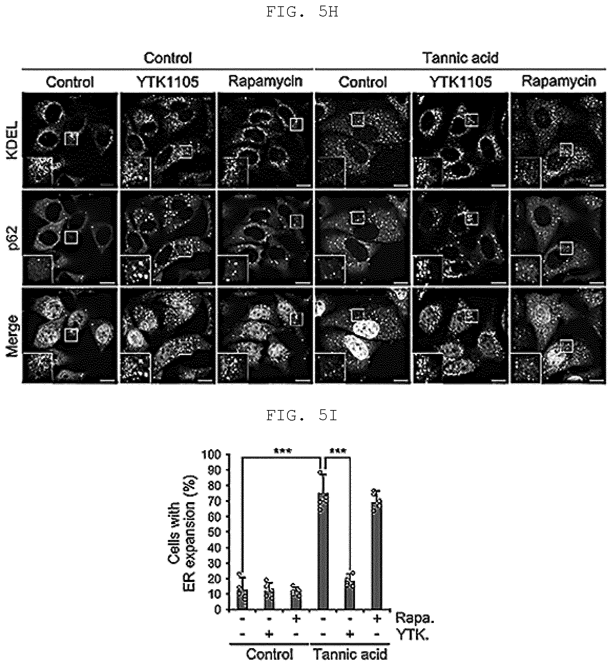

FIG. 5H illustrates the immunostaining results of KDEL signals in HeLa cells treated with or without tannic acid (30 µM, 24 h) and with YTK1105 (2.5 µM, 5 h) or rapamycin (2 µM, 18 h) (scale bar, 10 µm),
FIG. 5I is a graph illustrating the quantified results of FIG. 5H (n = 50 cells),

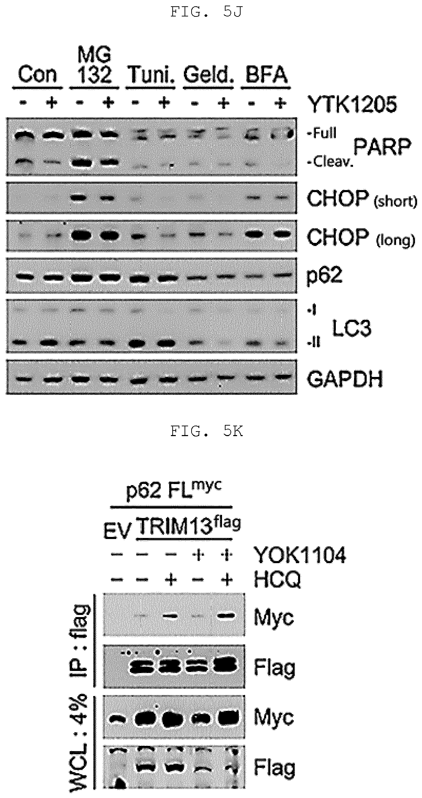

FIG. 5J illustrates the immunoblotting results of HEK293T cells treated with or without YTK1205 (2.5 µM, 24 h) and with MG132 (0.5 µM, 18 h), tunicamycin (0.25 µg/mL, 18 h), geldanamycin (500 nM, 18 h) or brefeldin A (0.3 µM, 18 h),
FIG. 5K illustrates the Co-IP assay results in HEK293T cells co-transfected with p62-myc and TRIM13-flag (24 h) or transfected with negative control empty vector (EV) and treated with HCQ (10 µM, 24 h), YOK1104 (2.5 µM, 4 h), or both, and
FIG. 5L illustrates the *In vivo* oligomerization assay results of TRIM13-flag in HEK293T cells treated with MG132 (2 µM, 24 h) and with or without YOK1104 (2.5 µM, 4 h) .

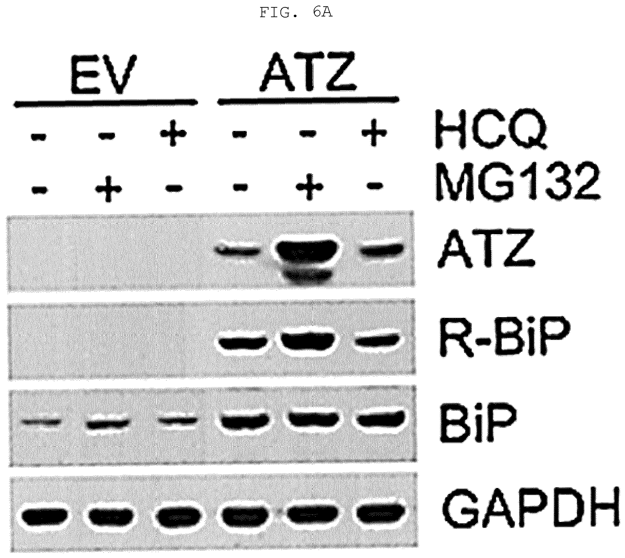

FIGS. 6A to 6L illustrate that ER-phagy modulated by p62, TRIM13, and Nt-Arg mediates clearance of alpha-antitrypsin mutant Z and autophagic targeting, in which
FIG. 6A illustrates the western blotting results of HeLa cells transfected with ATZ or negative control empty vector (EV) and treated with MG132 (2 µM, 18 h) or HCQ (10 µM, 24 h),

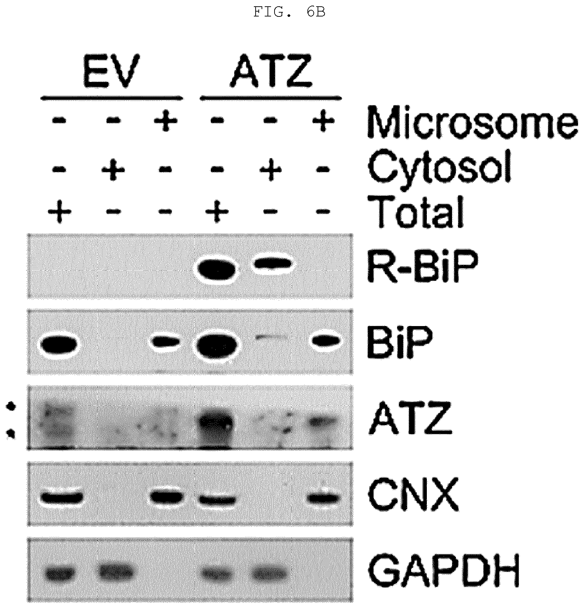

FIG. 6B illustrates the subcellular fractionation results of HeLa cells transfected with ATZ,

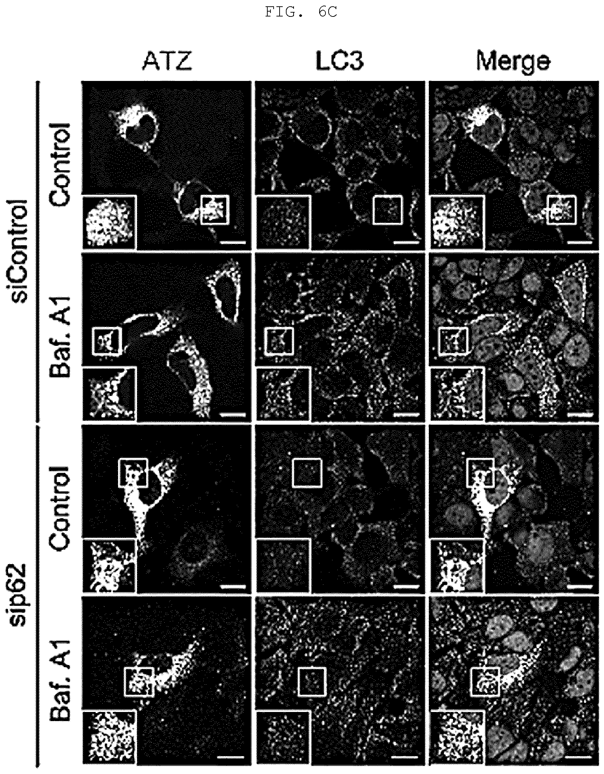

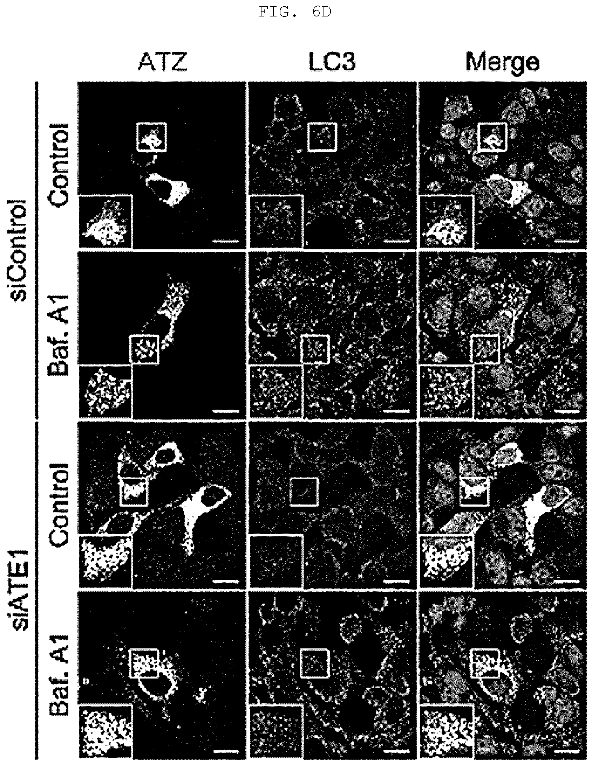

FIGS. 6C and 6D illustrate the co-localization analysis results of ATZ and LC3 in HeLa cells transfected with ATZ and treated with Baf. A1 (200nM, for 7 h) under *p62* or *ATE1* interference compared to a control (48 h) (scale bar, 10 µm),

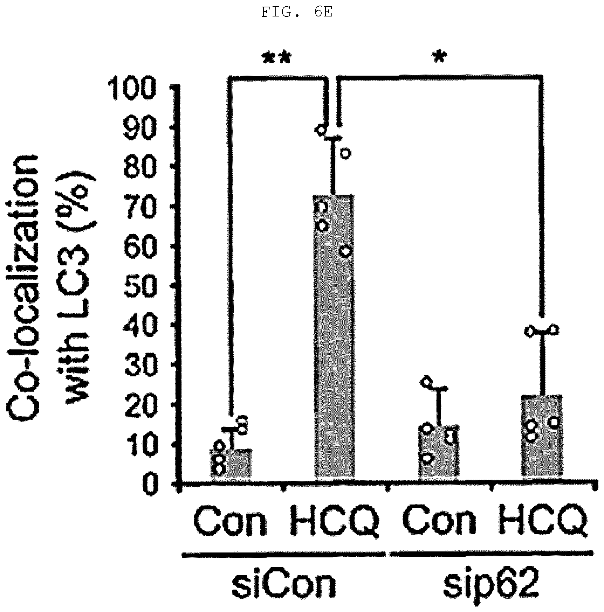

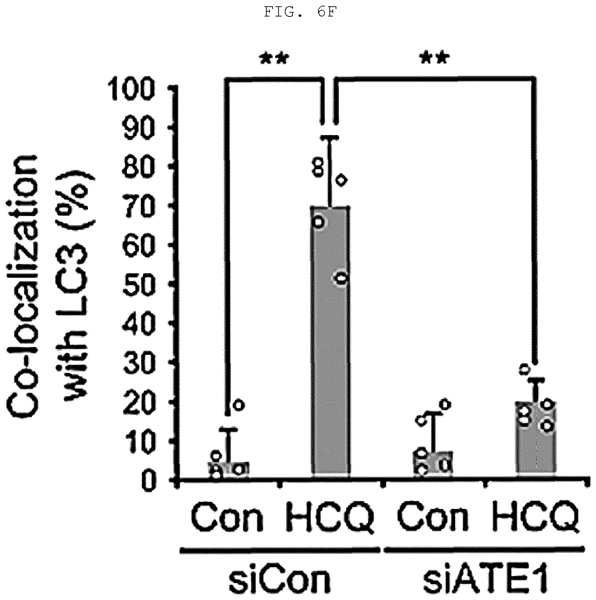

FIGS. 6E and 6F illustrate the quantified results of FIGS. 6C and 6D, respectively (n = 50 cells),

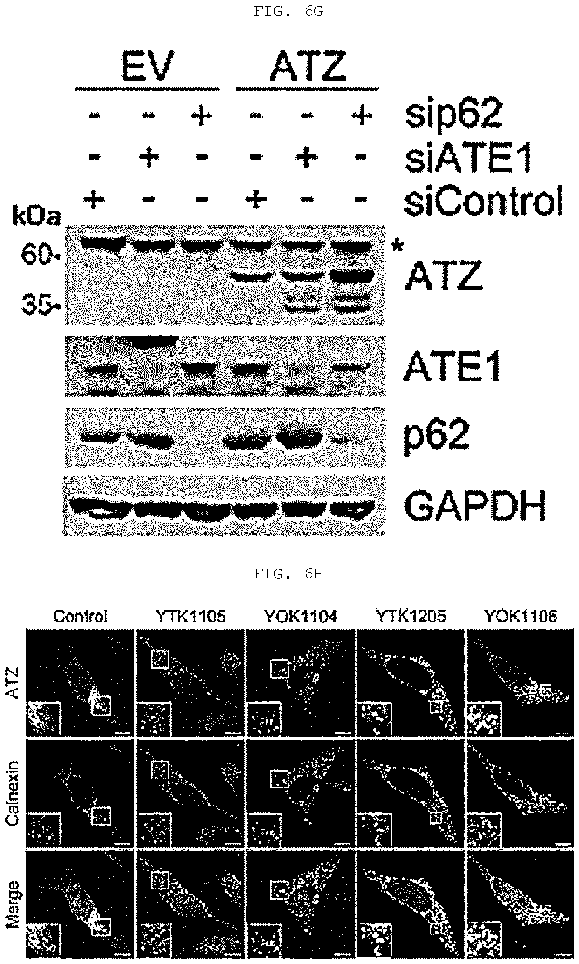

FIG. 6G illustrates the immunoblotting analysis results of HeLa cells transfected with ATZ under siRNA-mediated knockdown (48 h) of p62 or ATE1,

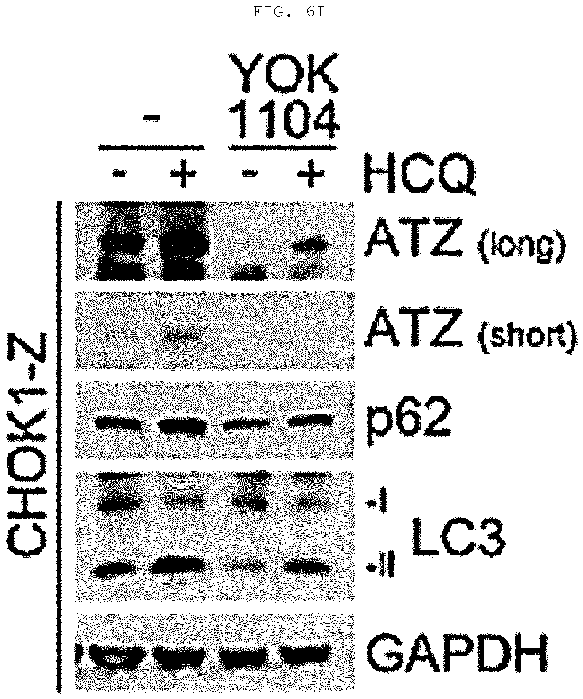

FIG. 6H illustrates the co-localization analysis results of ATZ and calnexin in ATZ-transfected HeLa cells treated with p62-ZZ ligands (5 µM, 9 h) (scale bar, 10 µm), FIG. 6I illustrates the results acquired by (I) treating CHOK1-Z cells stably expressing ATZ with HCQ (10 µM, 24 h), YOK1104 (2.5 µM, 5 h), or both and performing immunoblotting analysis on the CHOK1-Z cells,

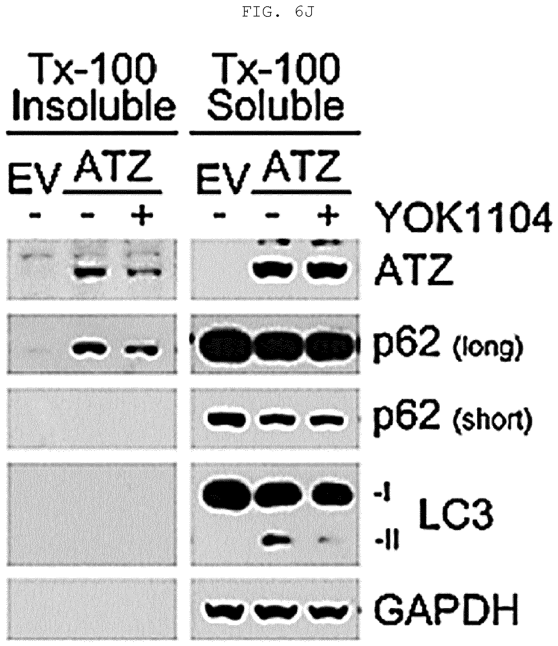

FIG. 6J illustrates the Triton X-100 insoluble/soluble fractionation assay results in HEK293T cells transfected with ATZ and treated with YOK1104 (2.5 µM, 3 h) for immunoblotting analysis,

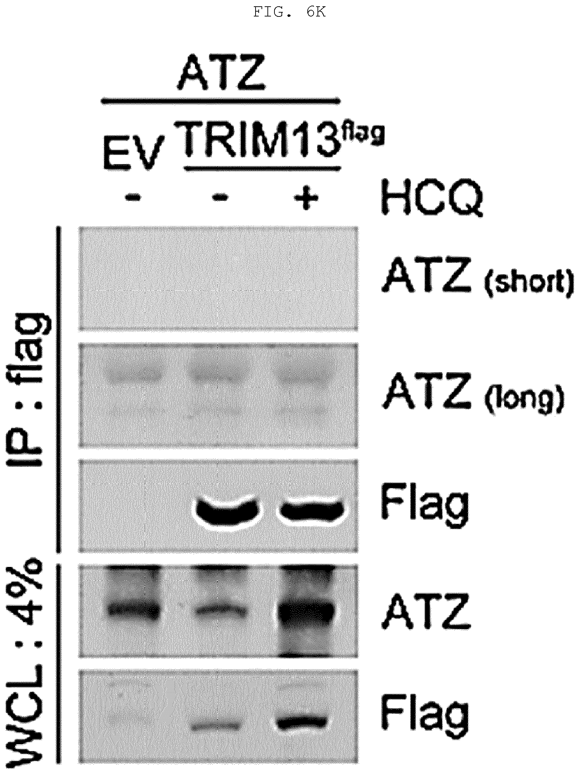

FIG. 6K illustrates the Co-IP assay results in HEK293T cells co-transfected (24 h) with TRIM13-flag and ATZ and then treated with HCQ (10 µM, 24 h), and

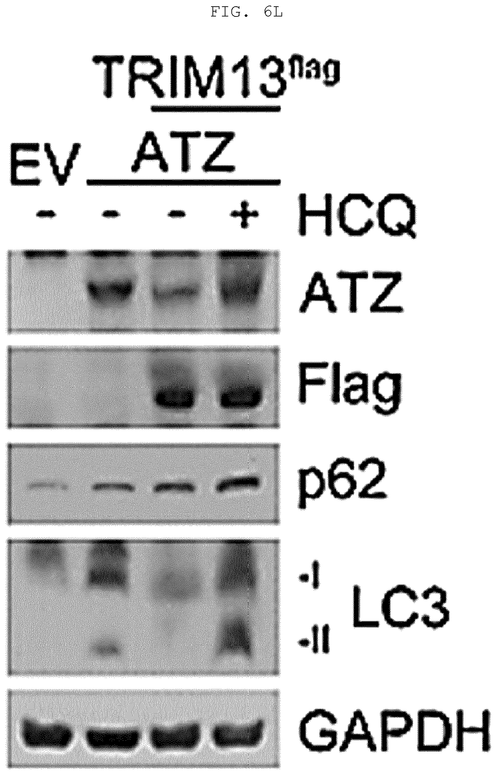

FIG. 6L illustrates the immunoblotting analysis results of HeLa cells co-transfected (24 h) with ATZ and TRIM13-flag and then treated with or without HCQ (10 µM, 24 h) .

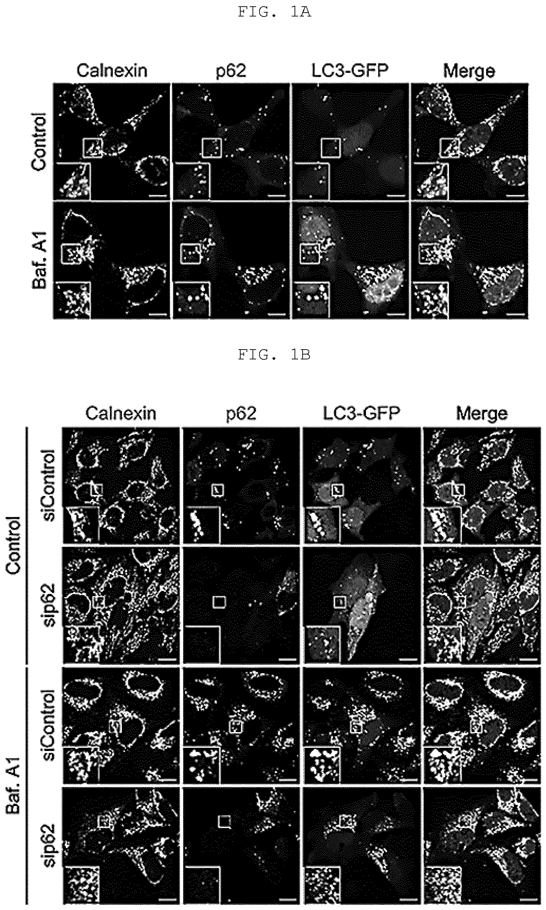

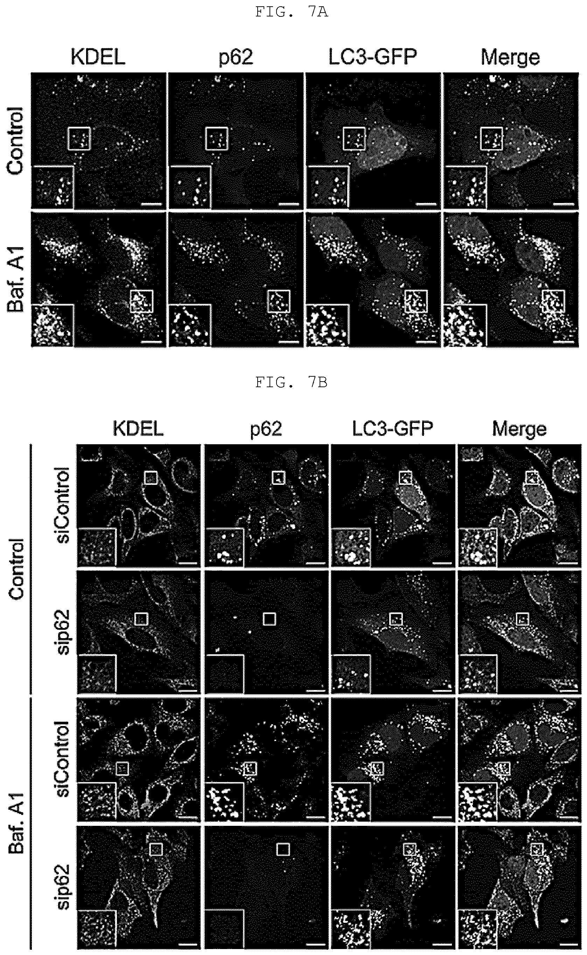

FIGS. 7A to 7I illustrate that p62 targets the ER for autophagy (related to FIGS. 1A to 1K), in which
FIG. 7A illustrates the co-localization analysis results of p62, KDEL and LC3-GFP puncta structures in HeLa cells treated with bafilomycin A1 (200 nM, 6 h) (scale bar, 10 µm),
FIG. 7B illustrates the results acquired by performing the same test as in FIG. 7A except that the test was performed under siRNA-mediated knockdown of p62 (48 h) compared to a control (scale bar, 10 µm),

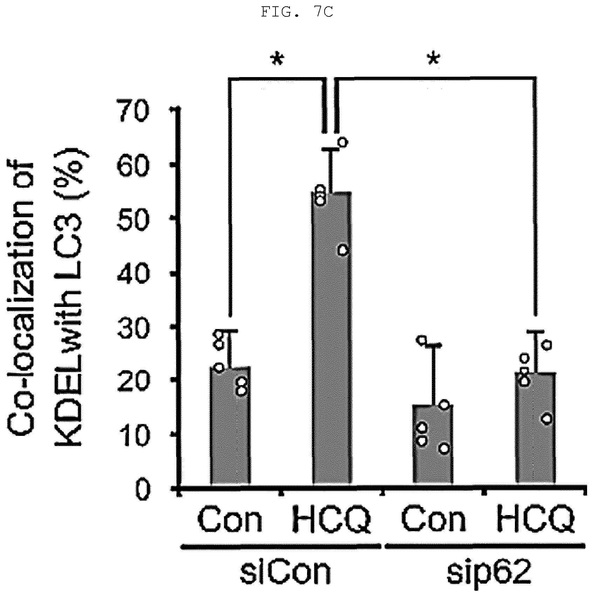

FIG. 7C is a graph illustrating the quantified results of FIG. 7B (n = 50 cells),

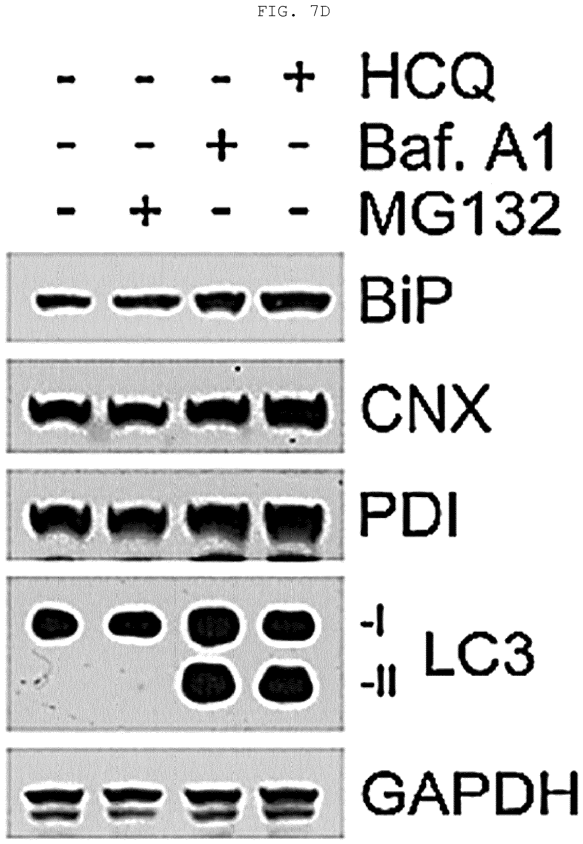

FIG. 7D illustrates the immunoblotting analysis results of HeLa cells treated with MG132 (2 µM, 18 h), bafilomycin A1 (200 nM, 6 h), or HCQ (10 µM, 24 h),

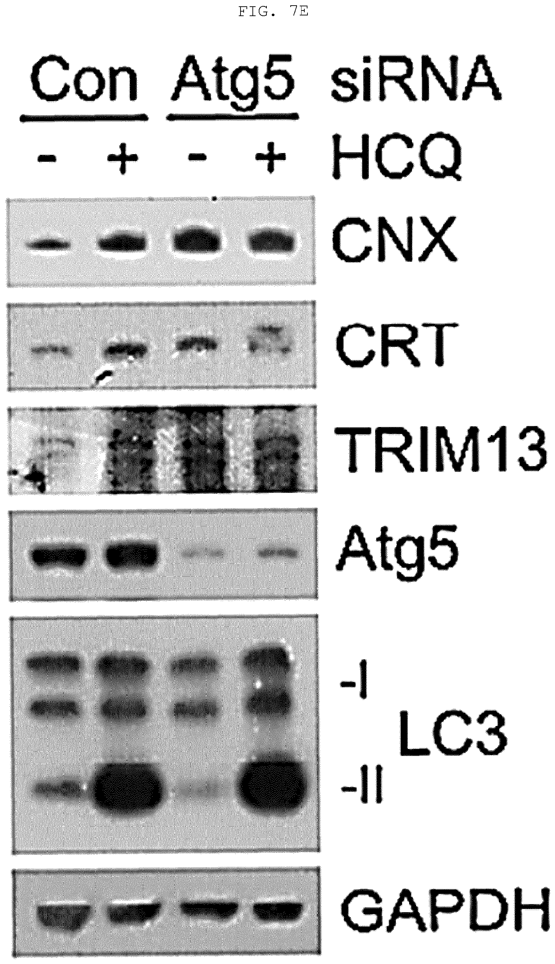

FIG. 7E illustrates the western blotting analysis results of HeLa cells under siRNA-mediated knockdown (48 h) of *ATG5* treated with or without HCQ (10 µM, 24 h) compared to a control,

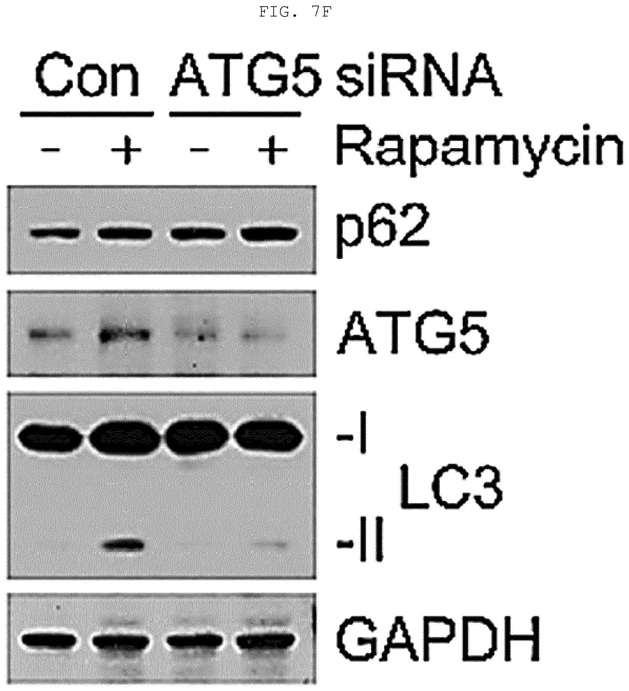

FIG. 7F illustrates the western blotting analysis results of HeLa cells under siRNA-mediated knockdown (48 h) of ATG5 treated with or without Rapamycin (10 µM, 24 h) compared to a control,

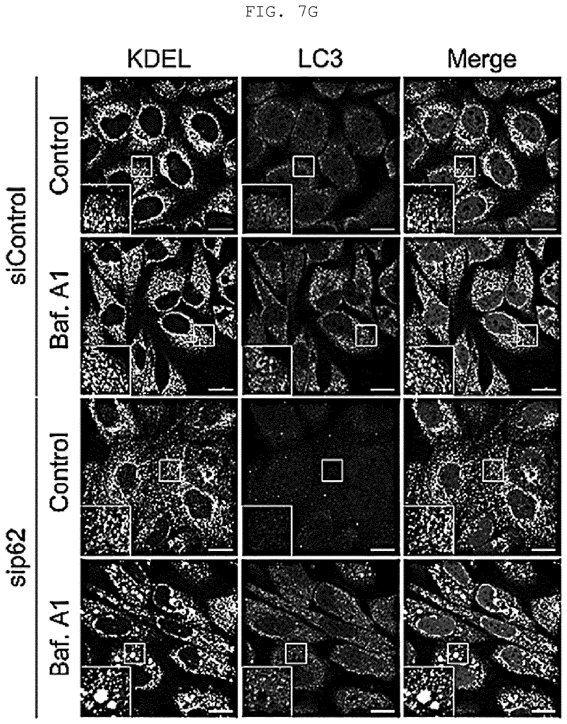

FIG. 7G illustrates the results acquired by performing the same test as in FIG. 7B except that immunostaining for LC3 and KDEL was performed (scale bar, 10 µm),

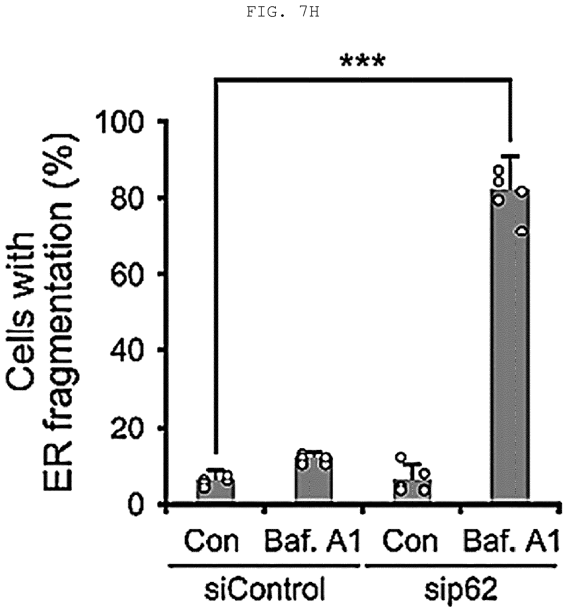

FIG. 7H is a graph illustrating the quantified results of FIG. 7G for ER fragmentation (n = 50 cells), and

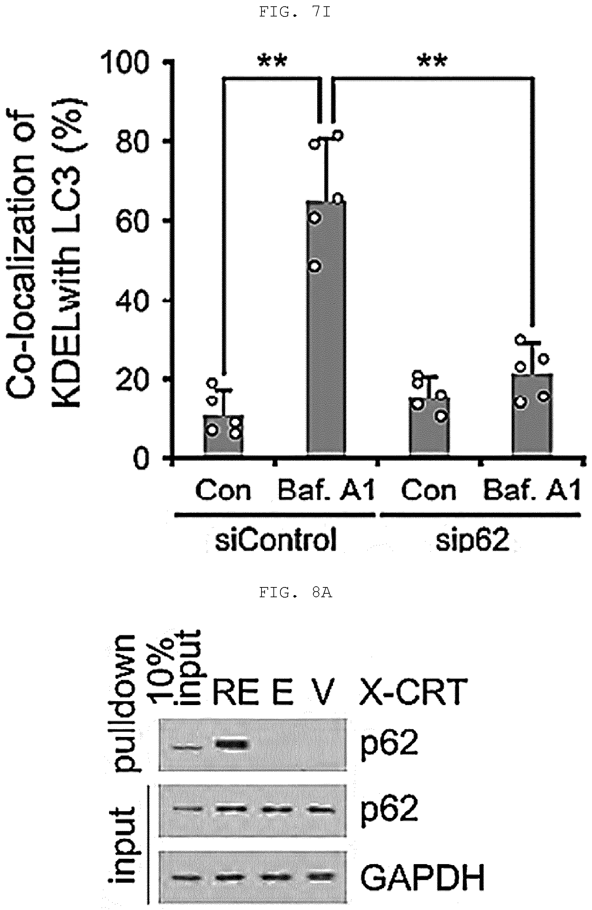

FIG. 7I is a graph illustrating the quantified results of FIG. 7G for co-localization of KDEL and LC3 (n=50 cells).
FIGS. 8A to 8K illustrate that Nt-arginylation is required for autophagic targeting of ER (related to FIGS. 2A to 2N), in which

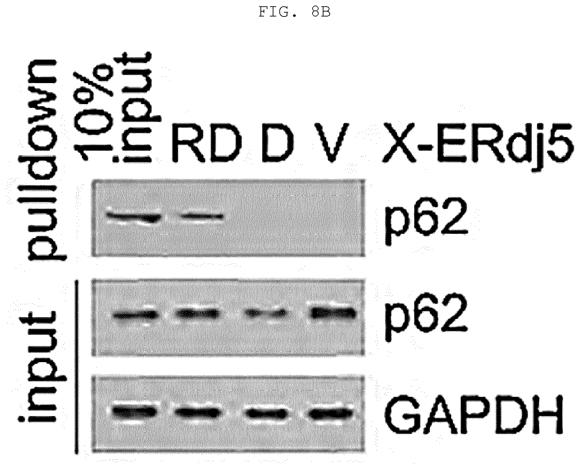

FIGS. 8A and 8B illustrate the *in vitro* pulldown assay results of X-CRT (X = E, V, or RE) or X-ERdj5 (X = D, V, or RD), respectively,

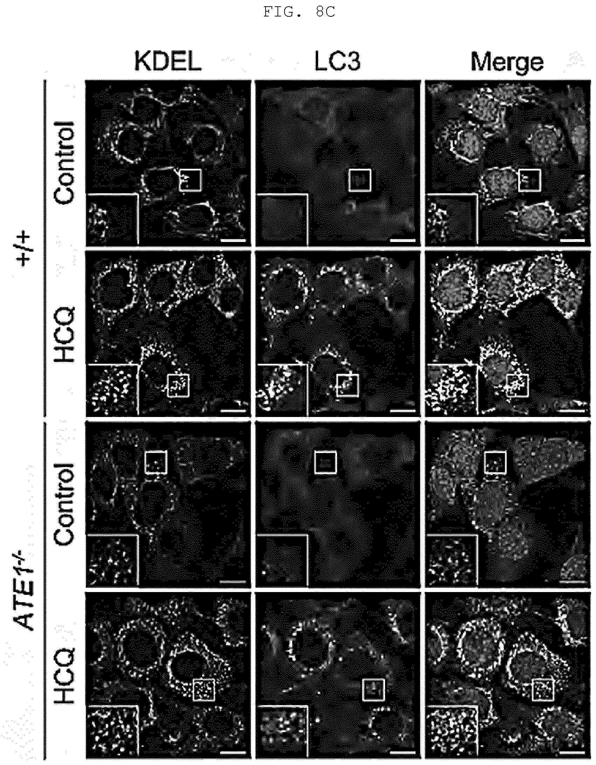

FIG. 8C illustrates the co-localization analysis results of LC3 and KDEL puncta structures in *ATE1*^{*-*/*-*} MEFs and a wild type treated with or without HCQ (10 µM, 24 h) (scale bar, 10 µm),

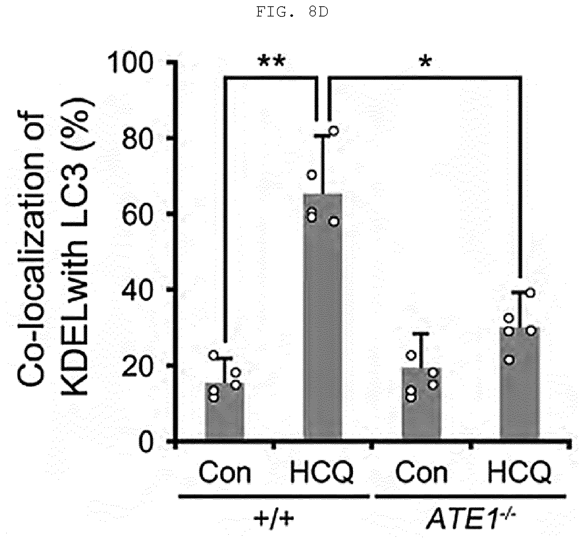

FIG. 8D is a graph illustrating the quantified results of FIG. 8C (n = 50 cells),

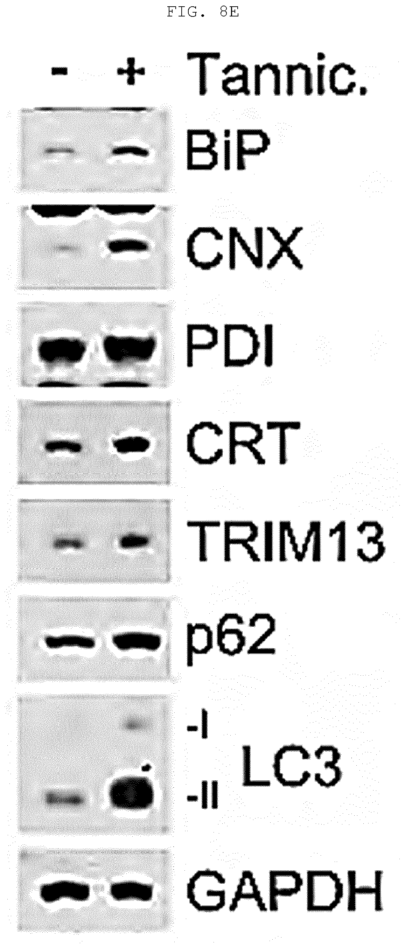

FIG. 8E illustrates the immunoblotting results of ER protein in HeLa cells treated with tannic acid (30 µM, 24 h),

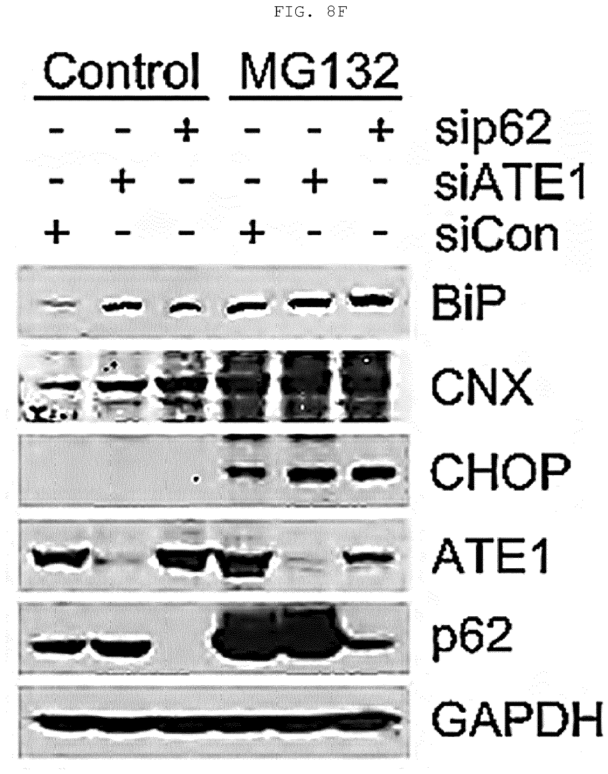

FIG. 8F illustrates the immunoblotting results of ER protein in HeLa cells treated with MG132 (2 µM, 24 h) under siRNA-mediated knockdown (48 h) of *ATE1* compared to a control,

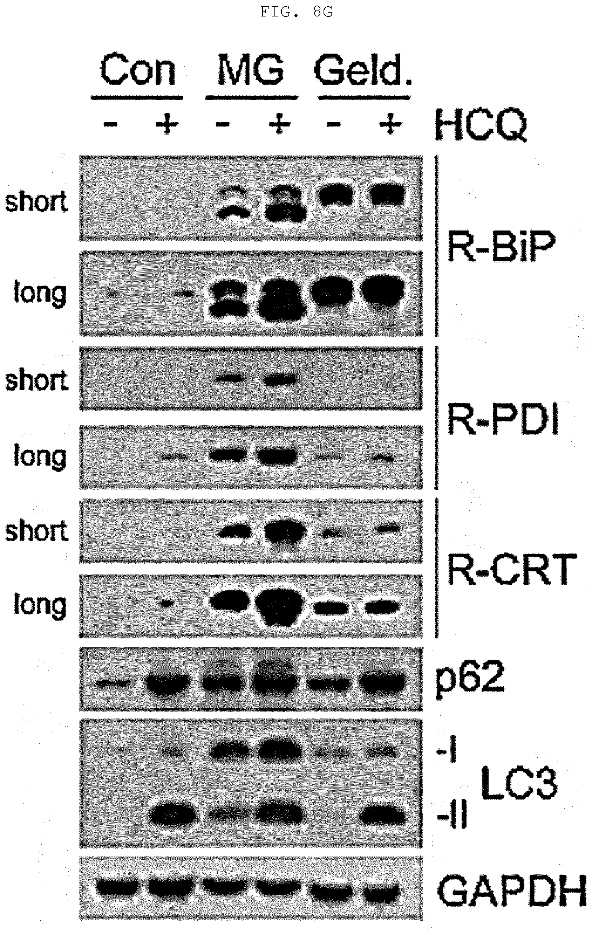

FIG. 8G illustrates the immunoblotting results of Nt-arginylated ER chaperones in HeLa cells treated with MG132 (2 µM, 24 h) or geldanamycin (1 µM, 24 h),

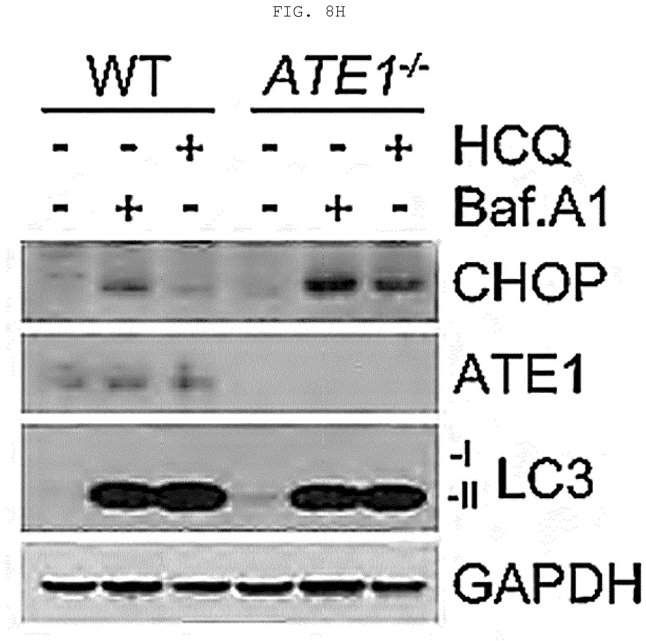

FIG. 8H illustrates the immunoblotting results in *ATE1*^{*-*/*-*} MEFs and a wild type treated with prolonged autophagy inhibition (bafilomycin A1; 200 nM, 24 h or HCQ; 25 µM, 24 h),

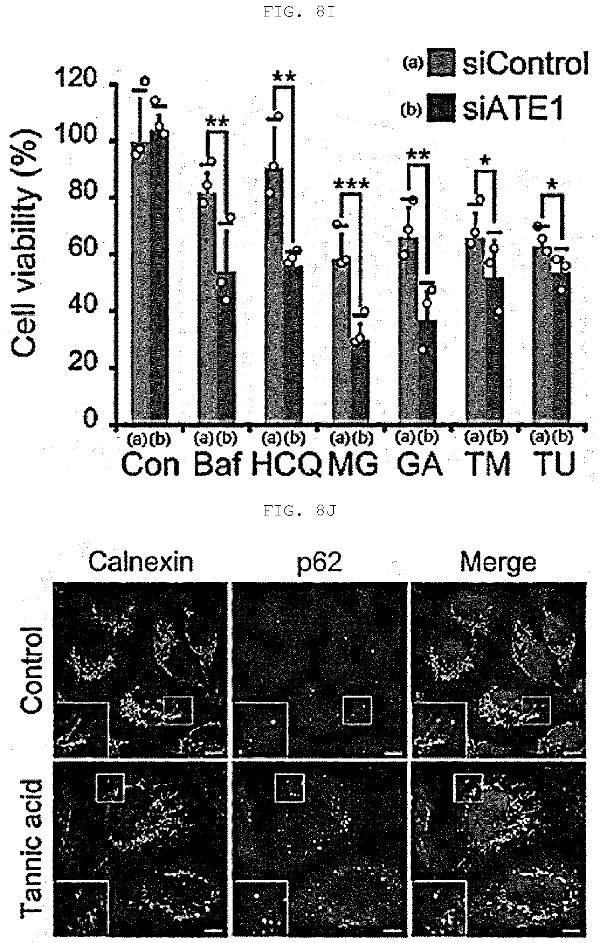

FIG. 8I illustrates the WST-based cell viability assay results of HeLa cells under RNA interference of *ATE1* treated with Baf. A1 (200 nM, 24 h), HCQ (10 µM, 24 h), MG132 (1 µM, 24 h), geldanamycin (1 µM, 24 h), thapsigargin (200 nM, 24 h), or tunicamycin (0.1 µg/mL, 24 h) compared to a control,
FIG. 8J illustrates the co-localization analysis results of p62 and calnexin in HeLa cells treated with tannic acid (30 µM, 24 h) (scale bar, 10 µm), and

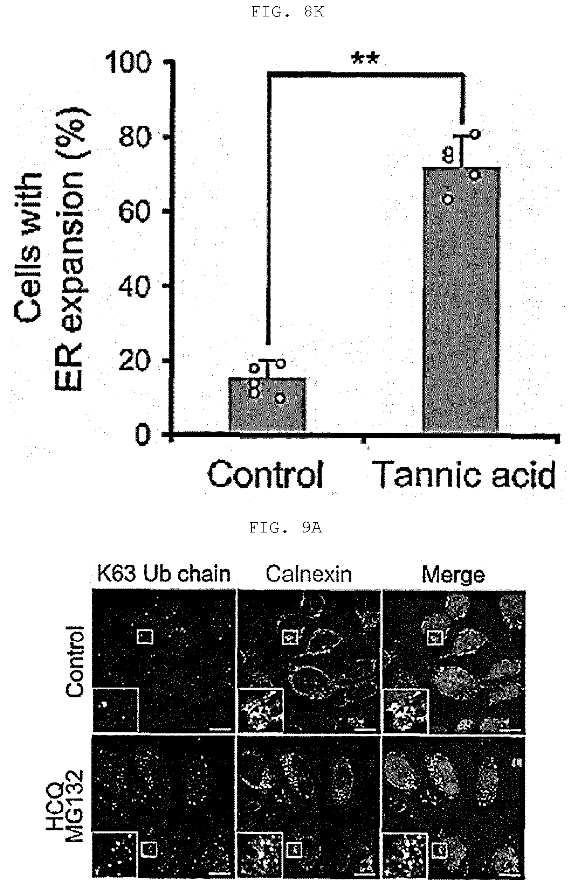

FIG. 8K is a graph illustrating the quantified results of FIG. 8J (n=50 cells).
FIGS. 9A to 9N illustrate that TRIM13 is ubiquitinated and is a receptor for p62 (related to FIGS. 3A to 4O), in which
FIG. 9A illustrates the co-localization analysis results of calnexin and K63-Ub puncta structures in HeLa cells treated with MG132 (2 µM, 18 h) and HCQ (10 µM, 24 h) compared to a case treated with DMSO (scale bar, 10 µm),

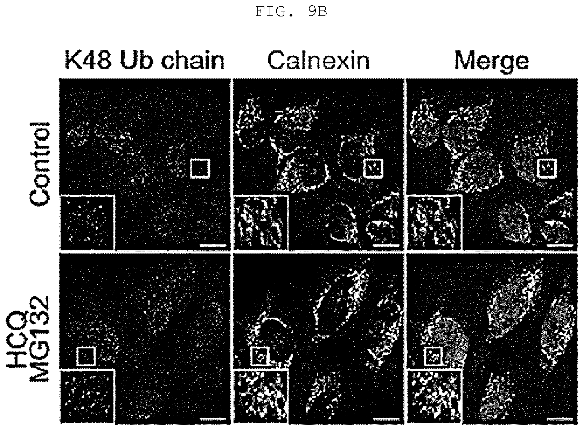

FIG. 9B illustrates the results acquired by performing the same test as in FIG. 9A except that HeLa cells are treated with K48 Ub chain,

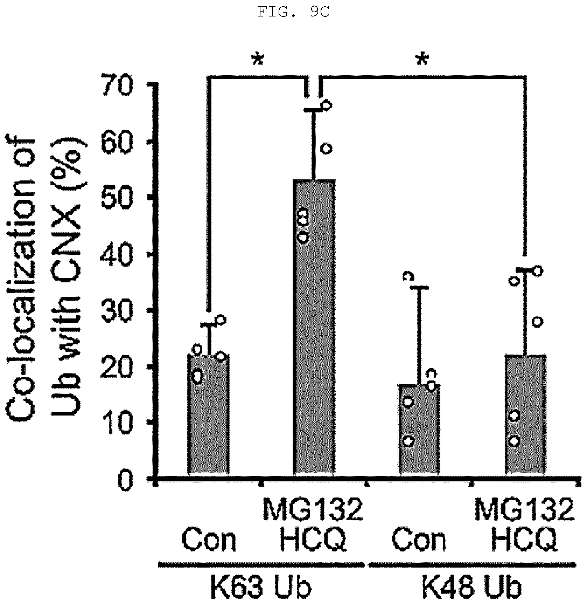

FIG. 9C is a graph illustrating the quantified results of FIGS. 9A and 9B (n = 50 cells),

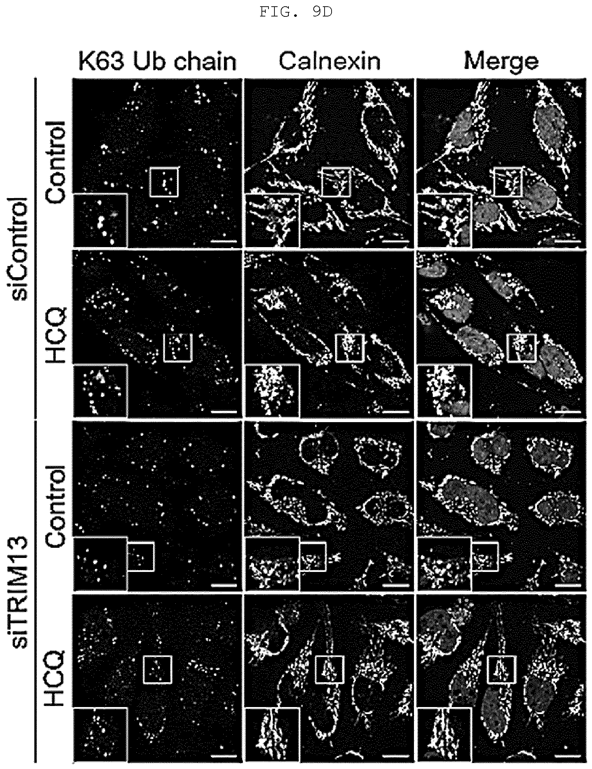

FIG. 9D illustrates the co-localization analysis results of calnexin and K63-Ub puncta structures in HeLa cells treated with or without HCQ (10 µM, 24 h) (scale bar, 10 um),

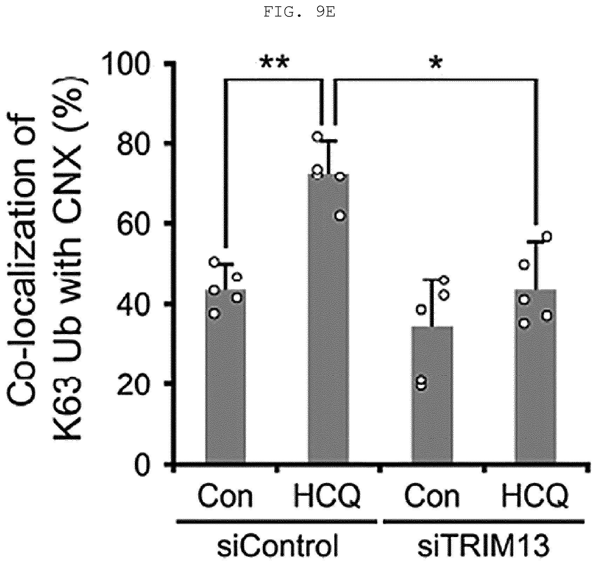

FIG. 9E is a graph illustrating the quantified results of FIG. 9D (n = 50 cells),

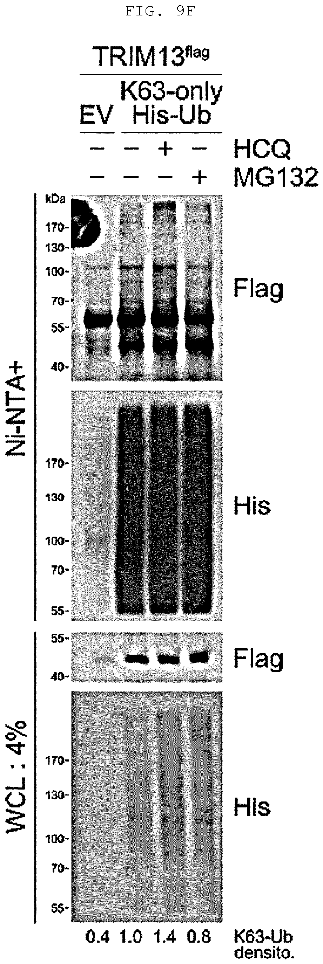

FIG. 9F illustrates the denaturation IP assay results in HEK293T cells co-transfected with TRIM13-flag or negative control empty vector (EV) and K63-only His-Ub and treated with HCQ (10 µM, 24 h) and MG132 (10 µM, 6 h),

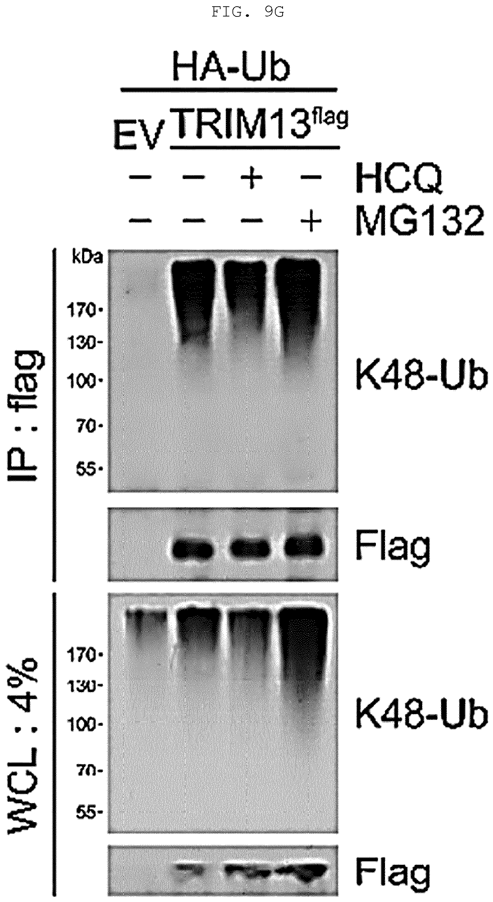

FIG. 9G illustrates the denaturation IP assay results in HEK293T cells co-transfected with full-length TRIM13-flag and HA-Ub and treated with HCQ (10 µM, 24 h), MG132 (10 µM, 6 h) or DMSO negative control,

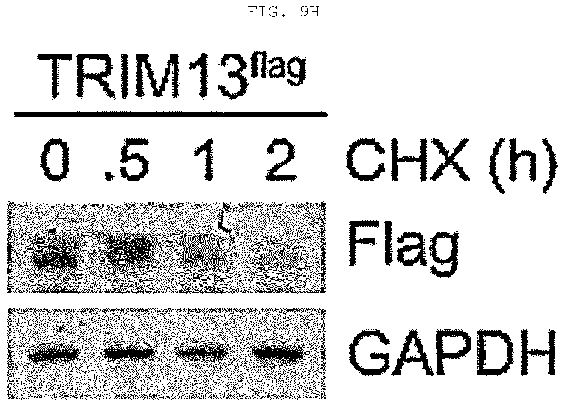

FIG. 9H illustrates the cycloheximide chase assay results in HeLa cells expressing TRIM13-flag (24 h) at the indicated time points,

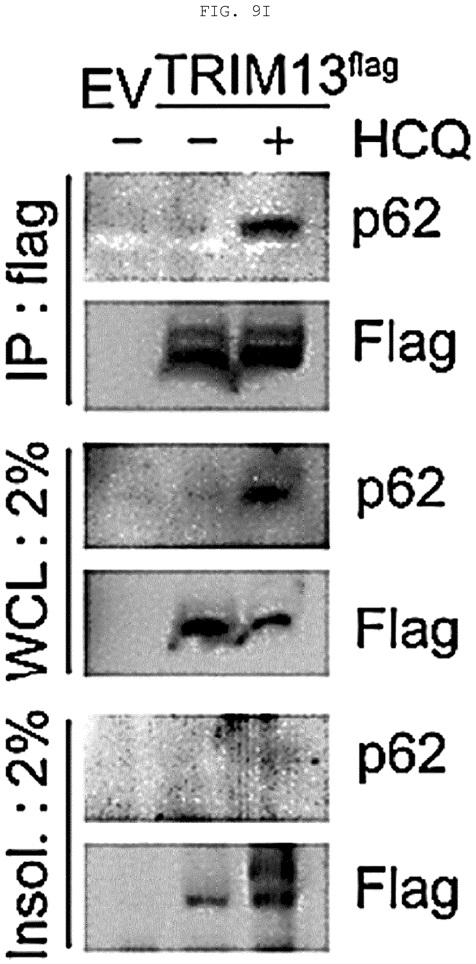

FIG. 9I illustrates the Co-IP assay results in HEK293T cells expressing TRIM13-flag and treated with or without HCQ (10 µM, 24 h),

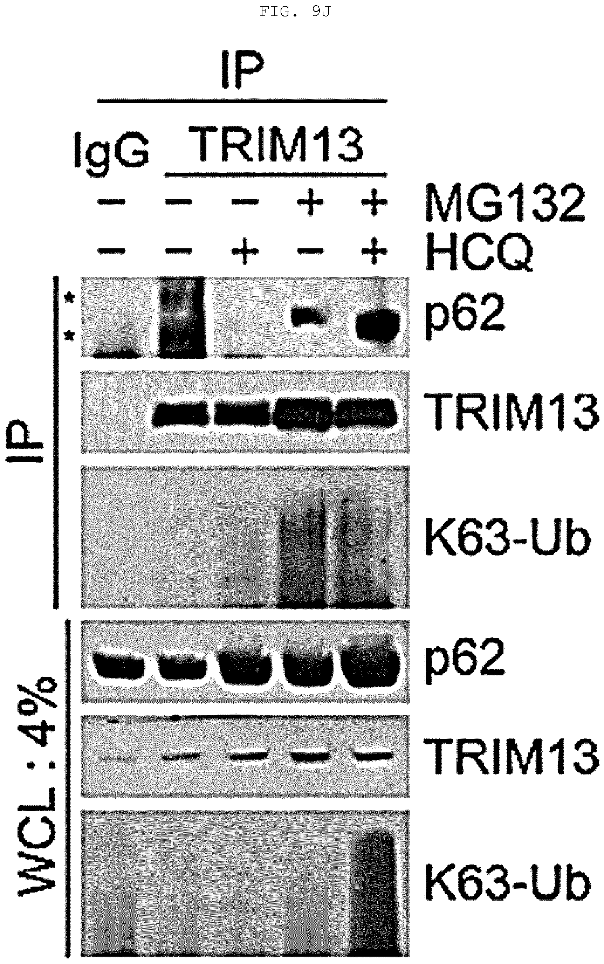

FIG. 9J illustrates the endogenous co-IP assay results in HEK293T cells treated with HCQ (10 µM, 24 h), MG132 (2 µM, 24 h), or both,

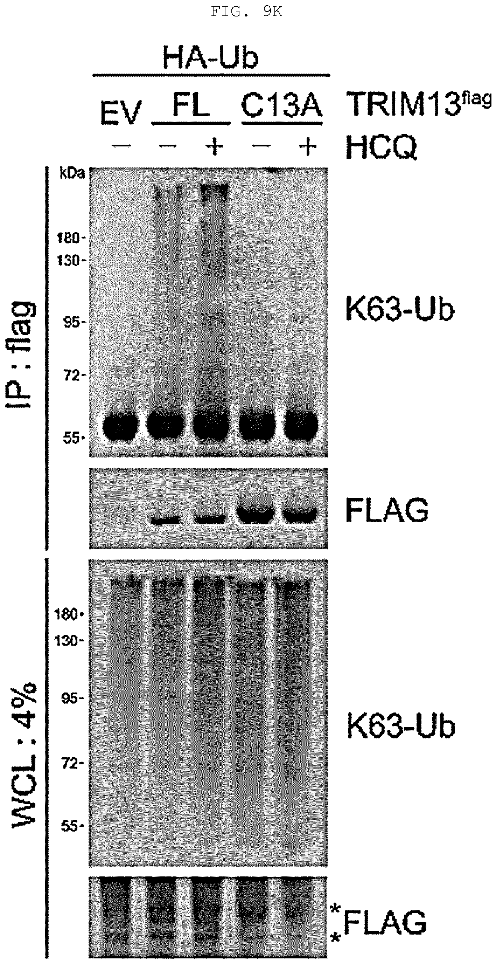

FIG. 9K illustrates the denaturation IP assay results in HEK293T cells co-transfected with a wild type or C13A TRIM13-flag and HA-Ub and treated with HCQ (10 µM, 24 h),

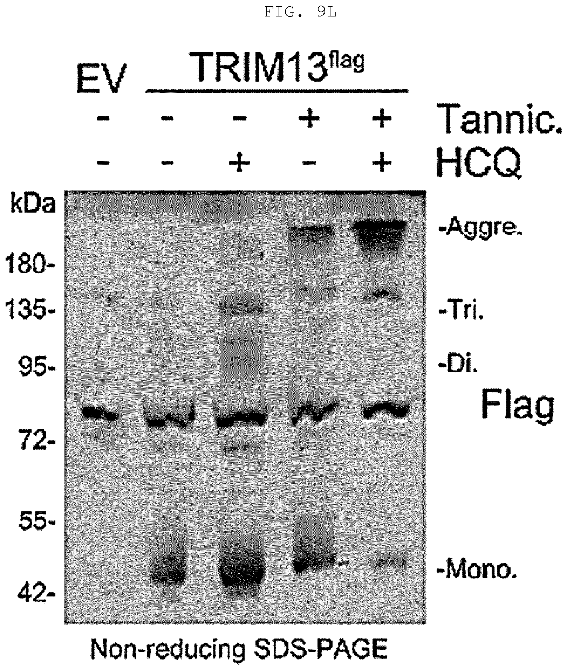

FIG. 9L illustrates the *in vivo* oligomerization assay results of RIM13-flag expressed in HEK293T cells treated with HCQ (10 µM, 24 h), tannic acid (30 µM, 24 h), or both,

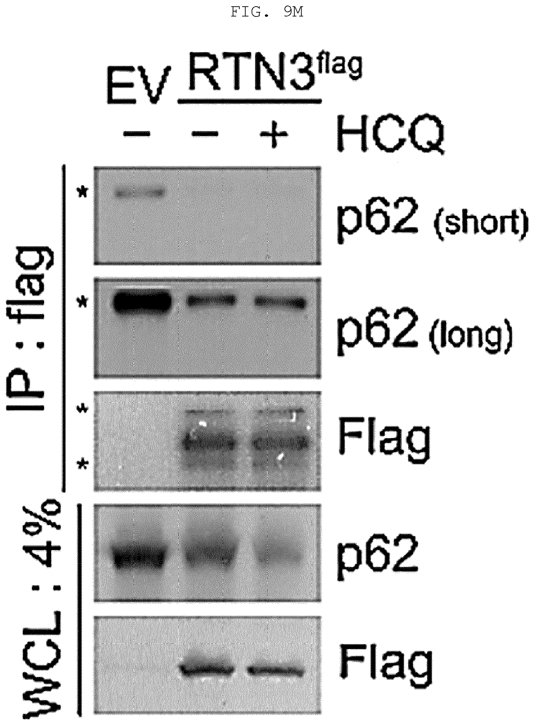

FIG. 9M illustrates the *in vivo* oligomerization assay results of RTN3-flag expressed in the same HEK293T cells as in FIG. 9L, and

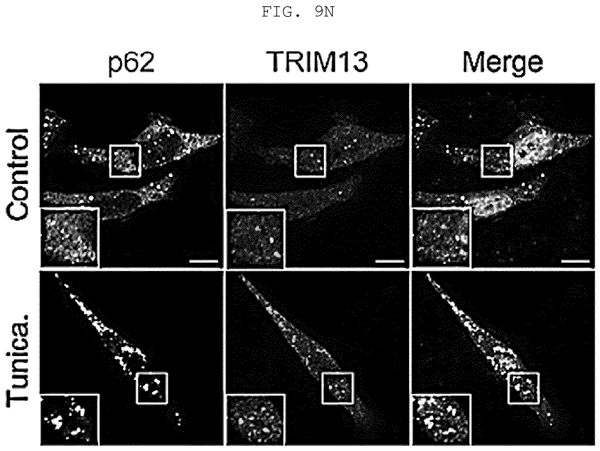

FIG. 9N illustrates the co-localization analysis results of p62 and TRIM13 puncta structures in HeLa cells treated with tunicamycin (0.1 µg/mL, 24 h) or negative control DMSO.

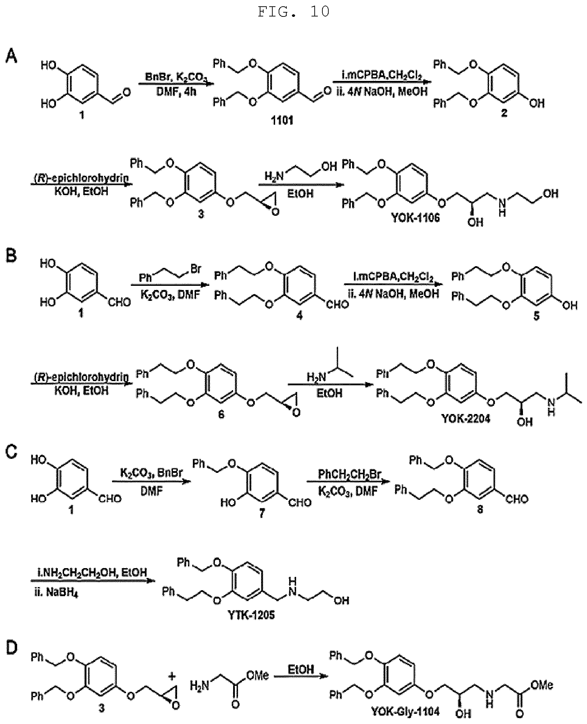

FIGS. 10A, 10B, 10C, and 10D are reaction schemes schematically illustrating the synthesis process of YTK-1205, YOK1106, YOK2204, and YOK-Gly-1104, which are small molecule compound ligands to the p62 ZZ domain.

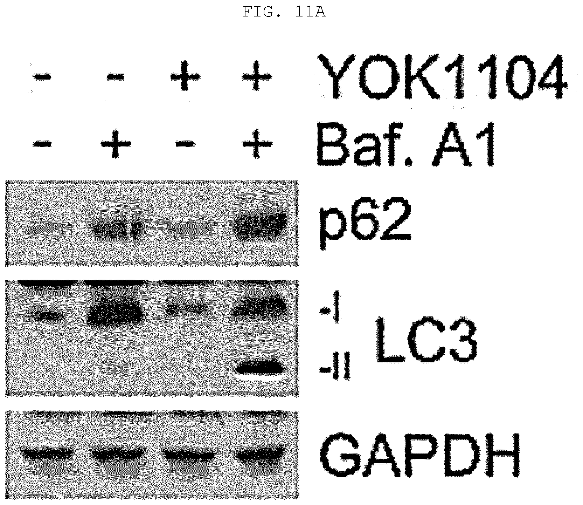

FIGS. 11A to 11O illustrate that p62-ZZ domain ligands induce autophagic targeting of ER, in which

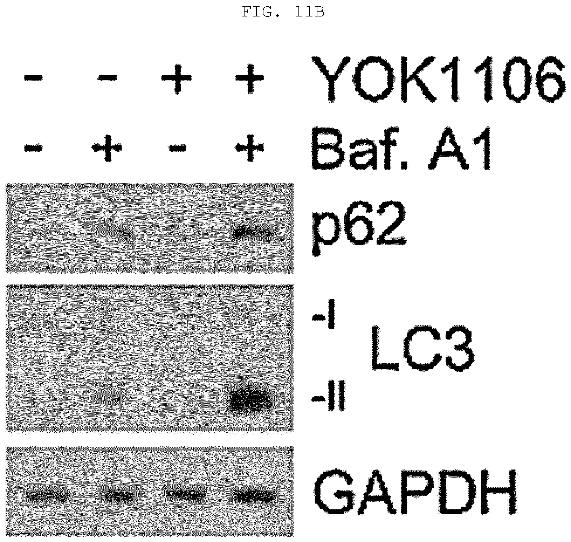

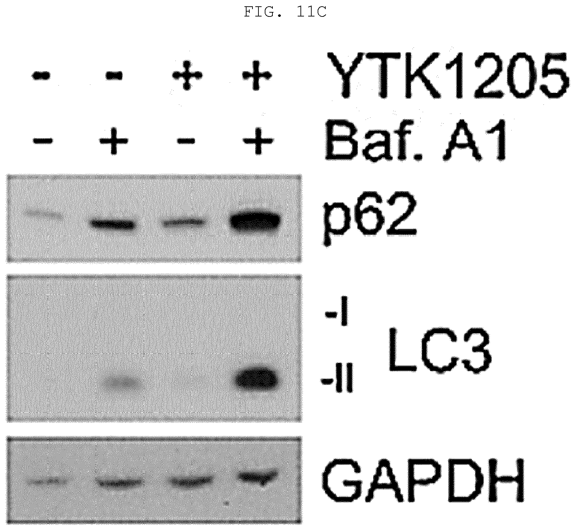

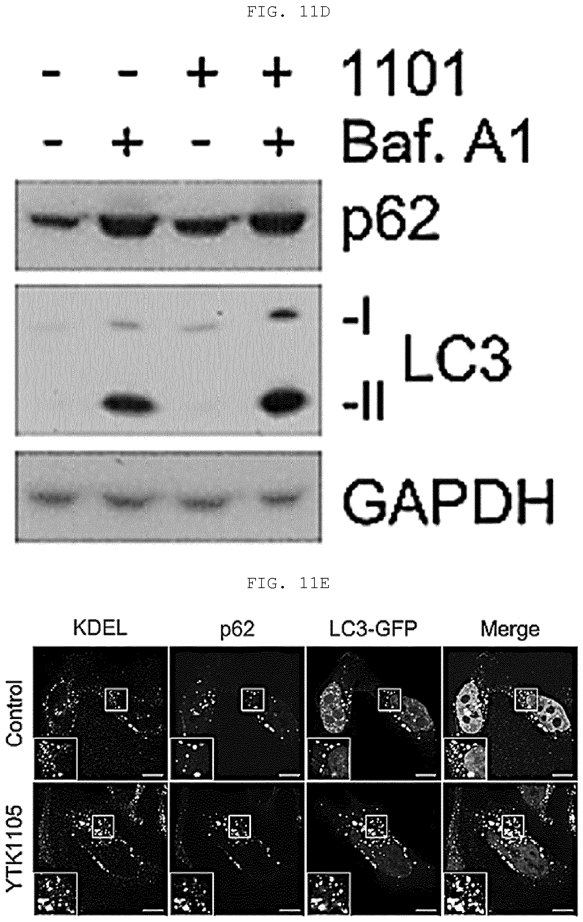

FIGS. 11A, 11B, 11C, and 11D illustrate the immunoblotting analysis results of HEK293T cells treated with YOK1104, YOK1106, YTK1205 or YTK1101 (2.5 µM, 5 h) in the presence or absence of HCQ (10 µM, 24 h),
FIG. 11E illustrates the co-localization analysis results of p62, KDEL and LC3-GFP in HeLa cells transiently transfected with LC3-GFP and treated with YTK1105 (2.5 µM, 5 h) (scale bar, 10 µm),

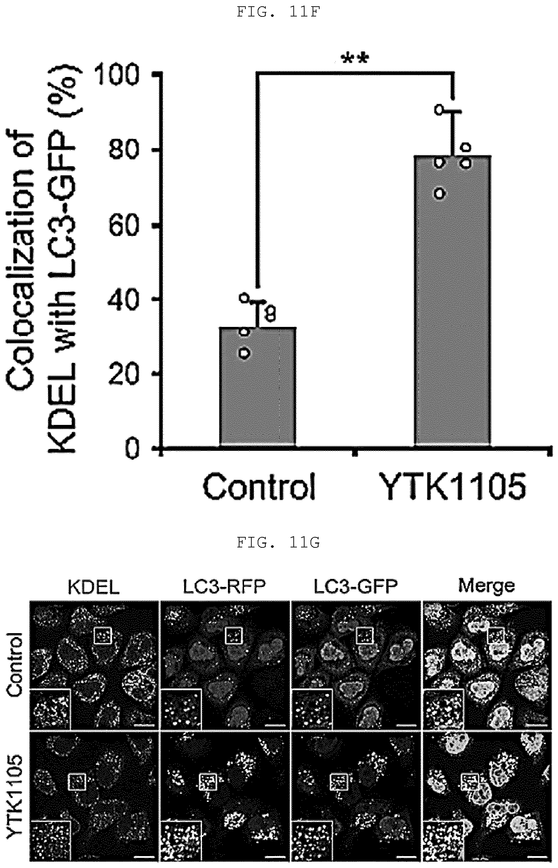

FIG. 11F is a graph illustrating the quantified results of FIG. 11E (n = 50 cells),
FIG. 11G illustrates the co-localization analysis results of stably expressed GFP-LC3, RFP-LC3 and KDEL in the same HeLa cells as in FIG. 11E (scale bar, 10 µm),

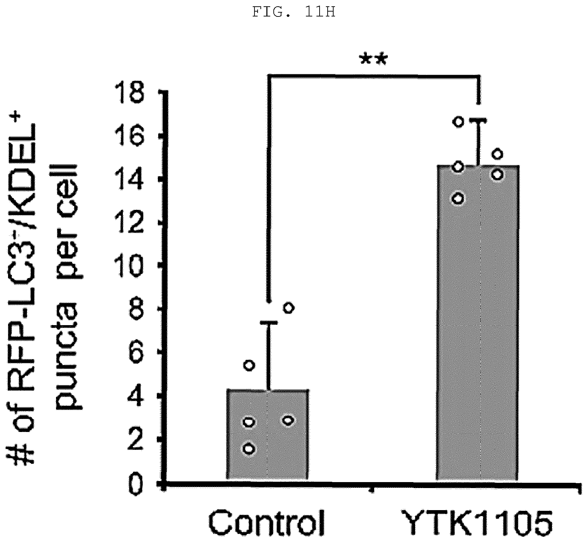

FIG. 11H is a graph illustrating the quantified results of FIG. 11G (n = 40 cells),

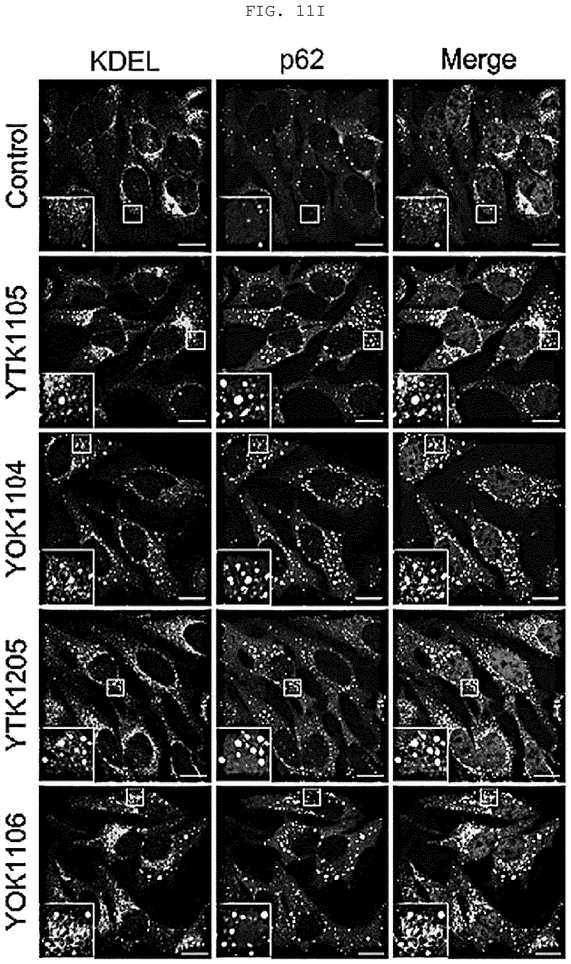

FIG. 11I illustrates the co-localization analysis results of p62 and KDEL in HeLa cells treated with YTK1105 (2.5 µM, 5 h), YOK1104 (2.5 µM, 5 h), YTK2205 (2.5 µM, 5 h), or YOK1106 (2.5 µM, 5 h) (scale bar, 10 µm),

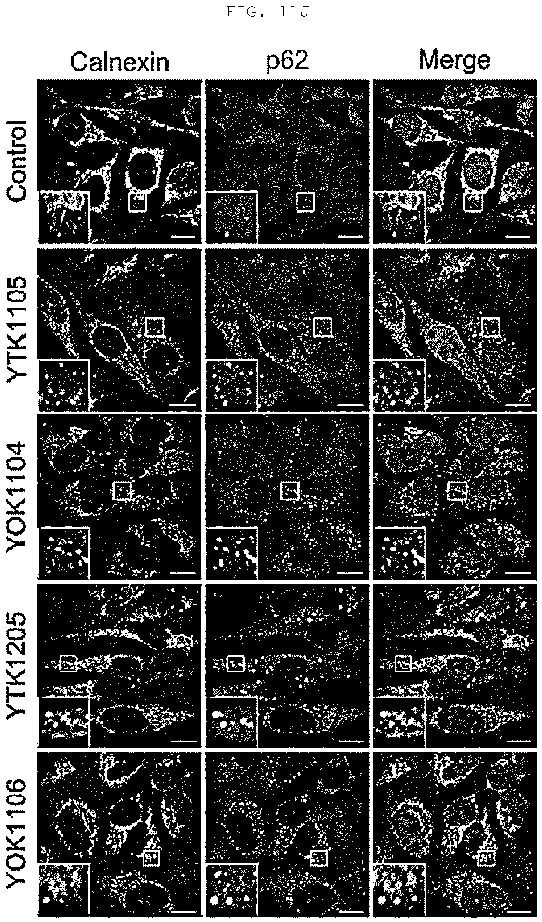

FIG. 11J illustrates the co-localization analysis results of p62 and calnexin in the same cells as in FIG. 11I (scale bar, 10 µm),

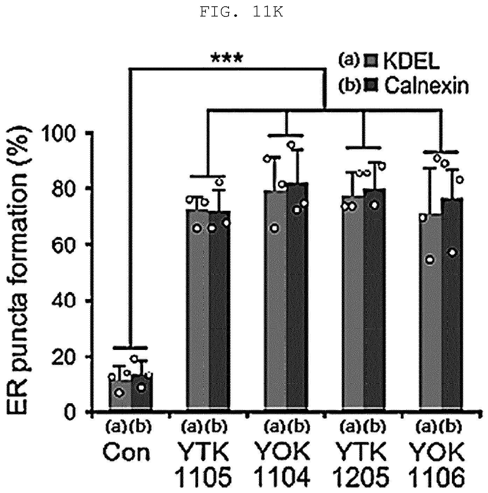

FIG. 11K is a graph illustrating the quantified results of FIGS. 11I and 11J for punctate formation of ER compartments (same as FIG. 11F),

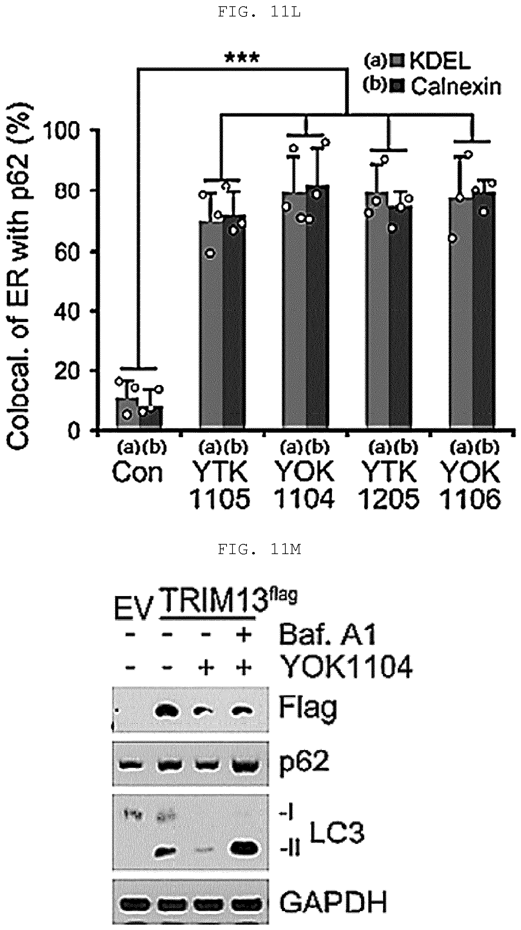

FIG. 11L is a graph illustrating the quantified results of FIGS. 11I and 11J for co-localization of ER compartments with p62,
FIG. 11M illustrates the Western blotting results of HeLa cells transfected with TRIM13-flag or negative control empty vector (EV), treated with YOK1104 (2.5 µM, 4 h), and treated with or without bafilomycin A1 (200 nM, 6 h),

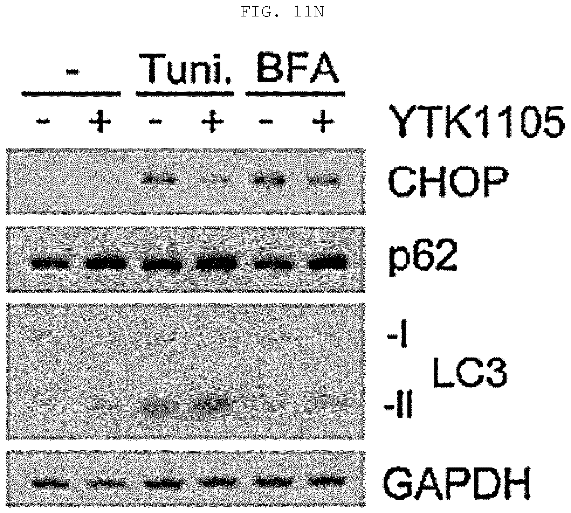

FIG. 11N illustrates the immunoblotting results of HEK293T cells treated with tunicamycin (0.25 µg/mL, 24 h) or brefeldin A (0.3 µM, 24 h) in the presence or absence of YTK1105 (5 µM, 24 h), and

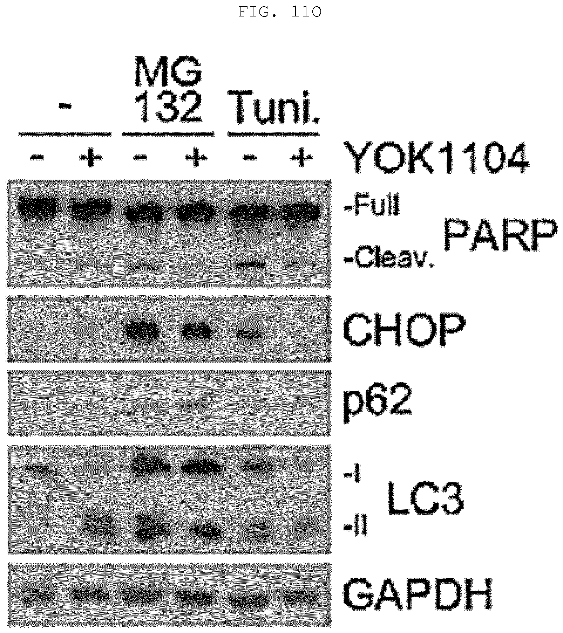

FIG. 11O illustrates the immunoblotting results of HEK293T cells treated with MG132 (1 µM, 24 h) or tunicamycin (0.25 µg/mL, 24 h) in the presence or absence of YOK1104 (5 µM, 24 h).

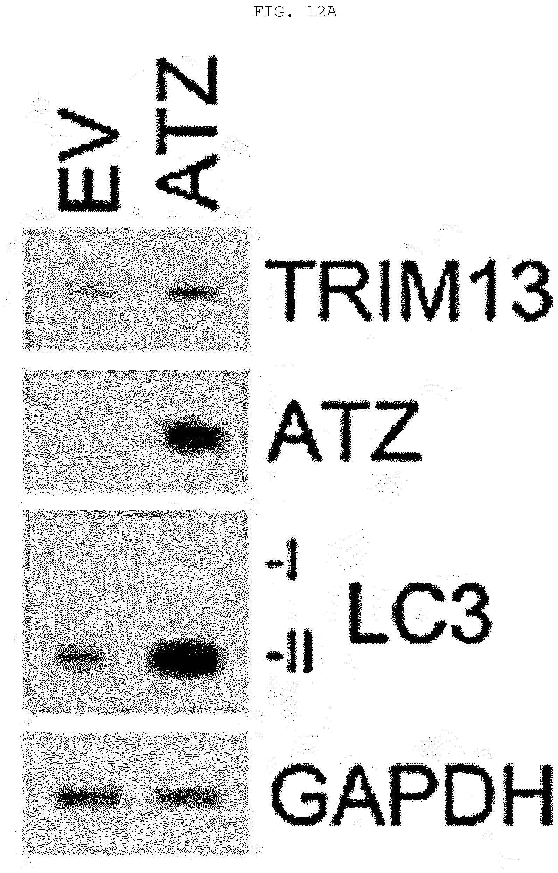

FIGS. 12A to 12H illustrate that p62/Nt-Arg/TRIM13-dependent ER-phagy degrades ATZ, in which
FIG. 12A illustrates the immunoblotting results of HeLa cells ectopically expressing ATZ or an empty vector,

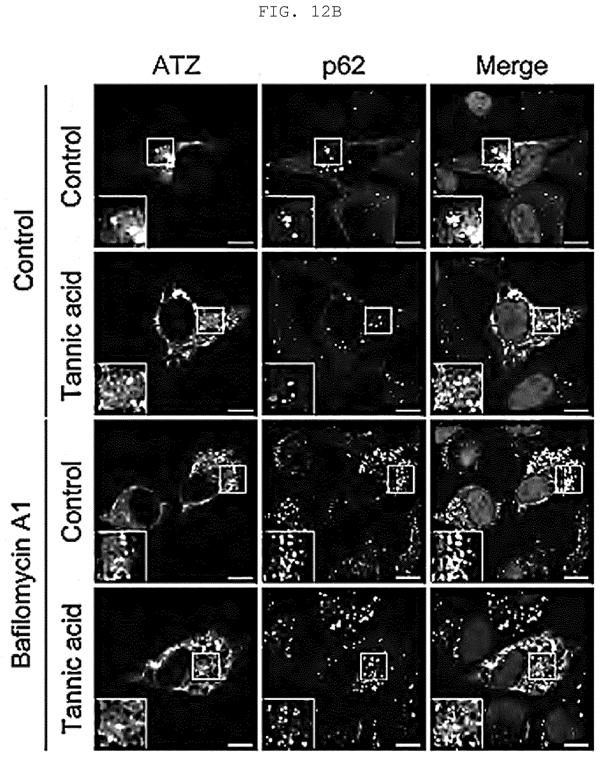

FIG. 12B illustrates the co-localization analysis results of ATZ and p62 in HeLa cells treated with bafilomycin A1 (200 nM, 18 h), tannic acid (30 µM, 24 h), or both (scale bar, 10 µm),

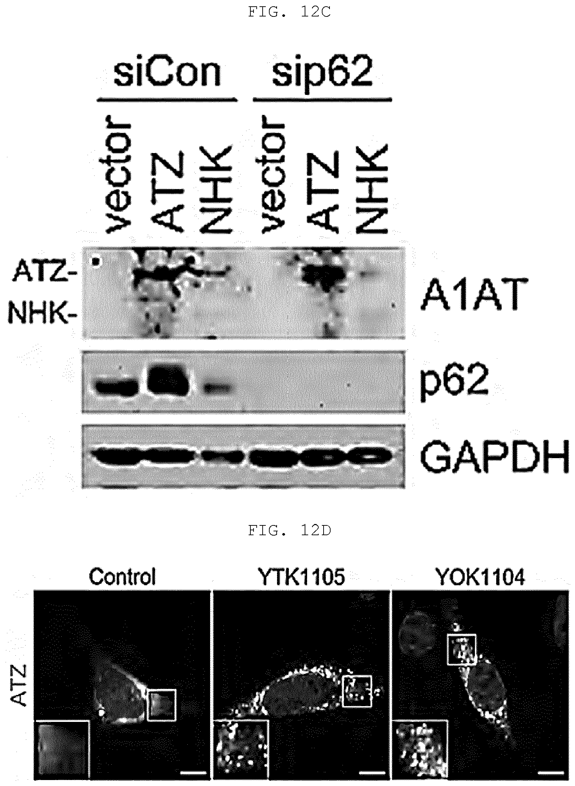

FIG. 12C illustrates the immunoblotting results of ATZ and NHK under siRNA-mediated knockdown (48 h) of control and *p62* in HeLa cells,
FIG. 12D illustrates the co-localization analysis results of ectopic ATZ in HeLa cells treated with YTK1105 (2.5 µM, 5 h) or YTK1104 (2.5 µM, 5 h) (scale bar, 10 µm),

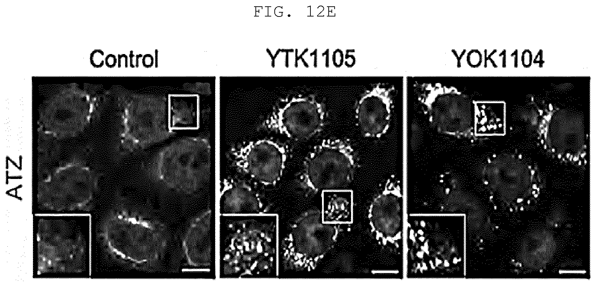

FIG. 12E illustrates the co-localization analysis results of ATZ in CHOK1-Z cells stably expressing ATZ and treated with YTK1105 (2.5 µM, 5 h) or YTK1104 (2.5 µM, 5 h) (scale bar, 10 µm),

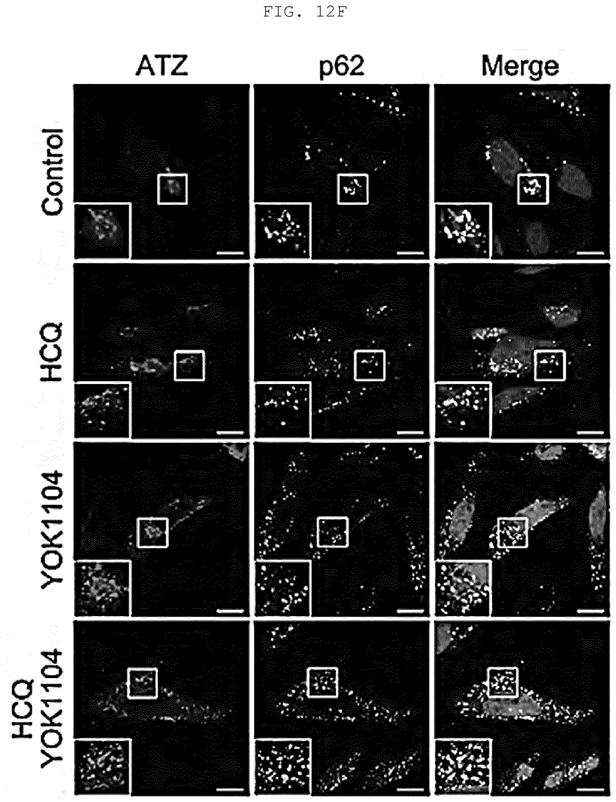

FIG. 12F illustrates the co-localization analysis results of ATZ and p62 in HeLa cells treated with YOK1104 (2.5 µM, 3 h) and then with HCQ (10 µM, 24 h) (scale bar, 10 µm),

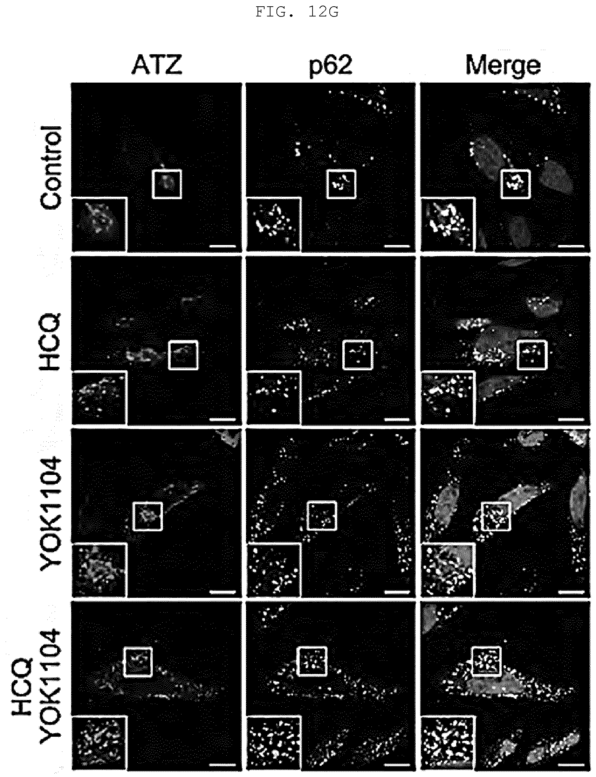

FIG. 12G illustrates the co-localization analysis results of ATZ and LC3 puncta structures in HeLa cells treated with YOK1104 (2.5 µM, 3 h) and then with HCQ (10 µM, 24 h) (scale bar, 10 µm), and

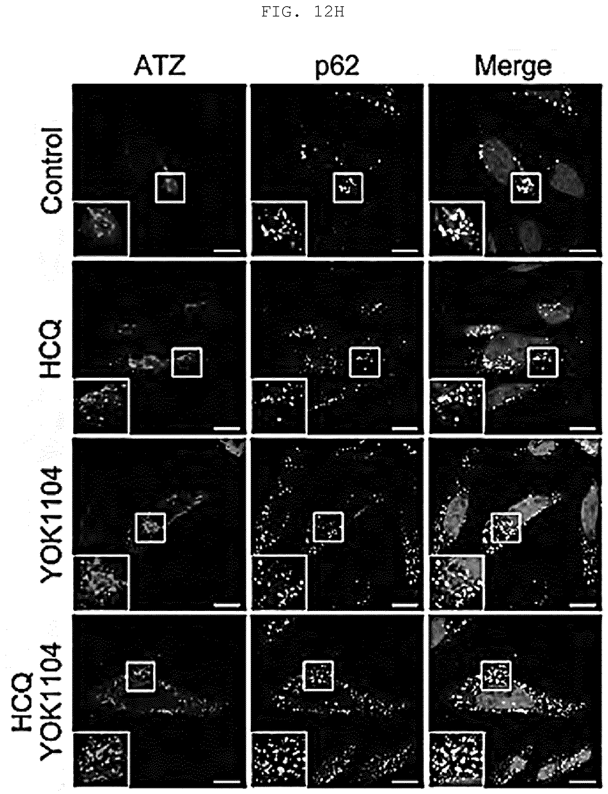

FIG. 12H illustrates the co-localization analysis results of ATZ and KDEL in HeLa cells transiently transfected with ATZ and treated with a p62-ZZ ligand compound (2.5 µM, 5 h) (scale bar, 10 µm).

### [Description of Embodiments]

Hereinafter, the present invention will be described in more detail with reference to the following Examples. However, these are only for illustrating the present invention, and the scope of the present invention is not limited by these Examples.

### Reference Example 1: Cell culture

HeLa, HEK293, HEK293T, +/+, *ATE1*^{*-*/}*⁻, p62*^{*-*/*-*} and *ATG5*^{*-*/-} mouse embryonic fibroblast (MEF), RFP-GFP-LC3 stable HeLa and ATZ-expressing stable CHOK1-Z cell lines were cultured in Dulbecco's Modified Eagle's Medium (DMEM; Gibco) supplemented with 10% Fetal Bovine Serum (FBS; Gibco) and antibiotics (100 units/mL penicillin and 100 µg/mL streptomycin) in a 5% CO₂ incubator. HeLa, HEK293, and HEK293T were obtained from ATCC, and p62 and ATG5 ⁺/⁺ and ⁻/⁻MEF were obtained from RIKEN, Japan. For knockout MEFs and stable cell lines, presence or absence of intended target proteins was confirmed by immunoblotting and/or immunocytochemistry.

### Reference Example 2: Cloning and site-directed mutagenesis

Plasmids for recombinant p62 expression were constructed as follows. PCR amplification of a full-length human p62 cDNA fragment (GenBank Accession No. NM_003900.5; Amino acid sequence of full-length p62: GenBank Accession No. NP_003891.1) obtained from the hMU012675 clone (21C Frontier Human Gene Bank) was followed by subcloning into the pcDNA3.1/*myc*-His plasmids (Thermo Fisher Scientific) at Eco*R*I/Xho*I* sites. The ΔPB1 (PB1 domain deletion) and ΔUBA domain (UBA domain deletion) p62 mutants were generated in an identical manner (site-directed mutagenesis) (see FIG. 4A; PB1 domain: sites aa 1-82 in the amino acid sequence of full-length p62, UBA domain: sites aa 387-436 in p62) .

Site-directed mutagenesis was performed using the QuickChange II XL Site-Directed Mutagenesis Kit according to the manufacturer's instructions (Agilent) to produce amino acid substitutions (Cys at aa 142, 145, 151, and/or 154 was substituted with Ala) in the zinc finger motifs (see FIG. 4A) of the p62-ZZ domain (sites aa 128-163 in p62). Alternatively, full-length p62-GFP and the ΔZZ (ZZ domain deletion) mutant p62-GFP were subcloned as described above but into the pEGFP-N1 plasmid (Clontech) at Eco*R*I/Xho*I* sites.

The plasmids encoding ATE1 R-transferase isoform (GenBank Accession No. NP_001001976.1) were constructed with reference to "Science. 2002 Jul 5;297(5578):96-9". To construct Ub-X-BiP-flag plasmids, corresponding cDNA fragments from Ub-X-BiP-GFP plasmid (Cha-Molstad et al., 2015) were subcloned into HindIII/Bam*H*I site of pcDNA3.1 using PCR amplification.

For recombinant full-length human TRIM13-flag plasmids, HEK293T cells were subjected to total RNA isolation using TRIzol (Thermo Fisher Scientific) followed by reverse transcriptase PCR to obtain full-length human TRIM13 cDNA (GenBank Accession No. NM_005798.5; full-length human TRIM13: GenBank Accession No. NP_005789.2) . This cDNA was then subcloned into Eco*R*I/Xho*I* sites of the pcDNA3.1-3xflag plasmid (Thermo Fisher Scientific). Site-directed mutagenesis was performed using the QuickChange II XL Site-Directed Mutagenesis Kit(Agilent) to induce amino acid substitution (Cys to Ala) at the 13^{th} residue of TRIM13(See Fig. 4A).

### Reference Example 3: Transfection

The plasmids prepared in Reference Example 2 were transfected into HeLa, HEK293 and HEK293T cells using the Lipofectamine 2000 Transfection Reagent according to the manufacturer's instructions (Invitrogen). For transfection into MEFs, Lipofectamine 3000 (Invitrogen) with Plus Reagent was used. siRNAs were transfected into HeLa, HEK293 and HEK293T cells using the Lipofectamine RNAiMAX Transfection Reagent (Invitrogen). For co-transfection of plasmids and siRNAs, Lipofectamine 2000 was used.

### Reference Example 4: Generation of antibodies to arginylated species of E¹⁹-BiP, E¹⁸-CRT and D¹⁸-PDI

Rabbit polyclonal antibodies to the arginylated forms of E¹⁹-BiP, E¹⁸-CRT and D¹⁸-PDI were generated, respectively, using the respective peptide sequences REEEDKKEDVGC, REPAVYFKEQ, and RDAPEEEDHVL (see Cha-Molstad et al., 2015). Briefly, rabbits via a custom service at AbFrontier, Inc. (South Korea) were immunized with the above peptides and boosted with incomplete Freund's adjuvant at 3-week intervals. Rabbit antisera was then purified using immobilized protein A specific to IgG, after which two-step affinity chromatography of negative and then of positive purification was performed. Antibody specificity was confirmed via immunoblotting.

### Reference Example 5: Immunoblotting

Cell pellets were washed with phosphate-buffered saline (PBS) and lysed in an SDS-based sample buffer (277.8 mM Tris-HCI, pH 6.8, 4.4% LDS, 44.4% (v/v) glycerol) with beta-mercaptoethanol. Alternatively, cell pellets or protein supernatants were lysed in a 5X Laemmli sample buffer (SDS-based sample buffer). Using SDS-PAGE, whole cell lysates were separated and transferred onto polyvinylidene difluoride membranes at 100 V for 2 h at 4°C. Subsequently, the membrane was blocked with 4% skim milk in PBS solution for 30 min at room temperature and incubated overnight with primary antibodies (see Reference Example 4), followed by incubation with host-specific HRP-conjugated secondary antibodies (Jackson laboratory) (1:10000 dilution). For signal detection, a mixture of ECL solution (Thermo Fisher Scientific) was applied onto the membrane and captured using X-ray films.

### Reference Example 6: In vitro peptide pulldown assay

A set of synthetic 12-mer peptides corresponding to the N-terminal sequences of the ER chaperones BiP, CRT and ERdj5 following their signal peptide cleavage, was C-terminally biotin-conjugated (by Dr. Jeong Kyu Bang at Korea Research Institute of Bioscience and Biotechnology; South Korea). The X¹⁹-BiP peptide (X-EEDKKEDVGTK-biotin) has Arg-Glu¹⁹ (permanently arginylated), Glu¹⁹ (native), or Val¹⁹ (Asp-to-Val mutant) at the N terminus. The X¹⁹-CRT peptide (X-PAVYFKEQFLK-biotin) has Arg-Glu¹⁹ (permanently arginylated), GlU¹⁹ (native), or Val¹⁹ (Asp-to-Val mutant) at the N terminus. The X⁹⁴⁻ERdj5 peptide (X-QDFYSLLGYSK-biotin) has Arg-Asp⁹⁴ (permanently arginylated), Asp⁹⁴ (native), or Val⁹⁴ (Asp-to-Val mutant) at the N terminus.

To cross-link the above peptides with resin beads, C-terminally biotin-conjugated peptides were mixed with high-capacity streptavidin agarose resin (Thermo Fisher Scientific) at a ratio of 0.5 mg of peptide per 1 mL of settled resin and incubated on a rotor at 4°C overnight. After washing five times with PBS, the peptide-bead conjugates were diluted with PBS at a 1:1 ratio. To prepare protein extracts, cells were collected by centrifugation and lysed by freezing and thawing at least 10 times in a hypotonic buffer [10 mM KCl, 1.5 mM MgCl2, and 10 mM Hepes (pH 7.9)] with a protease inhibitor mix (Sigma). After centrifugation at 14,300 × g at 4°C for 15 min, proteins were quantified using a BCA protein assay kit (Thermo Fisher Scientific). The total protein (200 µg) diluted in 300 µL of binding buffer [0.05% Tween-20, 10% glycerol, 0.2 M KCl, and 20 mM Hepes (pH 7.9)] were mixed with 50 µL of peptide-bead resin and incubated on a rotor at 4°C for 2 h. The protein-bound beads were collected by centrifugation at 2,400 × g for 3 min and washed five times with a binding buffer. The beads were resuspended in 25 µL of SDS sample buffer, heated at 95°C for 5 min, and subjected to SDS/PAGE and immunoblotting.

### Reference Example 7: Chemical synthesis and analysis of Nt-Arg-mimicking compounds

Ligand compounds to the p62-ZZ domain, YTK1105, YOK1104, YTK1205, YOK2204 and YOK1106, as well as the negative control ligands 1101 and YOK-Gly-1104 were synthesized as follows.

### 7.1 Synthesis of 3,4-bis(benzyloxy)benzaldehyde 1101

To a stirred solution of 3,4-dihydroxybenzaldehyde 1 (1.00 g, 7.25 mmol) in dry DMF (10 mL) was added anhydrous K₂CO₃ (5.00 g, 36.23 mmol), followed by benzyl bromide (2.1 mL, 18.11 mmol). The resulting mixture was stirred at room temperature for 2 h. Additional K₂CO₃ (2.4 g, 17.3 mmol) was added, and the mixture was heated at 70°C for 30 min and then cooled to room temperature. The mixture was partitioned between H₂O and ether (120 mL each). The organic layer was separated, and the water layer was extracted with ether (3 × 50 mL) . The pooled organic layers were washed with H₂O (2 × 50 mL) and saturated aqueous NaCl solution (50 mL). The combined organic layers were dried over anhydrous NaSO₄ and the solvent was evaporated in *vacuo.* The resulting residue was purified by silica gel column chromatography using hexane/ethyl acetate (7:3) to afford 3,4-bis(benzyloxy)benzaldehyde **1101** (2.19 g, 95%) as a cream-colored solid. ¹H NMR (CDCl₃) δ 9.81 (s, 1 H), 7.54-7.30 (m, 12 H), 7.04 (1H, d, *J* = 9.0 Hz), 5.27 (s, 2 H), 5.23 (s, 2 H); ESIMS m/z: 319.3[M+H]+.

### 7.2 Synthesis of 2-((3,4-bis(benzyloxy)benzyl)amino)ethan-1-ol hydrochloride YTK-1105

In dry ethanol (20 mL), 3,4-bis(benzyloxy)benzaldehyde **1101** (3.18 g, 10 mmol) was dissolved, and ethanolamine (0.61 g, 10 mmol) was added. The reaction mixture was stirred for 12 h at 60°C. The reaction solution was cooled to room temperature. NaBH₄ (0.57 g, 15 mmol) was added slowly, and the resulting solution was stirred for another 12 h. The solvent was evaporated in *vacuo,* and the residue was dissolved in water and extracted with ethyl acetate. The organic layers were combined and dried over anhydrous Na₂SO₄, filtered, and evaporated *in vacuo.* The residue was purified by flash column chromatography to generate the desired product 2-((3,4-bis(benzyloxy)benzyl)amino)ethan-1-ol (2.0g, 56%). ¹H NMR (400 MHz, CDCl₃) : *δ* 7.52-7.33 (m, 10H), 7.01-6.84 (m, 3H), 5.20 (s, 2H), 5.17 (s, 2H), 3.71 (s, 2H), 3.64 (t, *J*=4.8, 2H), 2.93 (s, 2H), 2.72 (t, *J*=4.8, 2H).

In absolute methanol (25 mL), 2-((3,4-bis(benzyloxy)benzyl)amino)ethan-1-ol (1.0 g, 2.75 mmol) was dissolved, and HCl gas was pumped for 1 h. The resulting mixture was stirred for another 2 h and evaporated to about 1 mL, and hexane was added to afford a solid, which was filtered and dried to afford the final compound 2-(3,4-bis(benzyloxy)benzyl)amino)ethan-1-ol hydrochloride **YTK-1105** (720 mg, 65%). ¹H NMR (400 MHz, DMSO-d6): δ 8.83 (bs, 2H), 7.52-7.46 (m, 5H), 7.31-7.32 (m, 1H), 7.26-7.20 (m, 5H), 7.12-7.10 (m, 1H), 7.05-7.03 (m, 1H), 5.24-5.22 (m, 1H), 5.14 (s, 2H), 5.11 (s, 2H), 4.07 (s, 2H), 3.67-3.63 (m, 2H), 2.90 (s, 2H). ¹³C NMR (400MHz, CDCl₃): *δ* 149.0, 148.2, 137.4, 133.3, 128.5, 127.8, 127.5, 127.4, 121.3, 115.4, 115.2, 71.5, 71.3, 60.8, 53.2, 50.7. LC-MS (ESI): m/z 364.3 [M+H]+.

### 7.3 Synthesis of 3,4-bis(benzyloxy)phenol 2

m-Chloroperbenzoic acid (0.78 g, 4.5 mmol) was added to a stirred solution of the 3,4-bis(benzyloxy)benzaldehyde **1101** (1 g, 3.0 mmol) in dichloromethane (15 mL), and the resulting mixture was stirred at room temperature for 4 h and then diluted with ethyl acetate. The organic solution was successively washed with saturated aqueous Na₂CO₃ solution and brine. The solvent was evaporated in *vacuo* to afford corresponding formate. NaOH (6 N) was added to a stirred solution of crude formate in MeOH (15 mL). After stirring at room temperature for 30 min, was added 10% aqueous HCl solution. The obtained reaction mixture was diluted with ethyl acetate (50 mL), washed with brine and dried over anhydrous Na₂SO₄. The flash chromatography (7:3 hexane/ethyl acetate) was performed to afford 3,4-bis(benzyloxy)phenol **2** (0.83 g, 86% (for 2 steps)) as a solid. ¹H-NMR (CDCl₃, 300 MHz): *δ* 7.25-7.42 (m, 10H), 6.80 (d, 1H, *J* = 9.0 Hz), 6.48 (d, 1H, *J* = 3.0 Hz), 6.29 (dd, 1H, *J* = 3.0 and 9.0 Hz), 5.08 (d, 4H, *J* = 15 Hz), 4.55 (s, 1H); ESIMS m/z: 307.25 [M+H]+.

### 7.4 Synthesis of 2-((3,4-bis(benzyloxy)phenoxy)methyl)oxirane 3

To a mixture of 3,4-dibenzyloxy phenol **2** (100 mg, 0.33 mmol) in ethyl alcohol (5 mL), aqueous potassium hydroxide solution (22 mg, 0.40 mmol, 1 mL water) and (R)-epichlorohydrin (41 µL, 0.50 mmol) were added. The resulting mixture was stirred for 15 h at room temperature. The solvent was removed under reduced pressure, and the residue was dissolved in water and extracted with ethyl acetate. The organic extract was washed with brine and dried over Na₂SO₄. The solvent was evaporated to afford a crude product, which was purified by column chromatography using hexane/ethyl acetate (7:3) to afford 2-((3,4-bis(benzyloxy)phenoxy)methyl)oxirane **3** (83 mg, 70%) as a white solid.

### 7.5 Synthesis of (R)-1-(3,4-bis(benzyloxy)phenoxy)-3-(isopropylamino)propan-2-ol YOK-1104

To a solution of 2-((3,4-bis(benzyloxy)phenoxy)methyl)oxirane **3** (15 mg, 0.004 mmol) in MeOH (2 mL) was added isopropylamine (0.21 mL, 2.6 mmol), and the resulting mixture was vigorously stirred at room temperature for 4 h (TLC-monitoring). Then, the solvent was removed under reduced pressure. The resulting residue was extracted with CH₂Cl₂ (3×10 mL). The combined organic layers were washed with brine (10 mL), dried over anhydrous Na₂SO₄ and concentrated under reduced pressure. The obtained crude product was purified by column chromatography (CH₂Cl₂/MeOH, 10:1) to afford pure (R)-1-(3,4-bis(benzyloxy)phenoxy)-3-(isopropylamino)propan-2-ol **YOK-1104** (72 mg, 86%) as a white powder and confirmed with ESIMS m/z: 423.5 [M+H]+.

### 7.6 Synthesis of (R)-1-(3,4-bis(benzyloxy)phenoxy)-3-((2-hydroxyethyl)amino)propan-2-ol YOK-R-1106

To a stirred solution of epoxide 3 (40 mg, 0.11 mmol) in EtOH (2 mL) was added isopropylamine (18 µL, 0.22 mmol), and the mixture was vigorously stirred at room temperature for 4 h (TLC-monitoring). Then, the solvent was removed under reduced pressure. The resulting residue was extracted with CH₂Cl₂ (3×10 mL). The combined organic layers were washed with brine (10 mL), dried over anhydrous Na₂SO₄ and concentrated under reduced pressure. The obtained crude product was purified by column chromatography (CH₂Cl_{2/}MeOH, 10:1) to afford pure (R)-1-(3,4-bis(benzyloxy)phenoxy)-3-((2-hydroxyethyl)amino)propan-2-ol **YOK-R-1106** (41 mg, 86%) as a white powder and confirmed with ESIMS m/z: 424.3 [M+H]+.

### 7.7 Synthesis of 3,4-diphenethoxybenzaldehyde 4

To a stirred solution of 3,4-dihydroxybenzaldehyde **1** (1.0 g, 7.25 mmol) in dry DMF was added (2-bromoethyl)benzene (2.48 mL, 18.1 mmol), followed by anhydrous K₂CO₃ (5.0 g, 36.2 mmol). The resulting mixture was stirred at room temperature for 2 h. Additional K₂CO₃ (2.4 g, 17.3 mmol) was added, and the mixture was heated at 70°C for 30 min and then cooled to room temperature. The mixture was partitioned between H₂O and ether (120 mL each). The organic layer was separated, and the water layer was extracted with ether (3 × 50 mL) . The pooled organic layers were washed with H₂O (2 × 50 mL) and saturated aqueous NaCl solution (50 mL) . The pale, straw-colored extracts were dried over anhydrous sodium sulfate, washed with hexanes (75 mL), and then concentrated. The resulting residue was purified by silica gel column chromatography using hexane/ethyl acetate (7:3) to afford 3,4-diphenethoxybenzaldehyde **4** (2.30 g, 92%) as a cream-colored solid. ¹H-NMR (CDCl₃, 300 MHz): *δ* 9.84 (s, 1H), 7.43 (dd, 1H, *J* = 3.0, 9.0 Hz), 7.42 (s, 1H), 7.35 (d, 8H, *J* = 6.0 Hz), 7.31-7.24 (m, 2H), 6.96 (d, 1H, *J* = 9.0 Hz), 5.62 (s, 1H), 4.36 (td, 4H, *J* = 3.0 and 6.0 Hz), 3.17 (td, 4H, *J* = 3.0 and 6.0 Hz); ESIMS m/z: 347.3 [M+H]+.

### 7.8 Synthesis of 3,4-diphenethoxyphenol 5

m-Chloroperbenzoic acid (0.40 g, 2.35 mmol) was added to a solution of the 3,4-diphenethoxybenzaldehyde **4** (0.5 g, 1.57 mmol) in dichloromethane (10 mL), and the mixture was stirred at room temperature for 4 h and then diluted with ethyl acetate. The organic solution was successively washed with saturated aqueous Na₂CO₃ solution and brine. The solvent was evaporated in *vacuo* to afford corresponding formiate. NaOH(6 N) was added to a stirred solution of crude formiate in MeOH (10 mL). After stirring at room temperature for 30 min, was added 10% aqueous HCl solution. The resulting reaction mixture was diluted with ethyl acetate (50 mL), washed with brine and dried over anhydrous Na₂SO₄. The flash chromatography (7:3 hexane/ethyl acetate) was performed to afford 3,4-diphenethoxyphenol **5** (0.40 g, 93%) as a white solid. ¹H-NMR (CDCl₃, 300 MHz): *δ* 7.22-7.35 (m, 10H), 6.76 (d, 1H, *J* = 9.0 Hz), 6.44 (d, 1H, *J* = 3.0 Hz), 6.30 (dd, 1H, *J* = 3.0 and 6.0 Hz), 4.74 (s, 1H), 4.13 (td, 4H, *J* = 3.0 and 9.0 Hz), 3.10 (td, 4H, *J* = 3.0 and 9.0 Hz).

### 7.9 Synthesis of (R)-2-((3,4-diphenethoxyphenoxy)methyl)oxirane 6

To a mixture of 3,4-diphenethoxyphenol **5** (0.35 g, 1.05 mmol) in ethyl alcohol (5 mL), aqueous potassium hydroxide solution (57.8 mg, 1.05 mmol, 100 µL water) and (R)-epichlorohydrin (1.13 mL, 5.0 mmol) were added. The resulting mixture was stirred for 15 h at room temperature. The solvent was removed under reduced pressure, and the residue was dissolved in water and extracted with ethyl acetate. The organic extract was washed with brine and dried over Na₂SO₄. The solvent was evaporated to afford a crude product, which was purified by column chromatography (silica gel 60-120 mesh), with an ethyl acetate and n-hexane mixture in 1:4 ratio to afford the desired product (R)-2-((3,4-diphenethoxyphenoxy)methyl)oxirane **6** (0.33 g, 80%).

### 7.10 Synthesis of (R)-1-(3,4-diphenethoxyphenoxy)-3-(isopropylamino)propan-2-ol YOK-2204

To a stirred solution of (R)-2-((3,4-diphenethoxyphenoxy)methyl)oxirane **6** (100 mg, 0.26 mmol) in EtOH (2 mL) was added isopropylamine (0.21 mL, 2.6 mmol), and the resulting mixture was vigorously stirred at room temperature for 4 h (TLC-monitoring). Then, the solvent was removed under reduced pressure. The resulting residue was extracted with CH₂Cl₂ (3×10 mL). The combined organic layers were washed with brine (10 mL), dried over anhydrous Na₂SO₄ and concentrated under reduced pressure. The obtained crude product was purified by column chromatography (CH₂Cl_{2/}MeOH, 10:1) to afford pure (R)-1-(3,4-diphenethoxyphenoxy)-3-(isopropylamino)propan-2-ol **YOK-2204** (99 mg, 86%) as a white powder. ¹H NMR (300 MHz, CDCl₃) : *δ* 7.40-7.20 (m, 10H) , 6.78 (d, 1H, *J* = 9.0 Hz ), 6.52 (d, 1H, *J* = 3.0 Hz ), 6.38 (dd, 1H, *J* = 3.0 and 9.0 Hz ), 4.14 (dt, 4H, *J* = 9.0 and 15.0 Hz ), 3.96-3.87 (m, 2H), 3.36 (brs, 2H), 3.11 (dt, 4H, *J* = 9.0 and 15.0 Hz ), 3.01-2.92 (m, 2H), 2.80 (dd, 1H, *J* = 9.0 and 12.0 Hz ), 1.19 (s, 3H), 1.17 (s, 3H) and also confirmed with ESIMS m/z: 424.3 [M+H]+.

### 7.11 Synthesis of 4-(benzyloxy)-3-hydroxybenzaldehyde 7

To a stirring solution of 3,4-dihydroxybenzaldehyde **1** (2.5 g, 18.1 mmol) in anhydrous acetonitrile (30 mL), was added K₂CO₃ (2.5 g, 18.1 mmol) followed by benzyl bromide (2.15 mL, 18.1 mmol) slowly at room temperature under an inert (N₂) atmosphere. The reaction solvent was removed by evaporation under reduced pressure. To the resulting residue was added cold 10% NaOH solution, and the mixture was stirred for 10 min, after which ethyl acetate (100 mL) was added. The resulting biphasic mixture was separated and the aqueous layer was acidified with 4 N HCl and extracted with DCM (3 × 300 mL). The combined organic layers were washed with brine solution and water, dried over Na₂SO₄, and concentrated under reduced pressure to afford a residue, which was purified by crystallization using ethyl acetate to afford 4-(benzyloxy)-3-hydroxybenzaldehyde **7** (3.50 g, 85 %) as a white powder. ¹H NMR (300 MHz, CDCl₃): δ 9.85 (s, 1H), 7.48-7.41 (m, 10H), 7.05 (d, 1H, *J* = 9.0 Hz), 5.90 (s, 1H), 5.22 (s, 2H); ESIMS m/z: 229 [M+H]+.

### 7.12 Synthesis of 4-(benzyloxy)-3-phenethoxybenzaldehyde 8

To a stirred solution of 4-(benzyloxy)-3-hydroxybenzaldehyde **7** (2.50 g, 10.96 mmol) in dry DMF (10 mL) was slowly added anhydrous K₂CO₃ (2.90 g, 21.0 mmol), followed by (2-bromoethyl)benzene (2.24 mL, 16.44 mmol). The resulting mixture was heated at 70°C for 2 h and then cooled to room temperature. The mixture was partitioned between H₂O and ether (20 mL each). The organic layer was separated, and the water layer was extracted with ether (3 × 20 mL). The pooled organic layers were washed with H₂O (2 × 20 mL) and saturated aqueous NaCl solution (20 mL). The pale, straw-colored extracts were dried over anhydrous sodium sulfate and concentrated. The resulting residue was purified by silica gel column chromatography using EtoAc:Hexane (1:9) to afford 4-(benzyloxy)-3-phenethoxybenzaldehyde **8** (3.28 g, 90%) as a cream-colored solid. ¹H-NMR (CDCl₃, 300 MHz): *δ* 9.84 (s, 1H), 7.48-7.26 (m, 12H), 7.02 (d, 1H, *J* = 9.0 Hz), 5.22 (s, 2H), 4.32 (t, 2H, *J* = 6.0 Hz),3.19 (t, 2H, *J* = 6.0 Hz).

### 7.13 Synthesis of 2-((4-(benzyloxy)-3-phenethoxybenzyl)amino)ethan-1-ol YTK-1205

To a stirred solution of 4-(benzyloxy)-3-phenethoxybenzaldehyde **8** (100 mg, 0.30 mmol) in dry ethanol (5 mL), and 2-aminoethan-1-ol (91.5 µL, 1.5 mmol) was added and the resulting reaction mixture was heated at 60°C. After completion of aldehydation, the reaction mixture was cooled to room temperature. NaBH₄ (17.1 mg, 0.45 mmol) was added slowly in portions, and the resulting reaction solution was stirred for another 6 h. The solvent was removed by being evaporated in *vacuo,* and the residue was dissolved in water and extracted with ethyl acetate. The organic layers were combined and dried over Na₂SO₄, filtered, and evaporated in *vacuo.* The resulting residue was purified by flash column chromatography to afford the desired product 2-((4-(benzyloxy)-3-phenethoxybenzyl)amino)-ethan-1-ol **YTK-1205** (97.7 mg, 86%). ¹H NMR (CD₃OD): *δ* 7.42-7.16 (m, 10H), 7.00 (d, 1H, *J* = 3.0 Hz ), 6.95 (d, 1H, *J* = 9.0 Hz ), 6.83 (dd, 1H, *J* = 3.0 and 9.0 Hz ), 5.02 (s, 2H), 4.24 (t, 2H, *J* = 6.0 Hz), 3.71 (s, 2H), 4.04 (s, 2H), 3.66 (t, 2H, *J* = 6.0 Hz), 3.10 (t, 2H, *J* = 6.0 Hz), 2.71 (t, *J* = 4.8, 2H). ESIMS m/z: 378 [M+H]+.

### 7.14 Synthesis of methyl (R)-(3-(3,4-bis(benzyloxy)phenoxy)-2-hydroxypropyl)glycinate YOK-Gly-1104

To a stirred solution of epoxide **3** (100 mg, 0.27 mmol) in EtOH (2 mL) was added glycine methyl ester hydrochloride (43.2 mg, 0.54 mmol), and the resulting mixture was vigorously stirred at room temperature for 4 h (TLC-monitoring). Then, the solvent was removed under reduced pressure. The resulting residue was extracted with CH₂Cl₂ (3×10 mL) . The combined organic layers were washed with brine (10 mL), dried over anhydrous Na₂SO₄ and concentrated under reduced pressure. The obtained crude product was purified by column chromatography (CH₂Cl₂/MeOH, 10:1) to afford pure methyl (R)-(3-(3,4-bis(benzyloxy)phenoxy)-2-hydroxypropyl)glycinate **YOK-Gly-1104** (102 mg, 82%) as a white powder and confirmed with ESIMS m/z: 453 [M+H]+.

### 7.15. Synthesis of compounds ATB1 to ATB29

Compounds ATB1 to ATB29 in Table 3 below were synthesized with reference to Reference Examples 7.1 to 7.14.

**[Table 3]**

| No. | Structure | No. | Structure |
|---|---|---|---|
| ATB-1 | 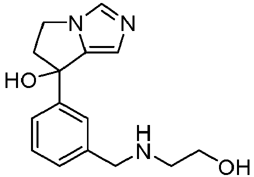 | ATB -16 | 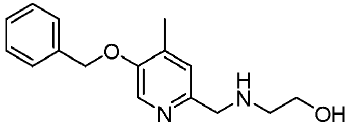 |
| ATB-2 | 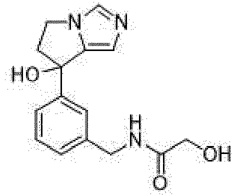 | ATB -17 | 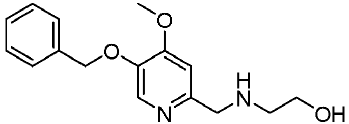 |
| ATB-3 | 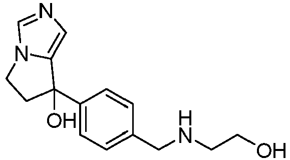 | ATB -18 | 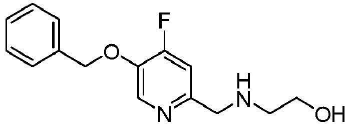 |
| ATB-4 | 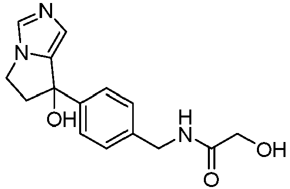 | ATB -19 | 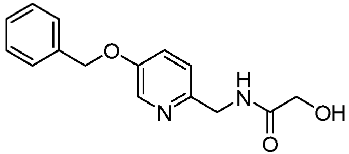 |
| ATB-5 | 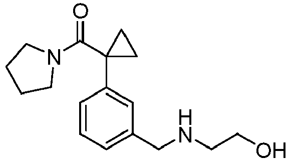 | ATB -20 | 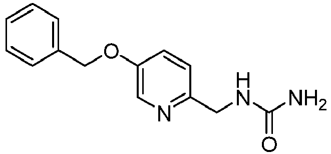 |
| ATB-6 | 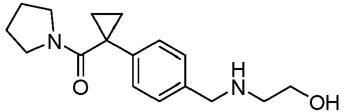 | ATB -21 | 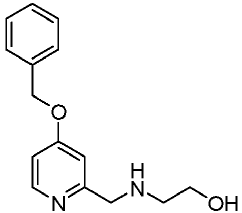 |
| ATB-7 | 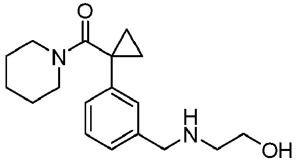 | ATB -22 | 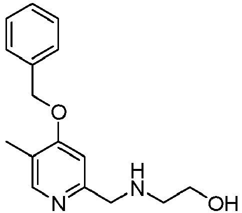 |
| ATB-8 | 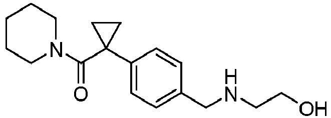 | ATB -23 | 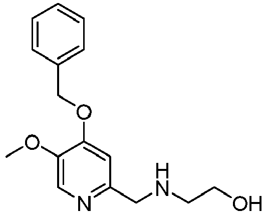 |
| ATB-9 | 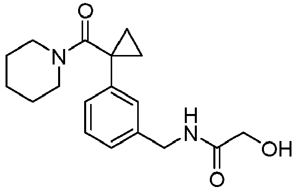 | ATB -24 | 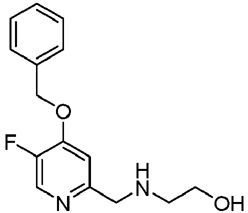 |
| ATB-10 | 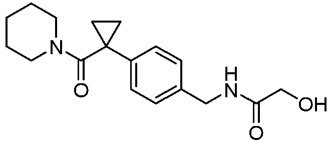 | ATB -25 | 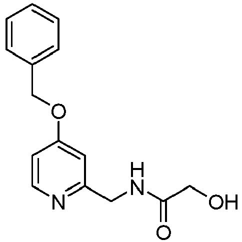 |
| ATB-11 | 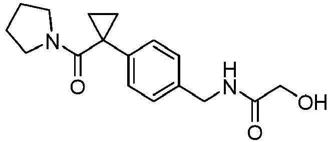 | ATB -26 | 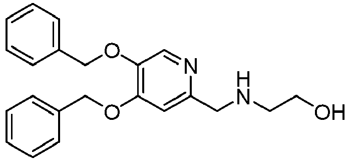 |
| ATB-12 | 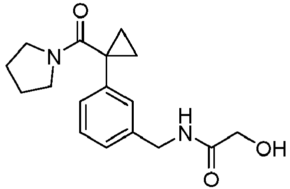 | ATB -27 | 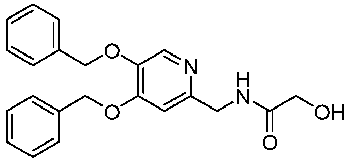 |
| ATB-13 | 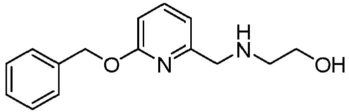 | ATB -28 | 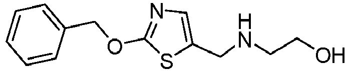 |
| ATB-14 | 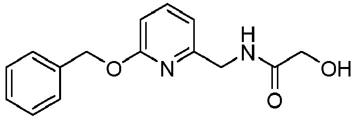 | ATB -29 | 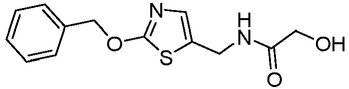 |
| ATB-15 | 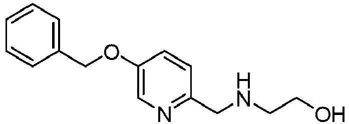 | | |

The synthesis schemes of the compounds are as follows:

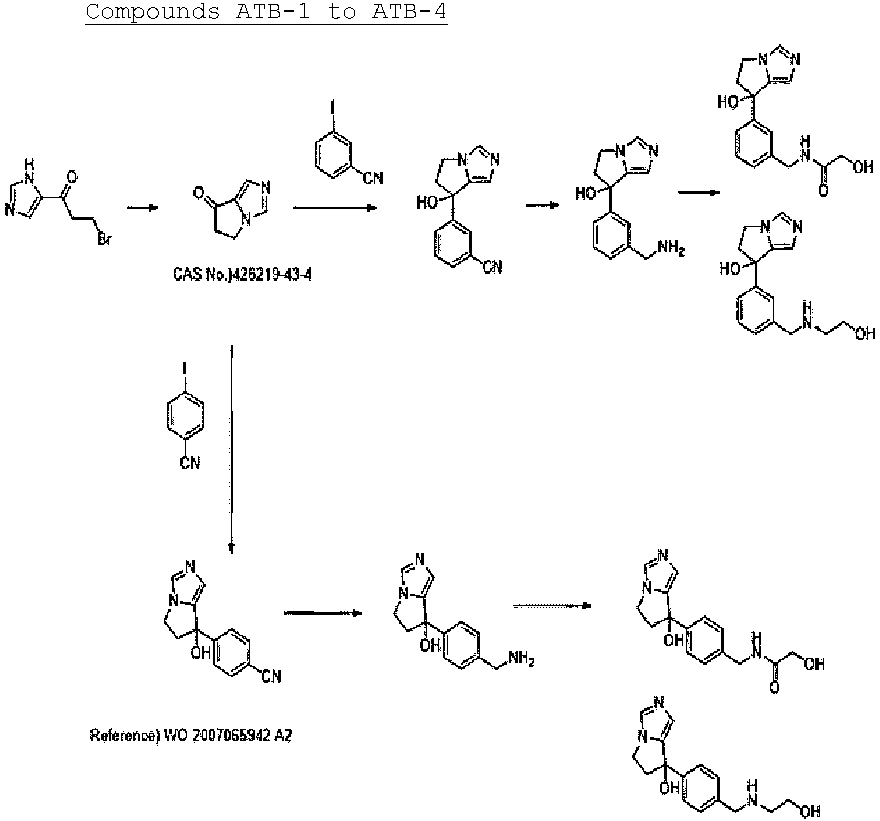

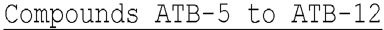

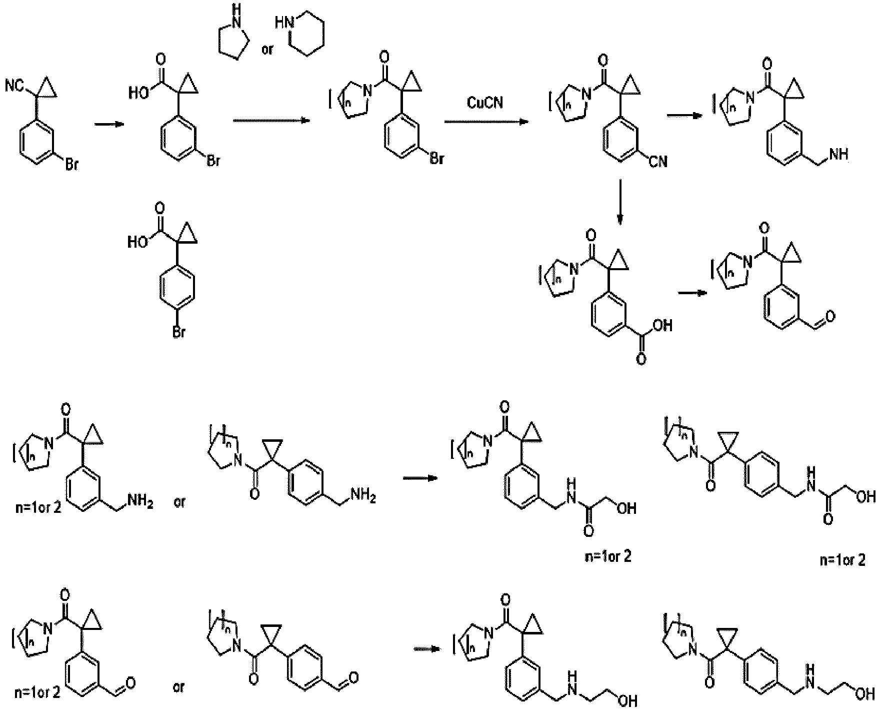

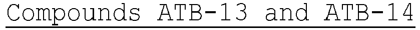

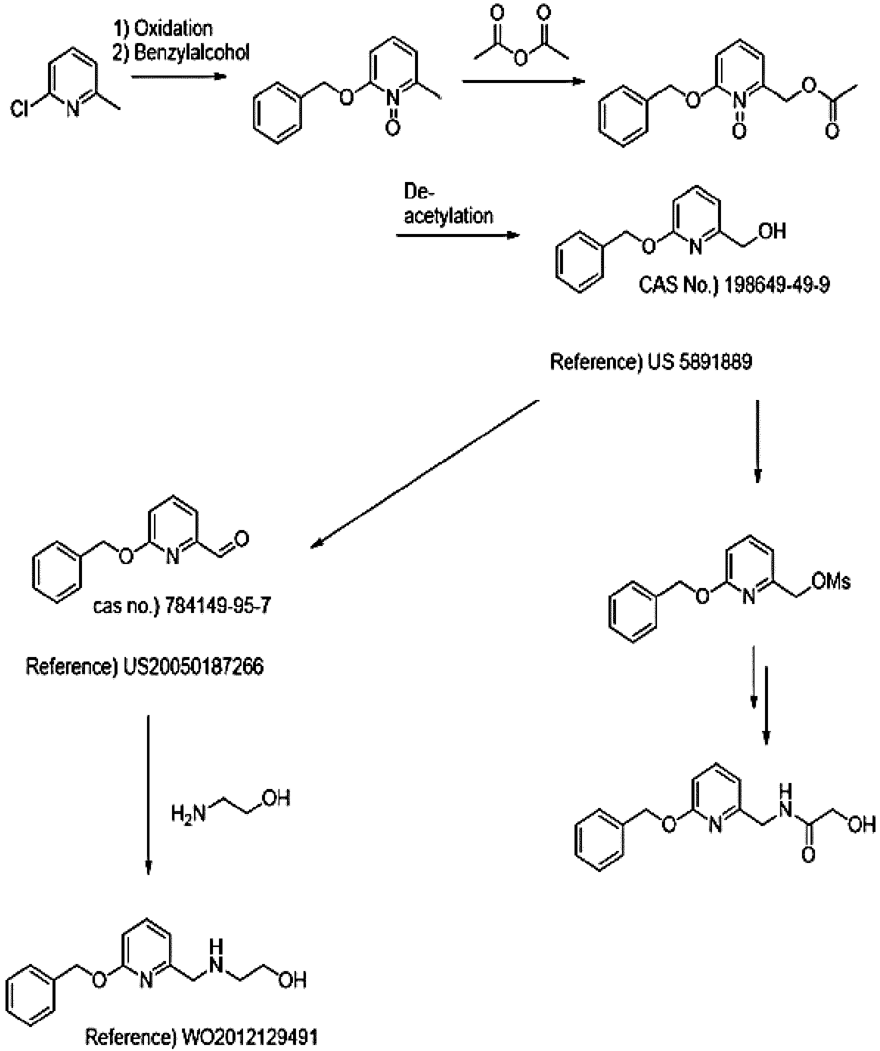

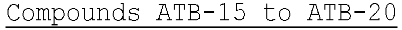

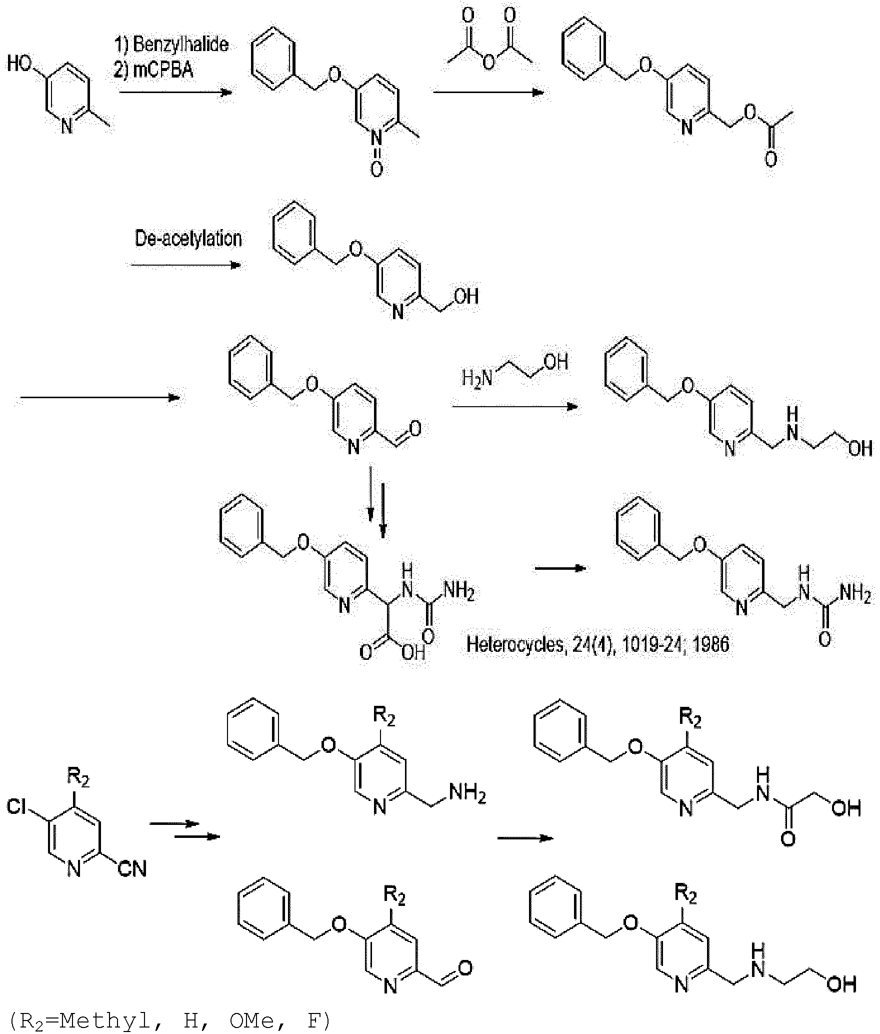

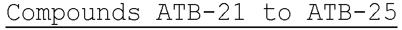

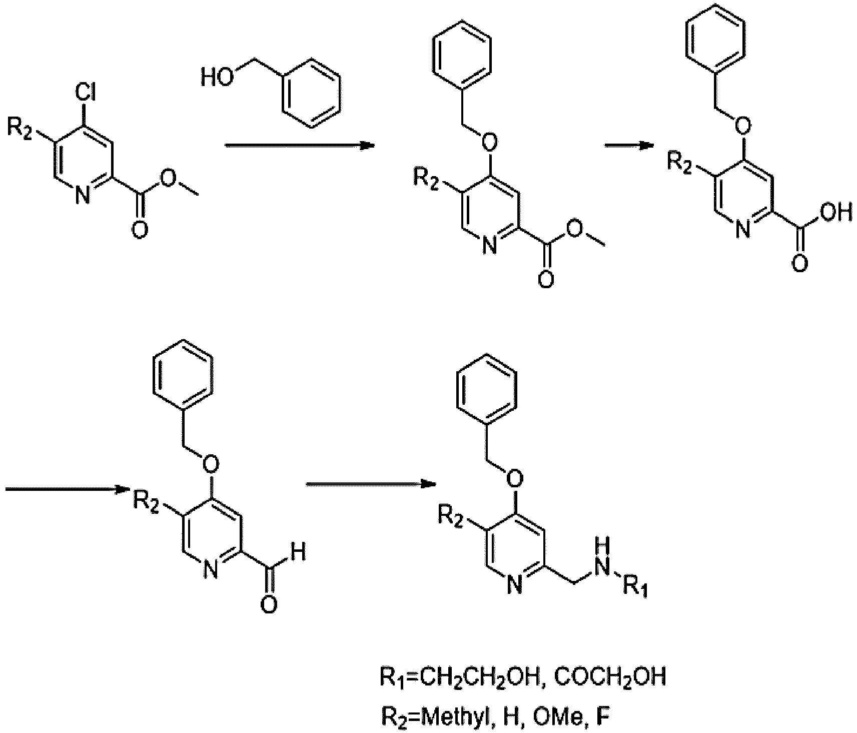

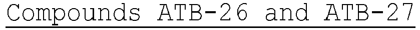

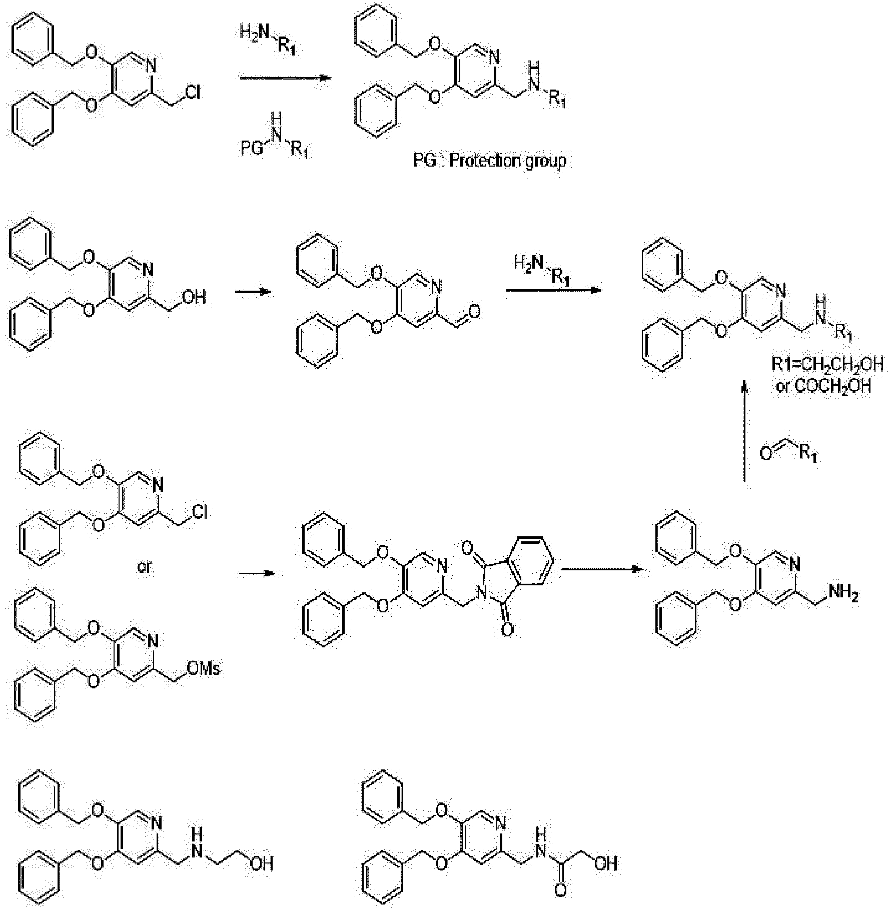

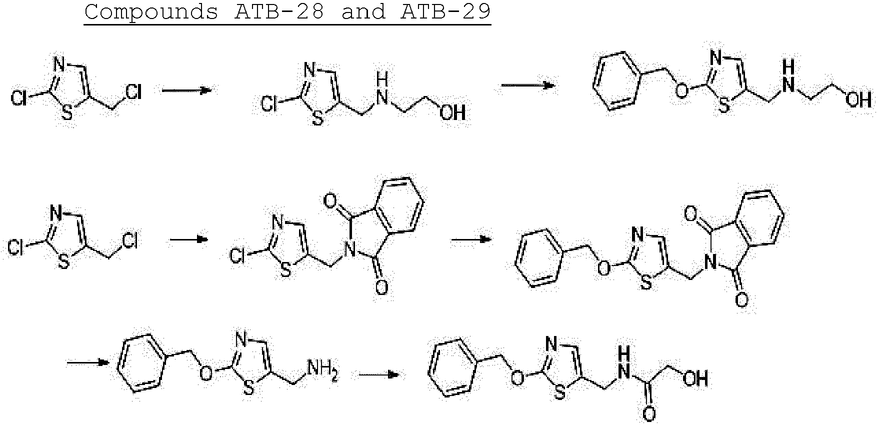

### Reference Example 8: Immunocytochemistry

To observe cellular localization of proteins, cells were cultured on cover slips coated with poly-L-lysine (Sigma). The cells were fixed with 4% paraformaldehyde in PBS (pH 7.4) for 15 min at room temperature and washed three times with PBS for 5 min. The cells were permeabilized with 0.5% Triton X-100 in PBS solution for 15 min and washed three times with PBS for 5 min. The cells were blocked with 2% BSA in PBS solution for 1 h at room temperature. After blocking, the cells were incubated overnight at 4°C with a primary antibody diluted in 2% BSA/PBS solution. After incubation, the cells were washed three times with PBS for 10 min and incubated with the Alexa Fluor-conjugated secondary antibody diluted in 2% BSA/PBS for 30 min at room temperature. Subsequently, the coverslips were mounted on glass slides using a DAPI-containing mounting medium (Vector Laboratories). Confocal images were taken by a laser scanning confocal microscope 510 Meta (Zeiss) and analyzed by Zeiss LSM Image Browser (ver. 4.2.0.121).

### Reference Example 9: Co-immunoprecipitation (co-IP)

To test protein interaction, co-immunoprecipitation assays were performed. For exogenous co-IP, HEK293T cells were transfected with recombinant p62, BiP, TRIM13, reticulophagy regulator 1 (FAM134B), reticulon 3 (RTN3) or ATZ (Z variant E342K) using Lipofectamine 2000. The cell pellets were scraped and pelleted by centrifugation, resuspended and lysed in an immunoprecipitation buffer (IP buffer) [50 mM Tris-HCl pH 7.5, 150 mM NaCl, 0.5% Triton X-100, 1mM EDTA, 1mM phenylmethylsulfonyl fluoride (PMSF; Roche) and protease inhibitor cocktail (Sigma)] on a rotor at 4°C for 30 min. Next, the supernatant and remaining pellets were passed through a 26-gauge 1 mL syringe 15 times and centrifuged at 13,000 g at 4°C and collected for the supernatant, to which normal mouse IgG (Santa Cruz) and Protein A/G-Plus agarose beads (Santa Cruz) were added to preclear the lysate on a rotor at 4°C overnight. The cell lysate was then incubated with M2 FLAG-affinity Gel agarose beads (Sigma) on a rotor at 4°C for 3 h. The gel beads were washed four times with IP buffer, resuspended in 2X Laemmli Sample Buffer, separated by SDS-PAGE and analyzed by immunoblotting with specified antibodies.

### Reference Example 10: Denaturation-immunoprecipitation

To test ubiquitination of ectopically expressed or endogenous TRIM13, denaturation immunoprecipitation assay was carried out. Briefly, cell pellets after trypsinization and centrifugation were resuspended in N-ethylmaleimide (NEM)-based buffer (10% SDS, 10 mM NEM in PBS), boiled at 100°C for 10 min and passed through a 26-gauge 1 mL syringe 15 times followed by centrifugation at 13,000 g at 4°C. The subsequent steps were identical to those during co-IP in Reference Example 9.

Alternatively, His-tagged mutant Ub constructs were transiently co-transfected into HEK293T cells together with constructs expressing TRIM13 using Lipofectamine 2000 for 24 h and subsequently treated with specified compounds (Reference Example 7) for indicated times. The cell pellets following trypsinization, collection and centrifugation were resuspended in 10 mM N-ethylmaleimide (NEM) solution in PBS with Ni-NTA+ beads (Sigma) in a binding buffer (pH 8, 6 M guanidium chloride, 0.1 M Na2HPO4/NaH2PO4, 10 mM Tris pH 8, 10 mM beta-mercaptoethanol, 5 mM NEM, and 5 mM imidazole) for overnight incubation at 4°C. The beads were then washed with a series of wash buffers designated A (pH 8, 6 M guanidium chloride, 0.1 M Na₂HPO₄/NaH₂PO₄, 10 mM Tris pH 8, and 10 mM β-ME), B (pH 8, 8 M urea, 0.1 M Na₂HPO₄/NaH₂PO₄, 10 mM Tris pH 8, 10 mM β-ME), C (pH 6.3, 8 M urea, 0.1 M Na₂HPO₄/NaH₂HPO₄, 10 mM Tris pH 8, 10 mM β-ME, 0.2% Triton X-100) and D (pH 6.3, 8 M urea, 0.1 M Na₂HPO₄/NaH₂PO₄, 10 mM Tris pH 8, 10 mM β-ME, 0.1% Triton X-100) at room temperature and incubated in an elution buffer (2X Laemmli Sample Buffer, 0.72 M BME, and 200mM imidazole) for 20 min. The samples were boiled at 100°C for 10 min and loaded for SDS-PAGE and immunoblotting analysis.

### Reference Example 11: ER expansion visualization and measurement

Using confocal microscopy of cells analyzed by immunocytochemistry, ER expansion was visualized by considered when KDEL- or calnexin-labelled ER occupied more than 80% of cell area. Furthermore, the ER area was calculated by ImageJ (NIH, Bethesda, v1.52) and the background threshold was manually defined and set for all images. The ER area was calculated and marked from the total cell area as a fraction after borders of each cell were set.

### Reference Example 12: In vitro p62 oligomerization

HEK293 cells were transiently transfected with a plasmid encoding p62-myc/his fusion proteins (Reference Example 2), resuspended in a lysis buffer [50 mM Hepes (pH 7.4), 0.15 M KCl, 0.1% Nonidet P-40), 10% glycerol, and a mixture of protease inhibitors and phosphatase inhibitor (Abcam)] and lysed by 10 cycles of freezing and thawing, followed by centrifugation at 13,000 × g for 20 min at 4°C. The protein concentration in the supernatant was determined using a BCA assay (Thermo Fisher Scientific). A total of 1 µg of protein was mixed with 50 mM of the Arg-Ala or Ala-Arg dipeptide (Anygen) or 1000 µM of p62-ZZ ligands in the presence of 100 µM bestatin (Enzo) at room temperature for 2 h. Next, a non-reducing 4X LDS sample buffer was added to each sample, heated at 95°C for 10 min, and resolved using 4-20% gradient SDS-PAGE (Bio-Rad). Immunoblotting analysis was carried out to monitor the conversion of p62 monomers into oligomers or aggregates using an anti-myc antibody.

### Reference Example 13: In vivo oligomerization

HEK293T cells were transfected with TRIM13-flag using Lipofectamine 2000 and treated with hydroxychloroquine (Sigma) for 24 h. To lyse the cells, the cells were subjected to a cycle of freezing/thawing and centrifuged at 13,000 g for 10 min after 30 min incubation on ice for supernatant collection. Protein concentration was measured using the Pierce BCA Protein Assay Kit (Thermo Fisher Scientific). Next, a non-reducing 4X LDS sample buffer was added to the sample lysate and followed by boiling at 100°C for 10 min. The samples were loaded on a 3% stacking and 8% separating SDS-PAGE. Immunoblotting assays were performed using an anti-Flag antibody (Sigma) to visualize the oligomeric complexes of TRIM13.

### Reference Example 14: Triton X-100-based insoluble/soluble fractionation

To determine the p62 ligand-degraded fraction of ATZ, cells expressing ectopic ATZ and treated with p62-ZZ ligands were harvested using a cell lysis buffer (20mM HEPES pH 7.9, 0.2M KCl, 1mM MgCl₂, 1 mM EGTA, 1% Triton X-100, 10% glycerol, protease inhibitor and phosphatase inhibitor) and incubated on ice for 15 min. After incubation, the cells were centrifuged at 13,000 g and 4°C for 10 min. The supernatant was collected as a soluble fraction and the pellet as an insoluble fraction. The insoluble fraction was thoroughly washed with PBS and lysed in an SDS-detergent lysis buffer (20 mM HEPES pH 7.9, 0.2 M KCl, 1 mM MgCl₂, 1 mM EGTA, 1% Triton x-100, 1% SDS, 10% glycerol, protease inhibitors and phosphatase inhibitors). The soluble and insoluble samples were added with a 5X Laemmli sample buffer, boiled at 100°C for 10 min and loaded on an SDS-PAGE gel.

### Reference Example 15: Subcellular fractionation

To analyze subcellular localization of ER chaperones and their arginylated forms, cells were trypsinized and pelleted by centrifugation at 1,500 × g at 4°C. The plasma membranes of the collected cells were resuspended and permeabilized using 0.01% digitonin (Thermo Fisher Scientific; BN2006) derived from *Digitalis purpurea* in a lysis buffer (110 mM KOAc, 25 mM K-HEPES, pH 7.2, 2.5 mM NaOAc and 1 mM EGTA). After centrifugation at 1,000 g for 5 min, the remaining supernatant was re-centrifuged at 15,000 × g and 4°C for 10 min to obtain a cytosolic fraction in the final supernatant comprising soluble cytosolic proteins. The microsome and nuclei fraction was pelleted by the initial centrifugation following digitonin permeabilization. The pellets were then resuspended and permeabilized in a RIPA-based buffer (50 mM Tris-HCl, pH 8.0, 150 mM NaCl, 1% NP-40, 0.5% sodium deoxycholate and 0.1% SDS) followed by centrifugation at 5,000 g for 10 min to separate the nuclei fraction as a pellet and the microsome fraction as a supernatant.

### Reference Example 16: Protein degradation cycloheximide-chase assay

To test the stability of ectopically expressed TRIM13, HeLa cells were transiently co-transfected with TRIM13-flag under siRNA-mediated knockdown of control (Thermo Fisher Scientific), ATE1 (Thermo Fisher Scientific) or p62 (Bioneer) for 48 h. Subsequently, the cells were treated with 10 µg/ml cycloheximide (Sigma) and collected at the indicated time points. The cells were completely lysed in an SDS-based 5X Laemmli sample buffer and boiled at 100°C for 10 min. After boiling, 10 µg of total protein lysate was loaded on an SDS-PAGE gel and analyzed by immunoblotting.

### Reference Example 17: Cell viability assay

Cell viability was quantified using the watersoluble tetrazolium salt-based EZ-Cytox cell viability assay kit (Dojindo Laboratory) according to the manufacturer's instructions. Briefly, following siRNA-mediated knockdown of control or ATE1 (48 h), HeLa cells in a 96-well plate were treated with the indicated ER stressors. Subsequently, an assay reagent solution (10 µL) was added to each well and the cells were incubated for 4 h at 37°C in a CO₂ incubator. Optical density (OD) values were measured at 450 nm using the Evolution 350 UV-Vis Spectrophotometer (Thermo Fisher Scientific).

### Reference Example 18: Transmission electron microscopy

For conventional transmission electron microscopy, HEK293T cells were treated with 2.5 µM YOK1104 for 6 h, scraped from culture dish and pelleted by centrifugation. The pellets were resuspended in 2.5% glutaraldeyhyde in 0.1 M sodium cacodylate buffer (pH 7.4) (Electron Microscopy Sciences) for overnight at 4°C. The fixative was replaced by a cacodylate buffer for the last 6 h, after which the cells were embedded in Epon resin. Subsequently, the cells were cut into 55-nm sections and stained with uranyl acetate and lead citrate using the Reichert Ultracut S Ultramicrotome (Leica Microsystems) and FEI Vitrobot Mark IV (Thermo Scientific), respectively. The cell sections were observed using the 200 kV transmission electron microscope FEI Tecnai G2-F20 (Thermo Scientific).

### Reference Example 19: Quantification and statistical analysis

For immunocytochemistry assays, cells were deemed to exhibit significant co-localization of two different proteins if more than ten clear puncta structures of the respective proteins showed association or full co-localization. Quantification results are expressed as the mean +/- S.D. value of three independent test results. For all data, the values represent the mean ± S.D or S.E.M. of at least three independent test results. The *P*-values were determined using ANOVA with two-tailed student's t-test (degree of freedom = n-1) or Prism 6 software (GraphPad). The statistical significance was determined as values of *p* < 0.05 (****p* < 0.001; ***p* < 0.01; **p*< 0.05).

### Example 1: Confirmation of role of p62 in ER-phagy and ER homeostasis

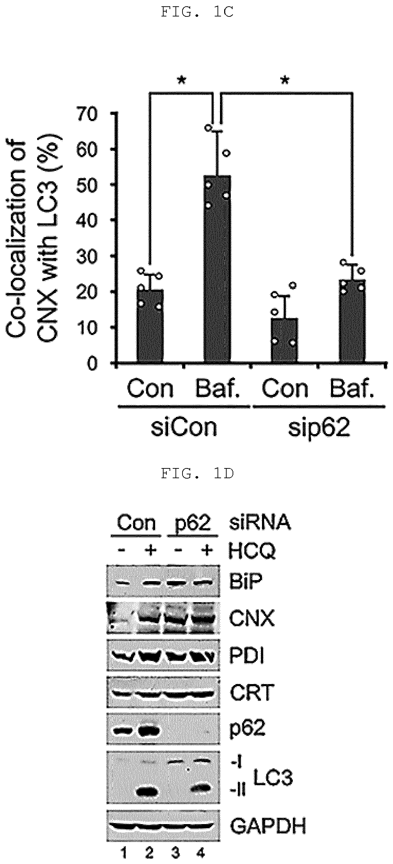

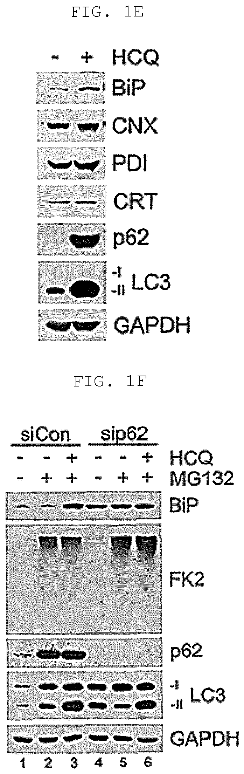

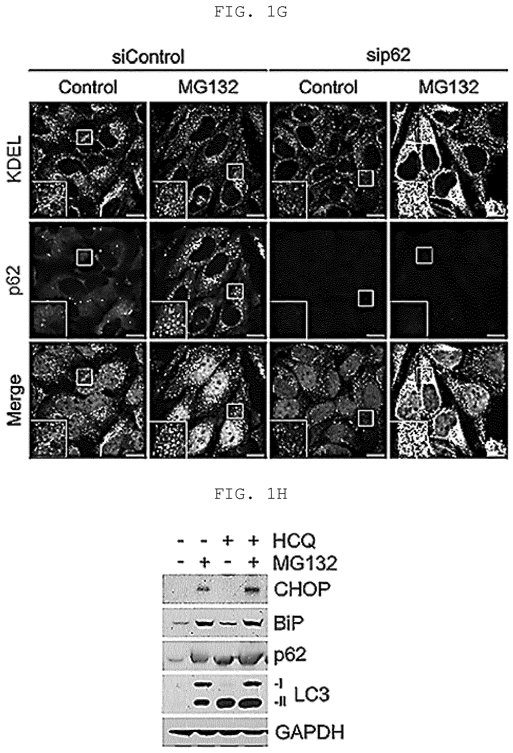

To elucidate the role of p62 in ER-phagy as an N-recognin, the localization of p62 was monitored in comparison to those of ER-residing proteins using immunostaining analysis. The p62 stainings marked the ER, forming puncta that were co-localized with the ER transmembrane protein calnexin (FIG. 1A) as well as proteins comprising the KDEL (Lys-Asp-Glu-Leu) sequence, an ER-retention signal (FIG. 7A). It was thus determined whether p62-associated ER compartments were subject to autophagic degradation. Autophagic flux assays using bafilomycin A1 showed that these puncta comprising ER proteins were targeted to LC3-GFP⁺ autophagic vacuoles (FIGS. 1A and 7A). Autophagic targeting (FIGS. 1B, 1C, 7B and 7C) and degradation (FIG. 1D) of ER proteins was inhibited by p62 knockdown (FIG. 1D) as strongly as by chemical inhibition of autophagy (FIG. 1E) or by Atg5 knockdown (FIG. 7E) as opposed to proteasomal inhibition (FIG. 7D). As these results suggest that p62-dependent macroautophagy mediates ER-phagy in normally growing cells, it was subsequently examined whether p62 mediated the autophagic degradation of ER compartments in stressed cells. When cells were treated with the proteasome inhibitor MG132, p62 knockdown impaired the turnover of BiP (FIG. 1F) as well as other KDEL (Lys-Asp-Glu-Leu) proteins (FIG. 1G). These results suggest that p62 mediates ER-phagy in normal cells as well as stressed cells.

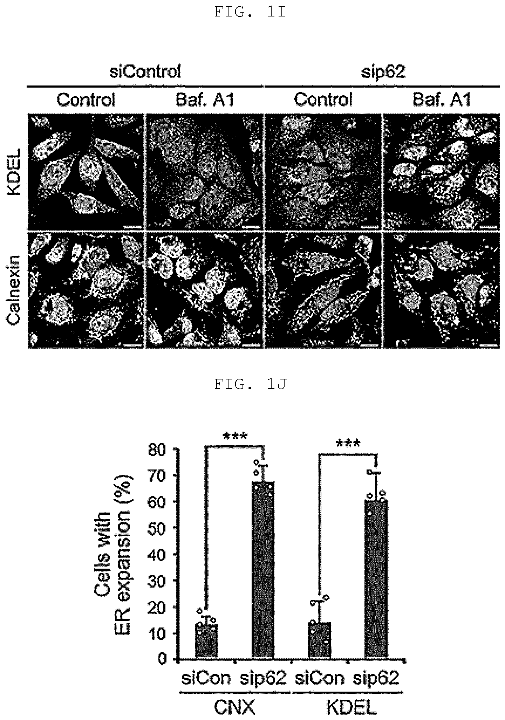

Next, it was tested whether p62 modulated the ER homeostasis. Immunoblotting analysis showed that autophagic inhibition rendered cells hypersensitive to ER stress as indicated by the increased level of CHOP (C/EBP Homologous Protein) (FIG. 1H). When p62 was depleted, the ER as a whole, as stained by calnexin and KDEL, lost its morphological integrity and spontaneously swelled (FIGS. 1I and 1J). The expanded ER was broken down into morphologically abnormal fragments (FIGS. 1I, 7F and 7G), which were devoid of LC3+ vacuoles (FIGS. 7F and 7H). Consistently, p62-deficient cells were hypersensitive to various ER stressors, triggering ER stress and apoptosis as confirmed by CHOP levels and PARP1 cleavage (FIG. 1K). These results suggest that p62-mediated macroautophagy plays a protective role in ER homeostasis.

### Example 2: Test of p62-mediated ER-phagy modulation by Nt-arginylation

It had been confirmed that the Nt-Arg (N-terminal arginine) of arginylated proteins bound to p62 and induced the oligomerization of p62. In the present Example, it was examined whether the Nt-Arg of arginylated proteins bound to p62 during ER-phagy and modulated the activity of p62. *In vitro* pulldown assays confirmed that the Nt-Arg of ER chaperones selectively bound to p62 (FIGS. 2A, 8A and 8B). *In vitro* oligomerization assays confirmed that the dipeptide Arg-Ala induced p62 oligomerization but the control dipeptide Ala-Arg did not induce p62 oligomerization (FIG 2B). When Nt-arginylation was inhibited using tannic acid, p62 punctate signals were delocalized from calnexin signals on the ER membrane as well as in the cytosol (FIGS. 2C and 2D), indicating that the Nt-Arg is required for the targeting of p62 to the ER.

Next, it was examined whether the Nt-Arg is indispensable for autophagic degradation of ER compartments. Indeed, autophagic targeting of KDEL⁺ proteins was abolished by inhibiting Nt-arginylation with not only tannic acid (FIGS. 2E and 2F) but also ATE1 knockout (FIGS. 8C and 8D). Moreover, either ATE1 knockdown (FIG. 2G) or inhibition (FIG. 8E) resulted in excessive accumulation of not only ER proteins such as BiP, CRT and PDI but also p62. A similar accumulation of these proteins was observed under ER stress caused by proteasomal inhibition (FIG. 8F). These results suggest that the Nt-Arg of arginylated proteins binds to p62, induces the polymerization of p62, a key step in ER-phagy, and induces autophagic flux of not only p62 but also various ER proteins.

Next, the importance of Nt-arginylation in ER homeostasis was assessed by monitoring ER stress responses and ER integrity. ER chaperones as well as their Nt-arginylated species were accumulated in the cytosol under ER stress caused by various challenges, ranging from misfolded protein accumulation and chaperone inhibition to impaired ER-Golgi trafficking and defective N-linked glycosylation (FIG. 2H). These Nt-arginylated ER chaperones, which were confirmed to bind to p62 (FIG. 2I), were subject to autophagic turnover (FIG 8G), suggesting that cellular Nt-Arg levels responded to ER stress and are ultimately degraded by autophagy. To rule out the possibility that cytosolic and non-arginylated species of these ER chaperones binds to p62 and modulate the activity of p62, Ub-R/V-BiP-flag fusion (cotranslationally cleaved to yield Arg- or Val-BiP-flag) proteins lacking their ER signal peptide and their ER retention signal KDEL peptide were constructed, ensuring their cytosolic localization (FIG. 2J). Co-IP analyses reveal that only Arg-BiP-flag interacts with p62 (FIG. 2K), indicating that the Nt-Arg residue of the arginylation-permissive ER chaperones is required for their binding with p62.

It was then examined whether this response modulated ER homeostasis. Cells deficient in ATE1 or the activity thereof were hypersensitive to autophagic inhibition (FIG. 8H) and ER stress (FIG. 2L). This stress response was followed by decreased viability (FIG. 8I) and spontaneous ER expansion (FIGS. 2M, 2N, 8C, 8J and 8K), leading to apoptosis (FIG. 2L). These results highlight the essential role of Nt-arginylation-mediated ER-phagy in the ER homeostasis in normally growing cells as well as those under ER stress and implicate the N-degron pathway in degradation of non-proteinaceous cellular materials.

### Example 3: Confirmation of activity of TRIM13, ER-associated receptor for p62 in ER-phagy

Given that p62 is an autophagic cargo adaptor that recognizes Ub chains on substrate proteins, it was tested whether ubiquitination was required for p62/Nt-Arg-dependent ER-phagy. To this end, the present inventors performed immunostaining analyses of different Ub linkage types. The results showed that the ER was marked by puncta positive for K63-linked Ub chains, which were subject to autophagic turnover (FIGS. 3A, 3B, 9A, 9C, 9D and 9E). The autophagic targeting of K63-linked Ub puncta was abolished in p62-deficient cells (FIGS. 3A and 3B). In contrast, K48-linked signals appeared to be diffusive throughout the cells (FIGS. 9B and 9C). These results implicate K63-linked ubiquitination in p62-mediated ER-phagy.

In search of an ER-associated receptor that binds to p62 and whose K63-linked ubiquitination enables p62-dependent ER-phagy, it was confirmed that the ER transmembrane E3 ligase TRIM13 ubiquitinated itself and other substrates via K63 linkage. Indeed, an autophagy flux assay showed that TRIM13 was degraded by macroautophagy and stabilized in ATG5 knockdown cells (FIG. 7E) as well as those treated with tannic acid (FIG. 8E). This implicates that TRIM13 may be the receptor for p62 in Nt-arginylation-mediated ER-phagy. When the ubiquitinated species of TRIM13 was selectively monitored (FIG. 3C), K63-ubiquitinated TRIM13 was the substrate of autophagy but not the substrate of proteasome (FIG. 3D and 9F). In contrast, K48-ubiquitinated TRIM13 was degraded by only the proteasome (FIG. 9G).

Next, it was examined whether TRIM13 was essential for ER-phagy. TRIM13 knockdown inhibited not only the localization of K63-Ub puncta on the ER but also the autophagic turnover (FIGS. 9D and 9E). TRIM13 knockdown also abolished autophagosomal targeting (FIGS. 3E and 3F) and lysosomal degradation (FIG. 3G, lanes 1 and 2 vs. 3 and 4) of ER proteins. A similar result was obtained under prolonged proteasomal inhibition (FIG. 3H). TRIM13 protein levels were drastically up-modulated by proteotoxic ER stress due to not only misfolded protein accumulation or chaperone inhibition but also impaired ER-Golgi trafficking or defective N-linked glycosylation (FIG. 3I). These results suggest that TRIM13 is an ER-associated receptor which is polyubiquitinated via K63 linkage and required for efficient ER-phagy as both constitutive and stress response systems.

Next, it was examined whether the autophagic degradation of TRIM13 required its interaction with p62. Cycloheximide degradation assays showed that p62 knockdown drastically inhibited the turnover of TRIM13 (FIGS. 3J and 9H). To examine whether TRIM13 was degraded as monomeric species or oligomeric species, *in* vivo oligomerization assays were performed using non-reducing SDS-PAGE. When autophagy was blocked, TRIM13 was accumulated as not monomeric species but oligomeric species ranging mainly from dimeric species to tetrameric species (FIG. 3K). Moreover, Co-IP assays showed that TRIM13 interacted with p62 (FIGS. 4B, 9I and 9J), which was strengthened upon autophagy inhibition. These results suggest that p62 binds to ER-associated TRIM13, forming a complex, whose oligomerization is a prerequisite to autophagic degradation and ER-phagy.

The domain of p62 that bound to TRIM13 was also dissected using p62 deletion mutants (FIG. 4A). Mapping analyses showed that PB1 domain was essential for binding to TRIM13 (FIG. 4C). Unexpectedly, the UBA domain of p62 was required not for binding to TRIM13 but for autophagic flux of the TRIM13-p62 complex (FIG. 4D). Given that the UBA domain of p62 likely binds to Ub chains on TRIM13, it was tested whether auto-ubiquitination of TRIM13 via K63 linkage was essential for ER-phagy. The C13A TRIM13 mutant, a catalytically inactive E3 ligase, could not be assembled with K63-Ub chains (FIG. 9K), suggesting that the primary means by which TRIM13 was ubiquitinated was auto-ubiquitination. The resulting mutant TRIM13-p62 complex was resistant to autophagic degradation (FIG. 4E). These results suggest that auto-ubiquitination of TRIM13 is essential but is not sufficient for ER-phagy.

### Example 4: Modulation of TRIM13 by Nt-arginylation

Given that the Nt-Arg binds to and activates p62, it was examined whether the N-degron Arg modulated TRIM13 via p62 in a *trans*-mode. Cycloheximide degradation assays showed that ATE1 knockdown inhibited the degradation of TRIM13 (FIG. 3I). Consistently, TRIM13 degradation was accelerated by overexpressing either ATE1 isoforms (FIG. 4F) or Arg-BiP-GFP (FIG. 4G), the latter being cotranslationally produced from the Ub-R/V-BiP-GFP fusion and lacking both the ER signal peptide and ER retention KDEL peptide (FIG. 2J). In contrast, Val-BiP-GFP lacking the Nt-Arg did not induce TRIM13 degradation (FIG. 4G). The non-reducing SDS-PAGE results showed that upon ATE1 inhibition by tannic acid, TRIM13 lost its oligomeric status, leading to the uncontrolled formation of aggregates (FIG. 4H and 9L). Thus, the Nt-Arg modulates the oligomerization of TRIM13 via p62.

Next, it was examined whether the Nt-Arg was essential for the interaction of TRIM13 with p62 and its degradation. Following tannic acid treatment, p62 normally bound to TRIM13, but TRIM13 and its complex with p62, which was normally degraded by autophagy, became metabolically stabilized (FIG. 4I, lanes 3 vs. 2 and 5 vs. 4). Consistently, co-IP analyses showed that p62 ZZ mutants that could not bind to the Nt-Arg normally bound to TRIM13 (FIG. 4J). When the turnover of the p62-TRIM13 was monitored, TRIM13 in the complex with p62 ZZ mutants was resistant to autophagic turnover, in contrast to wild-type p62 (FIG. 4J). This result was validated using p62 mutants harboring point mutations (C142A/C145A or C151A/C154A) in the zinc finger of ZZ domain, which rendered p62 unable to bind to Nt-Arg. These results suggest that Nt-Arg binding to p62 ZZ domain is essential for ER-phagy.

### Example 5: Identification of TRIM13 as platform for ER-phagy

In the present Example, it was examined how the p62/Nt-Arg/TRIM13 circuit induced fragmentation and/or membrane curvature for sequestration of ER-resident contents. The sequences of both p62 and TRIM13 were first analyzed. Any reticulon homology domain (RHD) that induced ER membrane curvature was not found. Then, in order to examine whether p62 interacted with previously identified RHD-carrying ER-phagy receptors, namely FAM134B and RTN3, it was observed through co-IP analyses that neither FAM134B (FIG. 4L) nor RTN3 (FIG. 9M) bound to p62 in a normal condition or an autophagy-inhibited condition.

Previous reports on Parkin-mediated mitophagy and ER-phagy in both yeast and mammals have identified mechanisms by which recruitment of autophagy initiation proteins to the organellophagy receptors mediates both autophagosome biogenesis and the delivery of organellular cargo therein. Specifically, the closure of isolation membrane unto itself also traps the omegasome(s) from which the isolation membrane originates, trapping ER-resident proteins and membrane proteins in the process. Given that many TRIM proteins function as platforms for not only selective autophagy cargo recognition but also autophagosome biogenesis, it was examined whether TRIM13 could selectively induce membrane curvature and/or fragmentation via autophagy induction during ER-phagy.

Co-IP analyses revealed that TRIM13 interacted with both Beclin-1 and VPS34 (FIG. 4M), members of the mammalian PI3K complex which can sense and induce membrane curvature and expansion of the isolation membrane originating from the omegasome during autophagosome nucleation. Moreover, these interactions were strengthened upon ER stress conditions (FIG. 4M). Consistent with these results, upon ER stress by tunicamycin treatment, TRIM13 formed punctate structures that were co-localized with not only p62 (FIG. 9N) but also the omegasome marker WIPI2 (FIG. 4N) and phagophore marker ATG16L. From the results that TRIM13 (FIGS. 3K, 4H and 9L) along with p62 (FIG. 2B) is degraded as oligomeric species, it is suggested that oligomeric TRIM13 may serve as a platform on or near the omegasome for both the Beclin-1/VPS34 complex and p62, for eventual engulfment by the isolation membrane/phagophore. This 'TRIM13-osome' may physically mark the site of ER-phagy via phagophore nucleation and anchor p62 for autophagosomal targeting of TRIM13-marked ER compartments.

### Example 6: Development of chemical N-degrons that modulates ER-phagy

To develop a pharmacological means to modulate ER-phagy, chemical mimics of the Nt-Arg were synthesized (FIGS. 5A and 10) based on the recent work and it was tested whether these ligands modulated the p62 functions for ER-phagy. Compared to negative controls (FIGS. 5B and 11D; 1101 and YOK-G-1104), the ligands selectively enhanced the oligomerization and aggregation of p62 (FIG. 5B) as well as cellular autophagic flux (FIGS. 11A, 11B and 11C). The co-localization analysis using endogenous or stably-expressed RFP-GFP-LC3 revealed that these facilitated autophagosomal (FIGS. 5C, 5D, 11E and 11F) and lysosomal (FIGS. 11G and 11H) targeting of the ER as punctate signals positive for p62 (FIGS. 11I to 11L). It was confirmed that the ligands induced delivery of entire parts of the ER (ER-phagy), as opposed to clusters of ER proteins (aggrephagy), to autolysosomes by transmission electron microscopy (FIG. 5E). Upon treatment with a compound YOK1104, HEK293T cells readily exhibited an abundance of autophagosomes and autolysosomes (FIG. 5E, 1^{st} column) coupled with short and fragmented ER scattered throughout the cytosol (FIG. 5E, 1^{st} and 2^{nd} column). Importantly, compared to those of control cells, autolysosomes in cells treated with the compound YOK1104 displayed the accumulation of ER fragments within their interior (FIG. 5E), indicating that the ligands induced the delivery of entire ER compartments. Next, it was tested whether the ligands could induce autophagic degradation of the ER and its contents via ER-phagy. Immunoblotting analysis results showed that the compound YOK1104 readily facilitated the degradation of BiP and PDI as well as TRIM13 following the up-modulation by proteotoxic ER stress (FIG. 5F). Autophagy flux assays confirmed that YOK1104 drove TRIM13 to autophagic degradation (FIG. 11M). These results suggest that the p62 ligands accelerate ER-phagy.

It was tested whether the chemical N-degrons could restore ER homeostasis by virtue of ER-phagy. Indeed, a compound YTK1105 rescued tannic acid-treated cells from ER stress and apoptosis in an autophagy-dependent manner (FIG. 5G). In cells treated with tannic acid, the ER spontaneously swelled into abnormal morphology, which was readily restored by YTK1105 (FIGS. 5H and 5I). To test whether this efficacy depended on p62, YTK1105 was compared to the general autophagy inducer rapamycin. In contrast to YTK1105, such rescue effects were not observed with rapamycin (FIGS. 5H and 5I). Importantly, p62 ligands also rescued cells from other types of ER stress and consequent apoptosis (FIGS. 5J, 11N and 11O), indicating that p62-ZZ ligands have the potential to ameliorate ER stress.

The mechanisms by which p62 ligands accelerated ER-phagy was investigated. Co-IP coupled with autophagy flux analysis using TRIM13 pulldown confirmed that YOK1104 enhanced autophagic degradation of the TRIM13-p62 complex (FIG. 5K). Hence, it was examined whether p62 ligands induced TRIM13 degradation as monomeric species or oligomeric species using non-reducing SDS-PAGE. YOK1104 selectively induced the degradation of oligomeric species but not that of monomeric species of TRIM13 (FIG. 5L). Collectively, the results demonstrate that the chemical N-degrons facilitate autophagic degradation of the TRIM13-p62 complex as an oligomeric form, providing a pharmaceutical means to modulate ER-phagy and maintain ER homeostasis.

### Example 7: ER protein quality control mediating activity of N-degron Arg via ER-phagy

Soluble misfolded proteins produced within the ER lumen are sorted out by molecular chaperones and delivered to ERAD (ER-Associated Degradation) for ubiquitination and proteasomal degradation. However, little is known about how ERAD-resistant insoluble aggregates trapped within the ER lumen are degraded. It was tested whether the N-degron Arg facilitated sequestration and autophagic degradation of insoluble misfolded aggregates accumulated in the ER lumen by using the Z variant (E342K; ATZ) of alpha1-antitrypsin (A1AT) as a misfolded and pathologically aggregation-prone substrate model of the metabolic proteinopathy alpha1-antitrypsin deficiency (ATD).

In ATD, which is the most common inherited metabolic liver disease, ATZ is misfolded and aggregated within the ER lumen of hepatocytes, whose chronic accumulation results in ER stress and consequent apoptosis of hepatocytes, leading to liver cirrhosis and even hepatocellular carcinoma. While the soluble monomeric species of ATZ are degraded by the proteasome via ERAD, its insoluble aggregated species is targeted to autophagy. It was confirmed that ectopic expression of ATZ, like that with proteotoxic ER stress (FIGS. 2I and 8F), induced Nt-arginylation of BiP (FIG. 6A) following its retrotranslocation to the cytosol (FIG. 6B). ER stress due to chemical proteotoxicity up-modulated the TRIM13 levels (FIG. 3I), ectopic expression of ATZ also up-modulated the TRIM13 protein levels (FIG. 12A). Moreover, RNA interference of either *p62* or *ATE1* (FIGS. 6C, 6D, 6E and 6F) as well as chemical inhibition of ATE1 (FIG. 12B) abolished autophagic targeting of ATZ and resulted in the accumulation of ATZ and its fragments (FIG. 6G). In contrast to aggregation-prone ATZ, the soluble and ERAD substrate NHK (null Hong Kong) variant of A1AT was not affected by p62 knockdown (FIG. 12C). These results suggest that N-degron-dependent ER-phagy selectively responds to the presence of ER-resident insoluble aggregates and sequesters and targets these for autophagic degradation.

The efficacy of synthetic p62 ligands in accelerating N-degron-dependent ER-phagy for ER protein quality control of ATZ was determined. Treatment with the ligands promoted puncta formation (FIGS. 6H, 12D and 12E), autophagic targeting (FIGS. 12F and 12G) and degradation of ATZ in an autophagy-dependent manner (FIG. 6I), especially in the detergent-insoluble fraction (FIG. 6J). Moreover, ATZ can be ubiquitinated by an ER transmembrane E3 ligase upon retrotranslocation and targeted to p62-dependent macroautophagy for lysosomal degradation, as a form of autophagic ERAD and/or aggrephagy. It was thus examined whether the p62/N-degron/TRIM13 circuit promoted ATZ degradation via ER-phagy but not autophagic ERAD or aggrephagy. Crucially, p62 ligand-induced ATZ puncta were strongly positive for the ER membrane (FIG. 6H) and ER luminal proteins (FIG. 12H). Moreover, the expression of TRIM13 led to autophagic clearance of ATZ (FIG. 6L) without a physical interaction between the two (FIG. 6K), which would have been required for autophagic ERAD or aggrephagy of ATZ following its retrotranslocation and ubiquitination. ATZ aggregates in the ER lumen are degraded by ER-phagy via the p62/Nt-Arg/TRIM13 circuit, whose response to the presence of ATZ aggregates, via the induction of Nt-arginylation and the TRIM13 levels, mediates ER proteostasis. These results not only provide a pharmaceutical means to eliminate pathogenic aggregates from the ER and alleviate ER stress but also suggest that the N-degron pathway mediates autophagic ER protein quality control.

## Claims

1. A pharmaceutical composition for preventing or treating a disease associated with ER-stress, which comprises p62 one or more selected from compounds listed in the following tables, or a pharmaceutically acceptable salt, stereoisomer, solvate, hydrate or prodrug thereof:
**[Table 4]**
| No. | Structure | No. | Structure |
|---|---|---|---|
| YOK 110 5 | 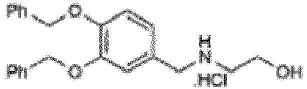 | YOK 220 4 | 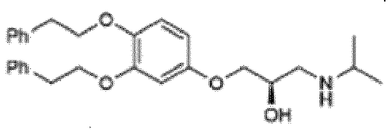 |
| YOK 110 4 | 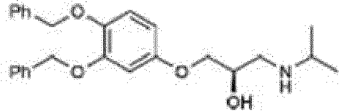 | YOK 110 6 | 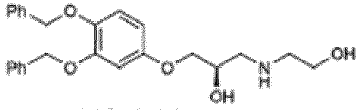 |
| YOK 120 5 | 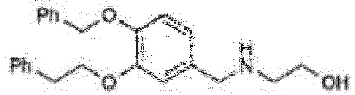 | ATB -15 | 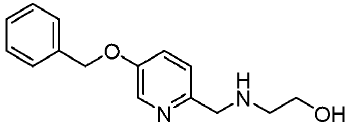 |
| ATB -1 | 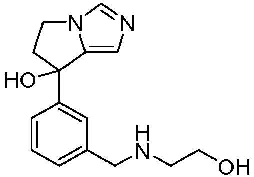 | ATB -16 | 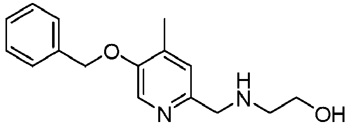 |
| ATB -2 | 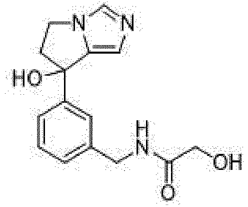 | ATB -17 | 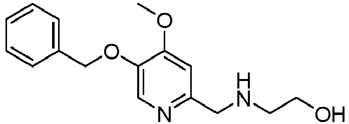 |
| ATB -3 | 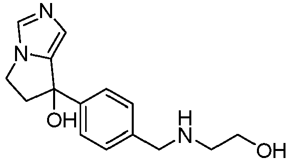 | ATB -18 | 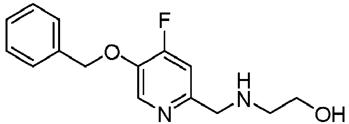 |
| ATB -4 | 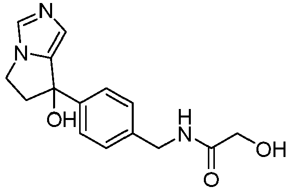 | ATB -19 | 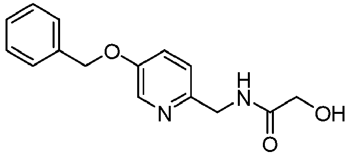 |
| ATB -5 | 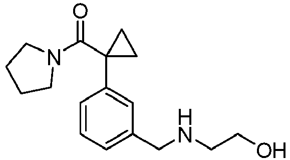 | ATB -20 | 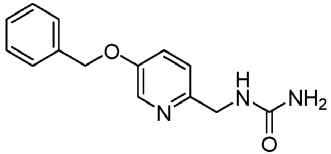 |
| ATB -6 | 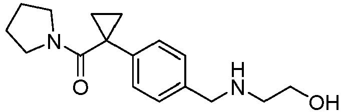 | ATB -21 | 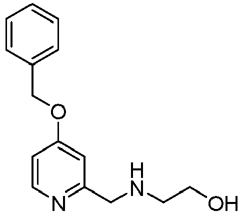 |
| ATB -7 | 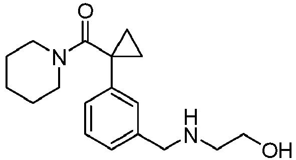 | ATB -22 | 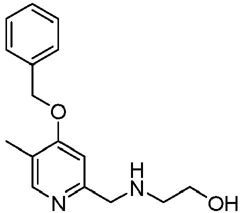 |
| ATB -8 | 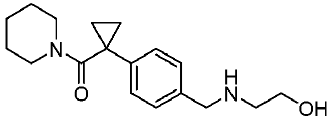 | ATB -23 | 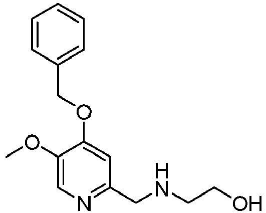 |
| ATB -9 | 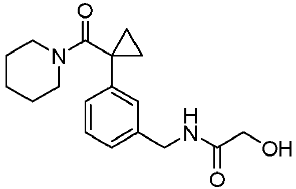 | ATB -24 | 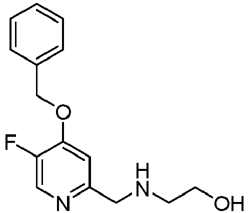 |
| ATB -10 | 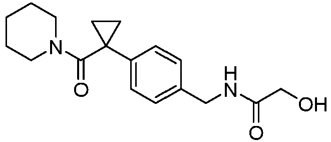 | ATB -25 | 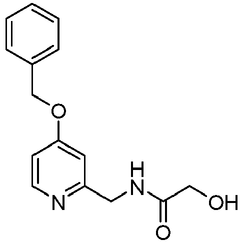 |
| ATB -11 | 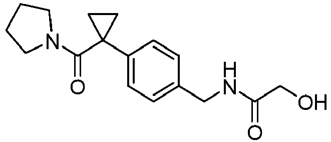 | ATB -26 | 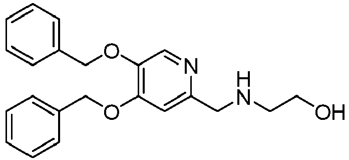 |
| ATB -12 | 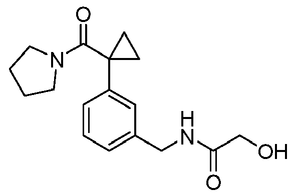 | ATB -27 | 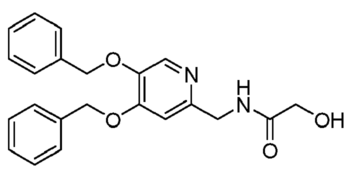 |
| ATB -13 | 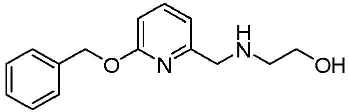 | ATB -28 | 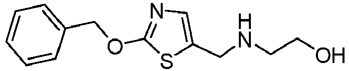 |
| ATB -14 | 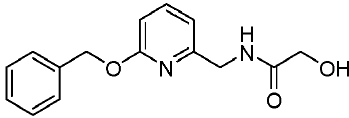 | ATB -29 | 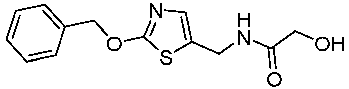 |
**[Table 5]**
| Structural Formula | No. | R1 | R2 |
|---|---|---|---|
| 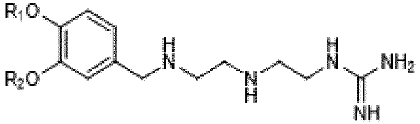 | YtK-1109 | -CH₂Ph | -CH₂Ph |
| | YtK-2209 | -CH₂CH₂Ph | -CH₂CH₂Ph |
| | YtK-3309 | -CH₂CH₂CH₂Ph | -CH₂CH₂CH₂Ph |
| | YtK-4409 | CH₂CH₂CH₂CH₂Ph | - CH₂CH₂CH₂CH₂Ph |
| | YtK-1209 | -CH₂Ph | -CH₂CH₂Ph |
| | YtK-1309 | -CH₂Ph | -CH₂CH₂CH₂Ph |
| | YtK-1409 | -CH₂Ph | CH₂CH₂CH₂CH₂Ph |
| 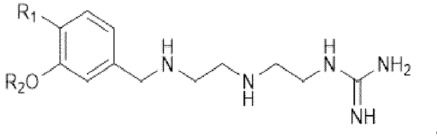 | YTK-109 | -H | -CH₂Ph |
| | YTK-209 | -H | -CH₂CH₂Ph |
| | YTK-309 | -H | -CH₂CH₂CH₂Ph |
| | YTK-409 | -H | CH₂CH₂CH₂CH₂Ph |
| 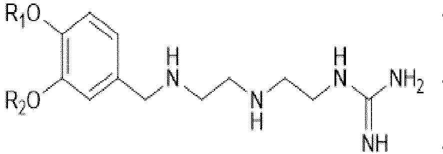 | YT-9-1 | -CH₂PhCl | -CH₂PhCl |
| | YT-9-2 | -CH₂PhF | -CH₂PhF |
| | YT-9-3 | -CH₂PhNMe₂ | -CH₂PhNMe₂ |
| | YT-9-4 | -CH₂PhNO₂ | -CH₂PhNO₂ |
| | YT-9-5 | -CH₂PhOMe | -CH₂PhOMe |
| | YT-9-6 | -CH₂PhOH | -CH₂PhOH |
| 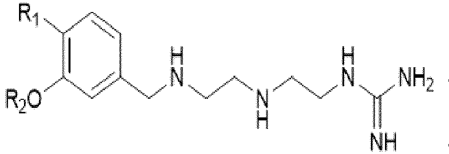 | YT-9-7 | -H | -CH₂PhCl |
| | YT-9-8 | -H | -CH₂PhF |
| | YT-9-9 | -H | -CH₂PhNMe₂ |
| | YT-9-10 | -H | -CH₂PhNO₂ |
| | YT-9-11 | -H | -CH₂PhOMe |
| | YT-9-12 | -H | -CH₂PhOH |

2. The pharmaceutical composition of claim 1, wherein the disease associated with ER-stress is a metabolic proteinopathy.

3. The pharmaceutical composition of claim 1, wherein the disease associated with ER-stress is diabetes, neurodegenerative disease, cancer, or metabolic syndrome.

4. A composition for inducing ER-phagy, which comprises p62 one or more selected from compounds listed in the following tables, or a pharmaceutically acceptable salt, stereoisomer, solvate, hydrate or prodrug thereof:
**[Table 6]**
| No. | Structure | No. | Structure |
|---|---|---|---|
| YOK 110 5 | 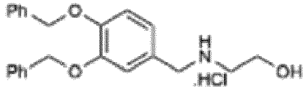 | YOK 220 4 | 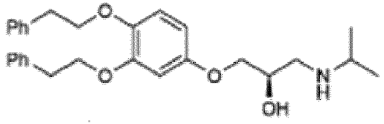 |
| YOK 110 4 | 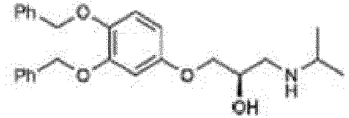 | YOK 110 6 | 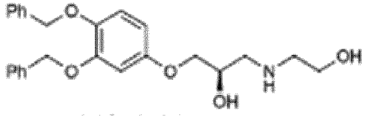 |
| YOK 120 5 | 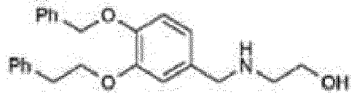 | ATB -15 | 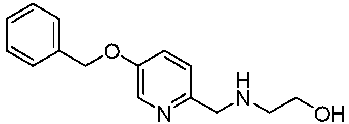 |
| ATB -1 | 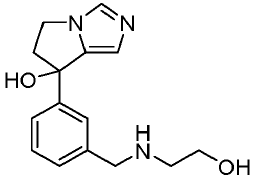 | ATB -16 | 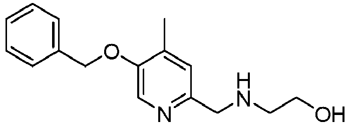 |
| ATB -2 | 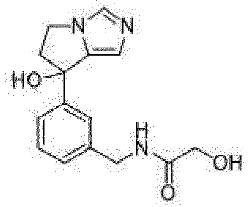 | ATB -17 | 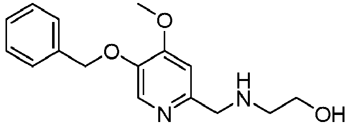 |
| ATB -3 | 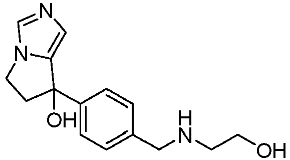 | ATB -18 | 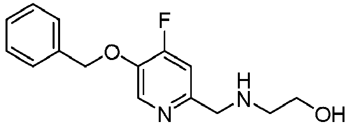 |
| ATB -4 | 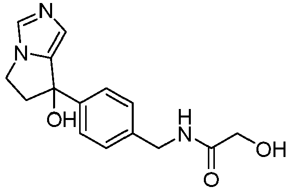 | ATB -19 | 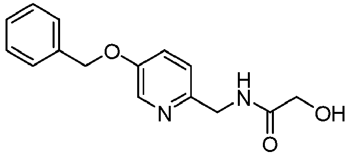 |
| ATB -5 | 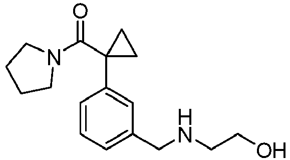 | ATB -20 | 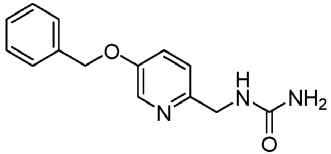 |
| ATB -6 | 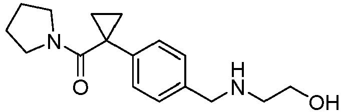 | ATB -21 | 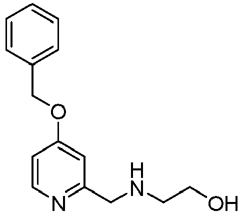 |
| ATB -7 | 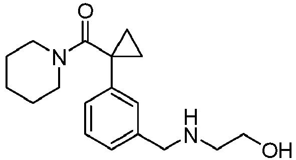 | ATB -22 | 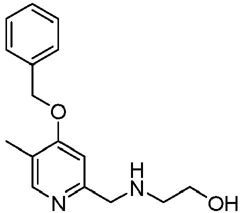 |
| ATB -8 | 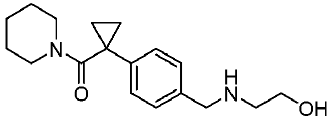 | ATB -23 | 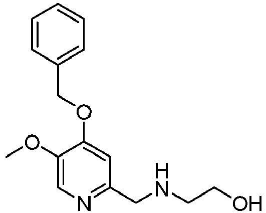 |
| ATB -9 | 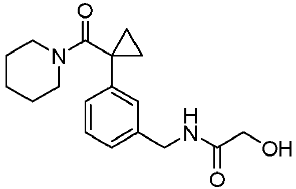 | ATB -24 | 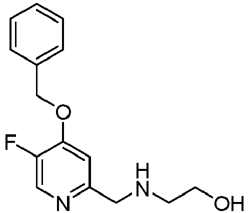 |
| ATB -10 | 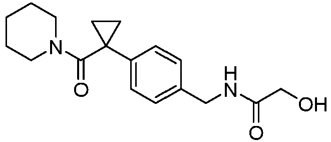 | ATB -25 | 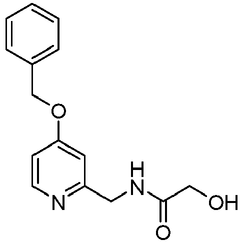 |
| ATB -11 | 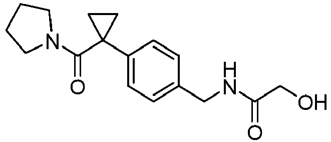 | ATB -26 | 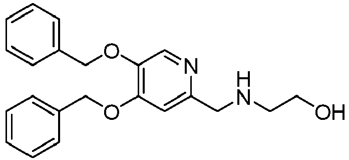 |
| ATB -12 | 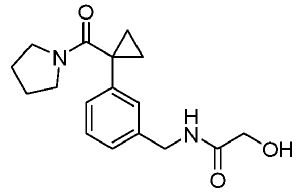 | ATB -27 | 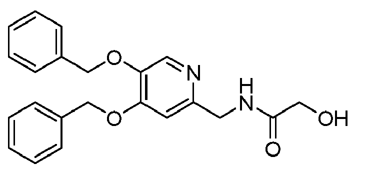 |
| ATB -13 | 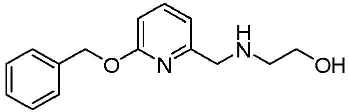 | ATB -28 | 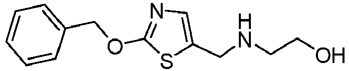 |
| ATB -14 | 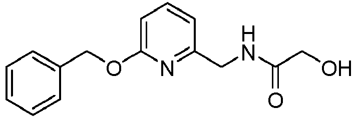 | ATB -29 | 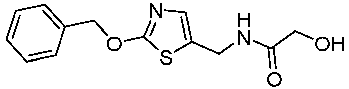 |
**[Table 7]**
| Structural Formula | No. | R1 | R2 |
|---|---|---|---|
| 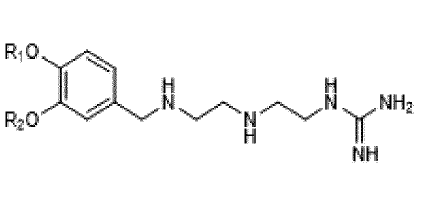 | YtK-1109 | -CH₂Ph | -CH₂Ph |
| | YtK-2209 | -CH₂CH₂Ph | -CH₂CH₂Ph |
| | YtK-3309 | -CH₂CH₂CH₂Ph | -CH₂CH₂CH₂Ph |
| | YtK-4409 | CH₂CH₂CH₂CH₂Ph | - CH₂CH₂CH₂CH₂Ph |
| | YtK-1209 | -CH₂Ph | -CH₂CH₂Ph |
| | YtK-1309 | -CH₂Ph | -CH₂CH₂CH₂Ph |
| | YtK-1409 | -CH₂Ph | CH₂CH₂CH₂CH₂Ph |
| 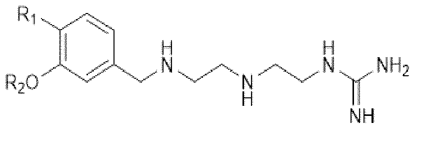 | YTK-109 | -H | -CH₂Ph |
| | YTK-209 | -H | -CH₂CH₂Ph |
| | YTK-309 | -H | -CH₂CH₂CH₂Ph |
| | YTK-409 | -H | CH₂CH₂CH₂CH₂Ph |
| 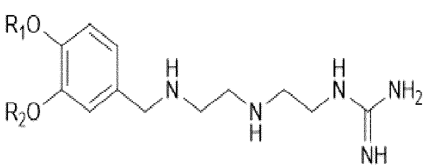 | YT-9-1 | -CH₂PhCl | -CH₂PhCl |
| | YT-9-2 | -CH₂PhF | -CH₂PhF |
| | YT-9-3 | -CH₂PhNMe₂ | -CH₂PhNMe₂ |
| | YT-9-4 | -CH₂PhNO₂ | -CH₂PhNO₂ |
| | YT-9-5 | -CH₂PhOMe | -CH₂PhOMe |
| | YT-9-6 | -CH₂PhOH | -CH₂PhOH |
| 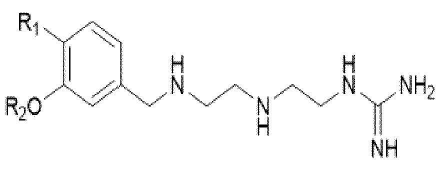 | YT-9-7 | -H | -CH₂PhCl |
| | YT-9-8 | -H | -CH₂PhF |
| | YT-9-9 | -H | -CH₂PhNMe₂ |
| | YT-9-10 | -H | -CH₂PhNO₂ |
| | YT-9-11 | -H | -CH₂PhOMe |
| | YT-9-12 | -H | -CH₂PhOH |

5. A composition for maintaining ER homeostasis or reducing ER stress, which comprises p62 one or more selected from compounds listed in the following tables, or a pharmaceutically acceptable salt, stereoisomer, solvate, hydrate or prodrug thereof:
**[Table 8]**
| No. | Structure | No. | Structure |
|---|---|---|---|
| YOK 110 5 | 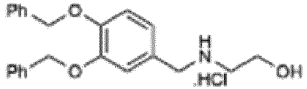 | YOK 220 4 | 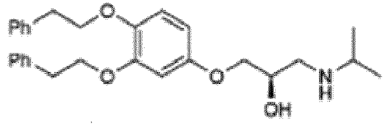 |
| YOK 110 4 | 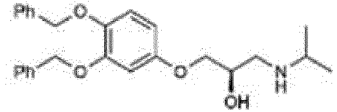 | YOK 110 6 | 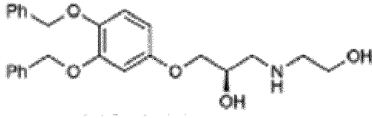 |
| YOK 120 5 | 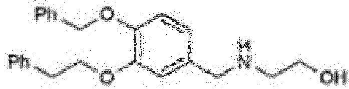 | ATB -15 | 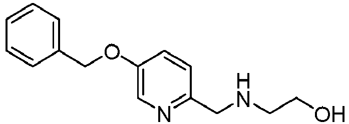 |
| ATB -1 | 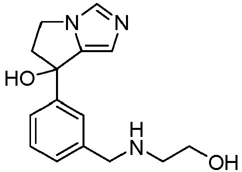 | ATB -16 | 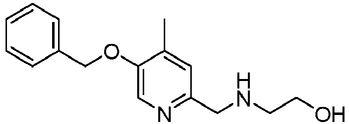 |
| ATB -2 | 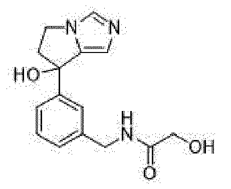 | ATB -17 | 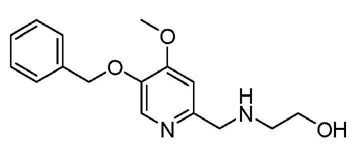 |
| ATB -3 | 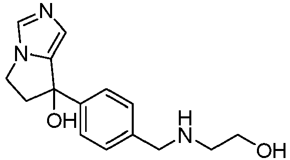 | ATB -18 | 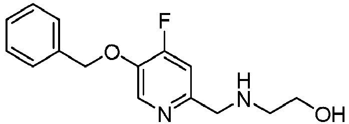 |
| ATB -4 | 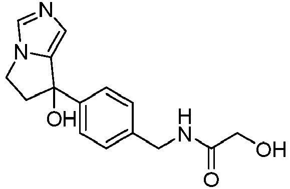 | ATB -19 | 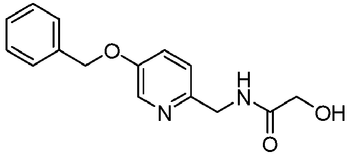 |
| ATB -5 | 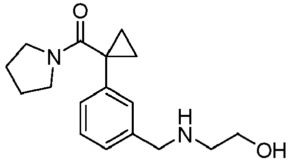 | ATB -20 | 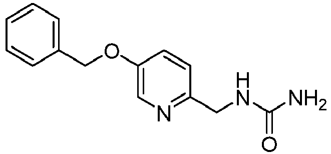 |
| ATB -6 | 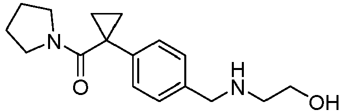 | ATB -21 | 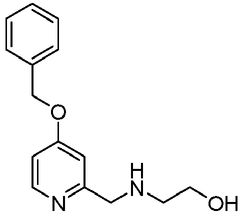 |
| ATB -7 | 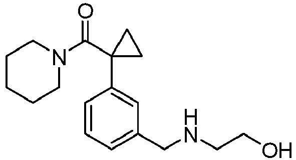 | ATB -22 | 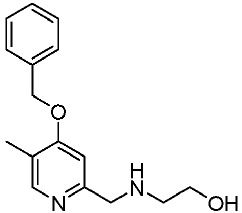 |
| ATB -8 | 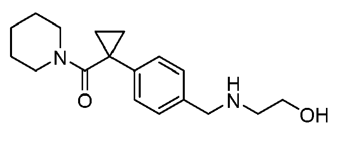 | ATB -23 | 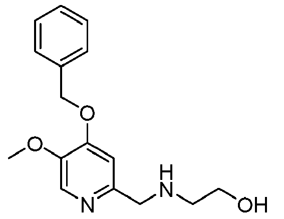 |
| ATB -9 | 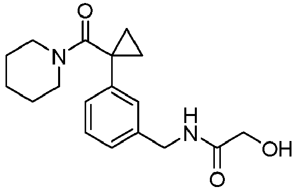 | ATB -24 | 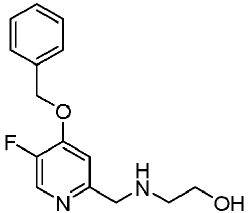 |
| ATB -10 | 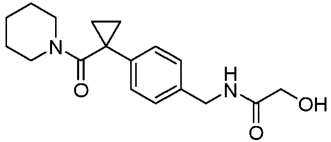 | ATB -25 | 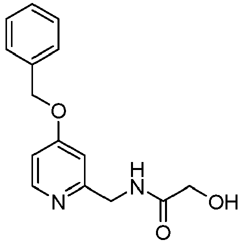 |
| ATB -11 | 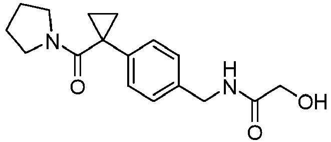 | ATB -26 | 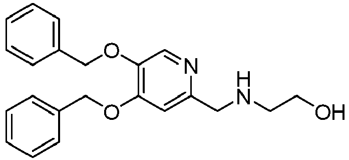 |
| ATB -12 | 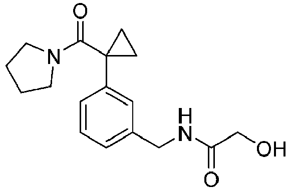 | ATB -27 | 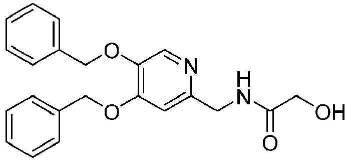 |
| ATB -13 | 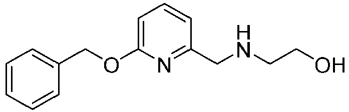 | ATB -28 | 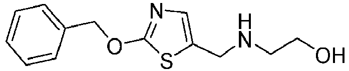 |
| ATB -14 | 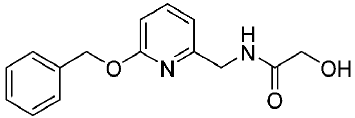 | ATB -29 | 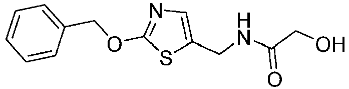 |
**[Table 9]**
| Structural Formula | No. | R1 | R2 |
|---|---|---|---|
| 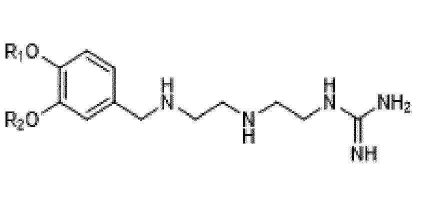 | YtK-1109 | -CH₂Ph | -CH₂Ph |
| | YtK-2209 | -CH₂CH₂Ph | -CH₂CH₂Ph |
| | YtK-3309 | -CH₂CH₂CH₂Ph | -CH₂CH₂CH₂Ph |
| | YtK-4409 | CH₂CH₂CH₂CH₂Ph | - CH₂CH₂CH₂CH₂Ph |
| | YtK-1209 | -CH₂Ph | -CH₂CH₂Ph |
| | YtK-1309 | -CH₂Ph | -CH₂CH₂CH₂Ph |
| | YtK-1409 | -CH₂Ph | CH₂CH₂CH₂CH₂Ph |
| 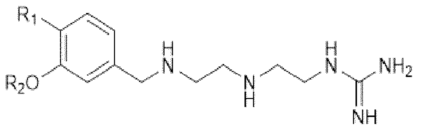 | YTK-109 | -H | -CH₂Ph |
| | YTK-209 | -H | -CH₂CH₂Ph |
| | YTK-309 | -H | -CH₂CH₂CH₂Ph |
| | YTK-409 | -H | - CH₂CH₂CH₂CH₂Ph |
| 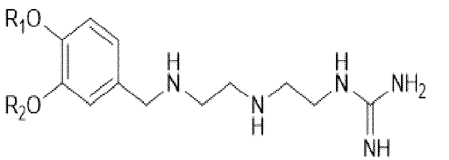 | YT-9-1 | -CH₂PhCl | -CH₂PhCl |
| | YT-9-2 | -CH₂PhF | -CH₂PhF |
| | YT-9-3 | -CH₂PhNMe₂ | -CH₂PhNMe₂ |
| | YT-9-4 | -CH₂PhNO₂ | -CH₂PhNO₂ |
| | YT-9-5 | -CH₂PhOMe | -CH₂PhOMe |
| | YT-9-6 | -CH₂PhOH | -CH₂PhOH |
| 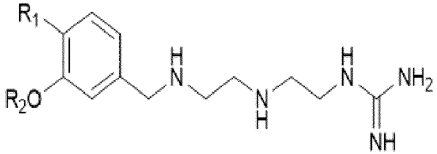 | YT-9-7 | -H | -CH₂PhCl |
| | YT-9-8 | -H | -CH₂PhF |
| | YT-9-9 | -H | -CH₂PhNMe₂ |
| | YT-9-10 | -H | -CH₂PhNO₂ |
| | YT-9-11 | -H | -CH₂PhOMe |
| | YT-9-12 | -H | -CH₂PhOH |

6. A composition for being added to a cell culture medium, which comprises p62 one or more selected from compounds listed in the following tables, or a pharmaceutically acceptable salt, stereoisomer, solvate, hydrate or prodrug thereof:
**[Table 8]**
| No. | Structure | No. | Structure |
|---|---|---|---|
| YOK 110 5 | 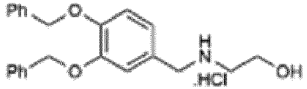 | YOK 220 4 | 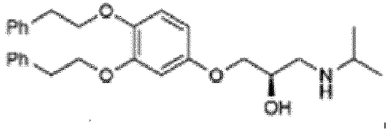 |
| YOK 110 4 | 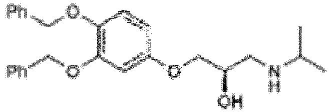 | YOK 110 6 | 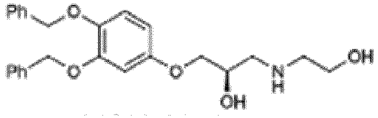 |
| YOK 120 5 | 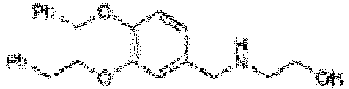 | ATB -15 | 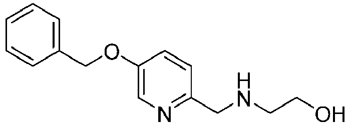 |
| ATB -1 | 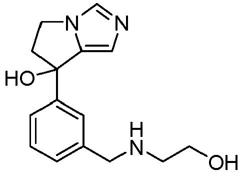 | ATB -16 | 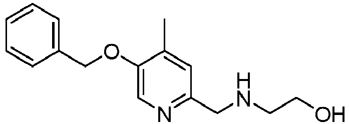 |
| ATB -2 | 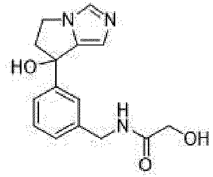 | ATB -17 | 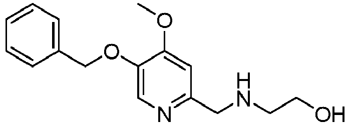 |
| ATB -3 | 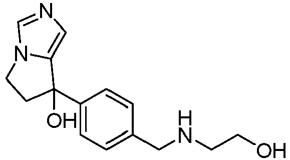 | ATB -18 | 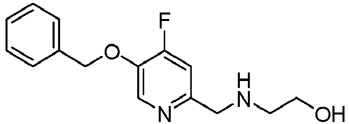 |
| ATB -4 | 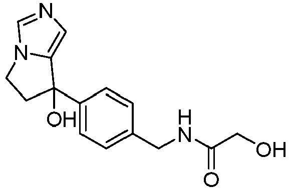 | ATB -19 | 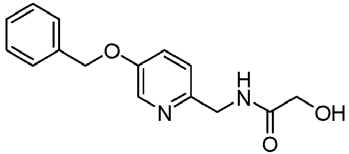 |
| ATB -5 | 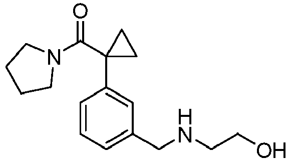 | ATB -20 | 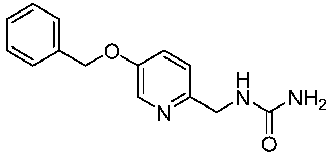 |
| ATB -6 | 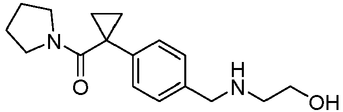 | ATB -21 | 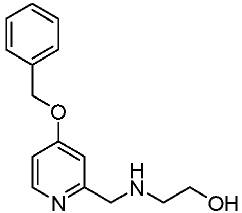 |
| ATB -7 | 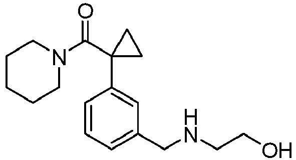 | ATB -22 | 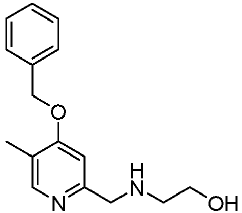 |
| ATB -8 | 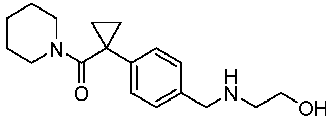 | ATB -23 | 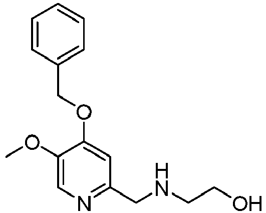 |
| ATB -9 | 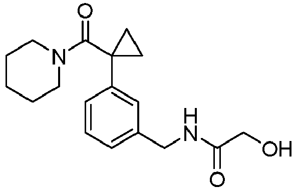 | ATB -24 | 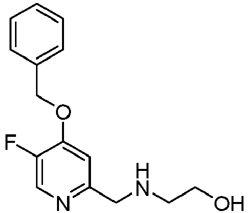 |
| ATB -10 | 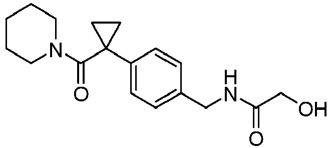 | ATB -25 | 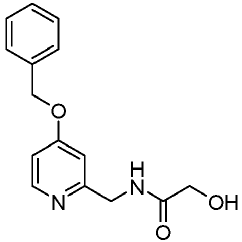 |
| ATB -11 | 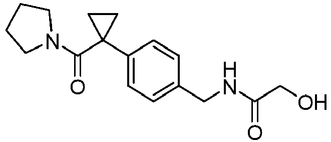 | ATB -26 | 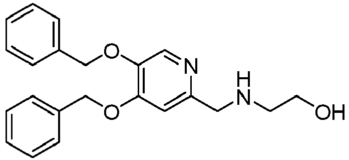 |
| ATB -12 | 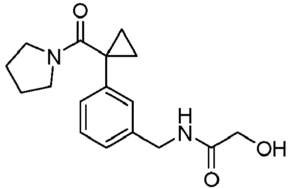 | ATB -27 | 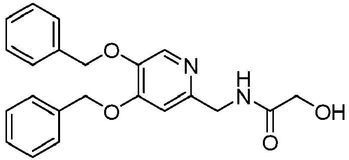 |
| ATB -13 | 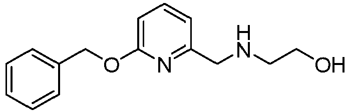 | ATB -28 | 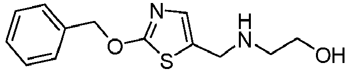 |
| ATB -14 | 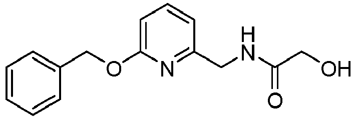 | ATB -29 | 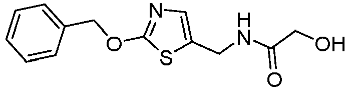 |
**[Table 9]**
| Structural Formula | No. | R1 | R2 |
|---|---|---|---|
| 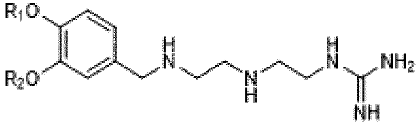 | YtK-1109 | -CH₂Ph | -CH₂Ph |
| | YtK-2209 | -CH₂CH₂Ph | -CH₂CH₂Ph |
| | YtK-3309 | -CH₂CH₂CH₂Ph | -CH₂CH₂CH₂Ph |
| | YtK-4409 | CH₂CH₂CH₂CH₂Ph | CH₂CH₂CH₂CH₂Ph |
| | YtK-1209 | -CH₂Ph | -CH₂CH₂Ph |
| | YtK-1309 | -CH₂Ph | -CH₂CH₂CH₂Ph |
| | YtK-1409 | -CH₂Ph | CH₂CH₂CH₂CH₂Ph |
| 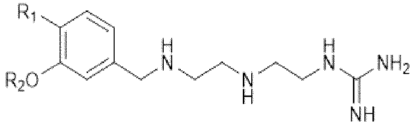 | YTK-109 | -H | -CH₂Ph |
| | YTK-209 | -H | -CH₂CH₂Ph |
| | YTK-309 | -H | -CH₂CH₂CH₂Ph |
| | YTK-409 | -H | - CH₂CH₂CH₂CH₂Ph |
| 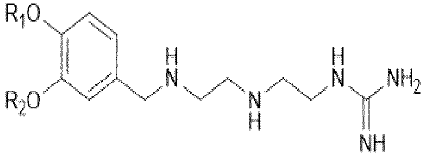 | YT-9-1 | -CH₂PhCl | -CH₂PhCl |
| | YT-9-2 | -CH₂PhF | -CH₂PhF |
| | YT-9-3 | -CH₂PhNMe₂ | -CH₂PhNMe₂ |
| | YT-9-4 | -CH₂PhNO₂ | -CH₂PhNO₂ |
| | YT-9-5 | -CH₂PhOMe | -CH₂PhOMe |
| | YT-9-6 | -CH₂PhOH | -CH₂PhOH |
| 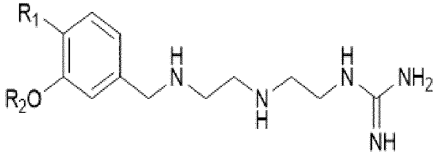 | YT-9-7 | -H | -CH₂PhCl |
| | YT-9-8 | -H | -CH₂PhF |
| | YT-9-9 | -H | -CH₂PhNMe₂ |
| | YT-9-10 | -H | -CH₂PhNO₂ |
| | YT-9-11 | -H | -CH₂PhOMe |
| | YT-9-12 | -H | -CH₂PhOH |

7. The composition of claim 6, wherein the cell is a cell for protein production.

8. A method of screening a candidate drug for preventing or treating a disease associated with ER-stress, which comprises:
contacting a candidate compound with a biological sample comprising p62 and a receptor associated with ER-phagy;
confirming formation of a complex of p62 and the receptor associated with ER-phagy, or oligomerization or aggregation of the receptor associated with ER-phagy; and
selecting the candidate compound as a candidate drug for preventing and/or treating a disease associated with ER-stress when the formation of a complex of p62 and the receptor associated with ER-phagy, or oligomerization or aggregation of the receptor associated with ER-phagy is confirmed, or increased more than in a biological sample which is not treated with the candidate compound.
